# EUROPEAN PATENT APPLICATION

(11) **EP 1 632 484 A1**
(43) Date of publication of application: **08.03.2006**
(21) Application number: 04745927.6
(22) Date of filing: 09.06.2004
(51) Int. Cl.: C07D 285/12, A61K 31/433, A61P 35/00

(54) **THIADIAZOLINE DERIVATIVE**

(30) Priority: 10.06.2003 JP 2003164727; 16.04.2004 JP 2004121324
(71) Applicant: KYOWA HAKKO KOGYO CO., LTD., Chiyoda-ku, Tokyo 100-8185 (JP); FUJI PHOTO FILM CO., LTD., Minamiashigara-shi, Kanagawa-ken 250-0193 (JP)
(72) Inventor: MURAKATA, Chikara, Sunto-gun, Shizuoka 4118731 (JP); INO, Yoji, Sunto-gun, Shizuoka 4118731 (JP); KATO, Kazuhiko, Sunto-gun, Shizuoka 4118731 (JP); YAMAMOTO, Junichiro, Sunto-gun, Shizuoka 4118731 (JP); KITAMURA, Yuji, Sunto-gun, Shizuoka 4118731 (JP); NAKAI, Ryuichiro, Sunto-gun, Shizuoka 4118731 (JP); NAKANO, Tomohisa, Chiyoda-ku, Toykoy 1008185 (JP); TSUJITA, Tetsuya, Chuo-ku, Tokyo 1038503 (JP)
(74) Representative: Sternagel, Fleischer, Godemeyer & Partner
(86) International application number: PCT/JP2004/008375
(87) International publication number: WO 2004/111024

(57) **Abstract**

A thiadiazoline derivative represented by the general formula (I), or a pharmacologically acceptable salt thereof: 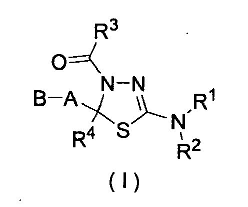 [wherein R¹ represents a hydrogen atom and the like, R² represents a hydrogen atom, -COR⁵ (wherein R⁵ represents lower alkyl and the like) and the like, R³ represents lower alkyl and the like, R⁴ represents aryl and the like, A represents -(CH₂)ₙ₋(wherein n represents an integer of 1 to 6) and the like, and B represents -NR⁶R⁷ (wherein R⁶ and R⁷ are the same or different and represent a hydrogen atom, lower alkyl and the like) and the like] is provided.

## Description

### Technical Field

The present invention relates to a thiadiazoline derivative or a pharmacologically acceptable salt thereof which is useful for therapeutic treatment of tumor and the like.

### Background Art

Pharmaceutical agents such as vinca alkaloids and taxanes, which are clinically important antitumor agents, have an action of binding to microtubules to inhibit the functions of spindles comprising microtubules as structural units. It is known that the functions of spindles are indispensable to localization of centromeres and correct separation of chromosomes at the time of cell division (mitotic phase of cell cycle), and inhibition of the functions thereof leads to inhibition of normal cell division and induce cell death of cancer cells [Biochem. Biophys. Res. Commun., Vol. 263, p.398 (1999)].

The microtubules are involved in maintenance of cell morphology, intracellular substance transport, and axonal transport of nerve fibers, as well as serve as molecular components of mitotic spindles. Accordingly, anticancer agents acting on the microtubule not only have an effect on cancer cells but also adversely affect on normal cells. For example, as side effects unique to the agents acting on the microtubule, peripheral nerve disorders due to the inhibition of the axonal transport of the nerve fibers have been recognized as clinical problems. Therefore, an agent that acts on a molecule, other than the microtubule, which is important for regulation of the spindle function during the mitotic phase of the cell cycle and inhibits the spindle functions in the same manner as existing microtubule-acting anticancer agents, is expected to be a potential novel anticancer agent which avoids the aforementioned side effects derived from the action on the microtubules observed for the existing anticancer agents.

The mitotic kinesins are proteins that are involved in the mitotic spindle regulation, and play an essential role for progression of the mitotic phase in cell cycle. These proteins have a function of moving proteins along microtubules using the energy produced by ATP hydrolysis, and belong to a class of functional proteins generally called "molecular motors". In the mitotic phase, the proteins are deeply involved in extension and maintenance of mitotic spindles, as well as formation of structure called spindle pole body, and further, they regulate progression of normal cell division through the movement of chromosomes along the spindle microtubules.

The mitotic kinesin Eg5 is one of the mitotic kinesins constituting an evolutionarily conserved subfamily. It is known that Eg5 has a function as a bipolar homotetramer molecule, and is involved in the formation of the bipolar spindle structure by crosslinking two of microtubules of the same direction and moving them in the direction toward the + (plus) end to cause sliding of two of the antiparallel microtubules, thereby keep - (minus) ends of microtubules at a distance and separate spindle pole bodies. The above functions of Eg5 were elucidated on the basis of the analysis of the human cells treated with anti-Eg5 antibody and a specific inhibitor [Cell, Vol. 83, p.1159 (1995); J. Cell Biol., Vol. 150, p.975 (2000); Jikken Igaku (Experimental Medicine), Vol. 17, p.439 (1999)].

The gene of human Eg5 was cloned in 1995, and the expression of a full-length human Eg5 recombinant protein by using an insect cell and functional analysis using the resulting protein were reported [Cell, Vol. 83, p.1159 (1995)]. The gene was registered in a public database as GenBank accession numbers: X85137, NM004523 and U37426. A biochemical analysis and structure analysis by crystallization of Eg5 utilizing an N-terminus portion of human Eg5, expressed by using *Escherichia coli* cells, were reported [J. Biological Chemistry, Vol. 276, p.25496 (2001); Chemistry & Biology, Vol. 9, p.989 (2002)], which applied a technique similar to the analysis utilizing Eg5 derived from *Xenopus laevis* having a high homology to the human Eg5 [Proc. Natl. Acad. Sci. USA, Vol. 96, p.9106 (1999); Biochemistry, Vol. 35, p.2365 (1996)].

It is known that the expression of Eg5 in human normal tissues are limited to testis, thymus and the like, and it has been reported, on the basis of results of analysis of tissues from cancer patients, that human Eg5 is more intensely expressed in tumor tissues compared with normal tissues [Proc. Natl. Acad. Sci. USA, Vol. 99, p.4465 (2002), US6414121B1].

As described above, the mitotic kinesin Eg5 is important as a target molecule of a novel mitotic phase acting agent and it is considered that an inhibitor against said molecule is promising as an agent for therapeutic treatment of diseases caused by abnormality of the regulation of cell proliferation.

As compounds having inhibitory activity against the human Eg5 enzyme, monastrol [Science, Vol. 286, p.971 (1999)], quinazoline derivatives (WO01/98278), phenathiazine derivatives (WO02/57244), triphenylmethane derivatives (WO02/56880), dihydropyrimidine derivatives (WO02/79149; WO02/79169), and the like were reported.

Thiadiazoline derivatives having inhibitory activity against a transcription factor STAT6 activation or those having integrin antagonistic action are known (Japanese Patent Unexamined Publication (KOKAI) No. 2000-229959; WO01/56994), and further, those having an antibacterial activity, ACE inhibitory activity or the like are also known (WO93/22311; Japanese Patent Unexamined Publication (KOKAI) No. 62-53976; J. Bangladesh Chem. Soc., Vol. 5, p.127 (1992)).

### Disclosure of the Invention

An object of the present invention is to provide a thiadiazoline derivative or a pharmacologically acceptable salt thereof which is useful for therapeutic treatment of a disease involving cell proliferation, for example, therapeutic treatment of a malignant tumor (breast cancer, gastric cancer, ovarian cancer, colon cancer, lung cancer, brain cancer, laryngeal cancer, hematological cancer, urinary or genital tumor including bladder cancer and prostate cancer, renal cancer, skin cancer, liver cancer, pancreatic cancer, uterine cancer, and the like), restenosis, cardiac hypertrophy, an immunologic disease, and the like.

The present invention relates to the following (1) to (34).
(1) A thiadiazoline derivative represented by the general formula (I), or a pharmacologically acceptable salt thereof: 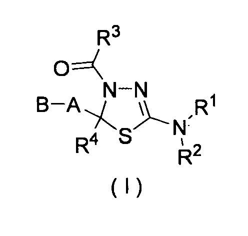 <wherein,
   R¹ represents a hydrogen atom, substituted or unsubstituted lower alkyl, substituted or unsubstituted lower alkenyl, substituted or unsubstituted lower alkynyl, or substituted or unsubstituted cycloalkyl,
   R² represents a hydrogen atom, or -COR⁵ (wherein R⁵ represents substituted or unsubstituted lower alkyl, substituted or unsubstituted lower alkenyl, substituted or unsubstituted lower alkynyl, or substituted or unsubstituted cycloalkyl), or
   R¹ and R² are combined together with the adjacent nitrogen atom to form a substituted or unsubstituted heterocyclic group,
   R³ represents a hydrogen atom, substituted or unsubstituted lower alkyl, substituted or unsubstituted lower alkenyl, substituted or unsubstituted lower alkynyl, or substituted or unsubstituted cycloalkyl,
   R⁴ represents substituted or unsubstituted aryl, or a substituted or unsubstituted heterocyclic group,
   A represents -(CH₂)ₙ- (wherein n represents an integer of 1 to 6), or a group of the formula (II) 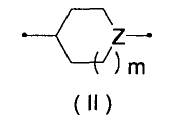 (wherein m represents an integer of 0 to 2, and Z represents CH or a nitrogen atom capable of binding to B), and
   (i) when A is -(CH₂)ₙ-, and n is 1 or 2,
      B represents -NR⁶R⁷ {wherein R⁶ represents a hydrogen atom, or lower alkyl, R⁷ represents substituted lower alkyl, -COR⁸ [wherein R⁸ represents substituted lower alkyl (provided that R⁸ is not trifluoromethyl), substituted lower alkoxy, substituted or unsubstituted aryloxy, a substituted or unsubstituted heterocyclic group, or -NR⁹R¹⁰ (wherein R⁹ and R¹⁰ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R⁹ and R¹⁰ are combined together with the adjacent nitrogen atom to form a substituted or unsubstituted heterocyclic group)], or R⁶ and R⁷ are combined together with the adjacent nitrogen atom to form a substituted or unsubstituted heterocyclic group},
      -OR¹¹ (wherein R¹¹ represents substituted lower alkyl, substituted or unsubstituted lower alkanoyl, substituted or unsubstituted lower alkylcarbamoyl, substituted or unsubstituted di-(lower alkyl)carbamoyl, or substituted or unsubstituted heterocyclylcarbonyl),
      -SR¹² (wherein R¹² has the same meaning as that of the aforementioned R¹¹), or CH=NR¹³ (wherein R¹³ represents hydroxy, or substituted or unsubstituted lower alkoxy),
   (ii) when A is -(CH₂)ₙ-, and n is an integer of 3 to 6 ,
      B represents -NR¹⁴R¹⁵ {wherein R¹⁴ and R¹⁵ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, -COR¹⁶ [wherein R¹⁶ represents substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, substituted or unsubstituted lower alkoxy, substituted or unsubstituted aryloxy, or -NR¹⁷R¹⁸ (wherein R¹⁷ and R¹⁸ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R¹⁷ and R¹⁸ are combined together with the adjacent nitrogen atom to form a substituted or unsubstituted heterocyclic group)], or -SO₂R¹⁹ [wherein R¹⁹ represents substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, or -NR²⁰R²¹ (wherein R²⁰ and R²¹ are the same or different, and represent a hydrogen atom, substituted or unsubstituted lower alkyl, substituted or unsubstituted lower alkenyl, substituted or unsubstituted lower alkynyl, or substituted or unsubstituted cycloalkyl, or R²⁰ and R²¹ are combined together with the adjacent nitrogen atom to form a substituted or unsubstituted heterocyclic group)], or R¹⁴ and R¹⁵ are combined together with the adjacent nitrogen atom to form a substituted or unsubstituted heterocyclic group},
      -OR²² (wherein R²² has the same meaning as that of the aforementioned R¹¹),
      -SR²³ (wherein R²³ has the same meaning as that of the aforementioned R¹¹), or
      -CH=NR²⁴ (wherein R²⁴ has the same meaning as that of the aforementioned R¹³),
   (iii) when A is a group of the formula (II),
      B represents a hydrogen atom, substituted or unsubstituted lower alkyl, substituted or unsubstituted lower alkanoyl, substituted or unsubstituted lower alkoxycarbonyl, or substituted or unsubstituted lower alkylsulfonyl>.
(2) The thiadiazoline derivative or a pharmacologically acceptable salt thereof according to (1), wherein R¹ is a hydrogen atom, or lower alkyl.
(3) The thiadiazoline derivative or a pharmacologically acceptable salt thereof according to (1) or (2), wherein R² is -COR⁵ (wherein R⁵ has the same meaning as that mentioned above).
(4) The thiadiazoline derivative or a pharmacologically acceptable salt thereof according to (3), wherein R⁵ is lower alkyl.
(5) The thiadiazoline derivative or a pharmacologically acceptable salt thereof according to (3), wherein R⁵ is tert-butyl.
(6) The thiadiazoline derivative or a pharmacologically acceptable salt thereof according to any one of (1) to (5), wherein R³ is lower alkyl.
(7) The thiadiazoline derivative or a pharmacologically acceptable salt thereof according to any one of (1) to (5), wherein R³ is tert-butyl.
(8) The thiadiazoline derivative or a pharmacologically acceptable salt thereof according to any one of (1) to (7), wherein R⁴ is substituted or unsubstituted aryl.
(9) The thiadiazoline derivative or a pharmacologically acceptable salt thereof according to any one of (1) to (7), wherein R⁴ is phenyl.
(10) The thiadiazoline derivative or a pharmacologically acceptable salt thereof according to any one of (1) to (9), wherein A is -(CH₂)ₙ- (wherein n has the same meaning as that mentioned above).
(11) The thiadiazoline derivative or a pharmacologically acceptable salt thereof according to (10), wherein n is 1 or 2.
(12) The thiadiazoline derivative or a pharmacologically acceptable salt thereof according to (11), wherein B is -NR⁶R⁷ (wherein R⁶ and R⁷ have the same meanings as those mentioned above, respectively).
(13) The thiadiazoline derivative or a pharmacologically acceptable salt thereof according to (12), wherein R⁶ is a hydrogen atom.
(14) The thiadiazoline derivative or a pharmacologically acceptable salt thereof according to (12) or (13), wherein R⁷ is -COR⁸ (wherein R⁸ has the same meaning as that mentioned above).
(15) The thiadiazoline derivative or a pharmacologically acceptable salt thereof according to (12), wherein R⁶ and R⁷ are combined together with the adjacent nitrogen atom to form a substituted or unsubstituted heterocyclic group.
(16) The thiadiazoline derivative or a pharmacologically acceptable salt thereof according to (10), wherein n is an integer of 3 to 6.
(17) The thiadiazoline derivative or a pharmacologically acceptable salt thereof according to (10), wherein n is 3.
(18) The thiadiazoline derivative or a pharmacologically acceptable salt thereof according to (17), wherein B is -NR¹⁴R¹⁵ (wherein R¹⁴ and R¹⁵ have the same meanings as those mentioned above, respectively).
(19) The thiadiazoline derivative or a pharmacologically acceptable salt thereof according to (18), wherein R¹⁴ is a hydrogen atom.
(20) The thiadiazoline derivative or a pharmacologically acceptable salt thereof according to (18) or (19), wherein R¹⁵ is substituted or unsubstituted lower alkyl.
(21) The thiadiazoline derivative or a pharmacologically acceptable salt thereof according to (18) or (19), wherein R¹⁵ is -COR¹⁶ (wherein R¹⁶ has the same meaning as that mentioned above).
(22) The thiadiazoline derivative or a pharmacologically acceptable salt thereof according to (21), wherein R¹⁶ is a substituted or unsubstituted heterocyclic group.
(23) The thiadiazoline derivative or a pharmacologically acceptable salt thereof according to (21), wherein R¹⁶ is -NR¹⁷R¹⁸ (wherein R¹⁷ and R¹⁸ have the same meanings as those mentioned above, respectively).
(24) The thiadiazoline derivative or a pharmacologically acceptable salt thereof according to (18) or (19), wherein R¹⁵ is -SO₂R¹⁹ (wherein R¹⁹ has the same meaning as that mentioned above).
(25) The thiadiazoline derivative or a pharmacologically acceptable salt thereof according to any one of (1) to (9), wherein A is a group of the formula (II).
(26) The thiadiazoline derivative or a pharmacologically acceptable salt thereof according to (25), wherein Z is a nitrogen atom.
(27) The thiadiazoline derivative or a pharmacologically acceptable salt thereof according to (25) or (26), wherein B is a hydrogen atom, or substituted or unsubstituted lower alkyl.
(28) A pharmaceutical composition which comprises the thiadiazoline derivative or a pharmacologically acceptable salt thereof according to any one of (1) to (27) as an active ingredient.
(29) A mitotic kinesin Eg5 inhibitor which comprises the thiadiazoline derivative or a pharmacologically acceptable salt thereof according to any one of (1) to (27) as an active ingredient.
(30) An antitumor agent which comprises the thiadiazoline derivative or a pharmacologically acceptable salt thereof according to any one of (1) to (27) as an active ingredient.
(31) A method for inhibiting a mitotic kinesin Eg5 which comprises administering an effective amount of the thiadiazoline derivative or a pharmacologically acceptable salt thereof according to any one of (1) to (27).
(32) A method for therapeutic treatment of a malignant tumor which comprises administering an effective amount of the thiadiazoline derivative or a pharmacologically acceptable salt thereof according to any one of (1) to (27).
(33) Use of the thiadiazoline derivative or a pharmacologically acceptable salt thereof according to any one of (1) to (27) for the manufacture of a mitotic kinesin Eg5 inhibitor.
(34) Use of the thiadiazoline derivative or a pharmacologically acceptable salt thereof according to any one of (1) to (27) for the manufacture of the antitumor agent.

Hereinafter, compounds represented by the general formula (I) are referred to as "Compound (I)". The compounds having the other formula numbers are referred to in the same manner.

In the definition of each group of the general formula (I),
(i) examples of the lower alkyl moiety in the lower alkyl, the lower alkoxy, the lower alkanoyl, the lower alkoxycarbonyl, the lower alkylcarbamoyl, the di-(lower alkyl)carbamoyl, and the lower alkylsulfonyl include straight or branched chain alkyl having 1 to 10 carbon atoms, for example, methyl, ethyl, propyl, isopropyl, butyl, isobutyl, sec-butyl, tert-butyl, pentyl, isopentyl, neopentyl, hexyl, heptyl, octyl, nonyl, decyl and the like. The two lower alkyl moieties in the di-(lower alkyl)carbamoyl may be the same or different.
(ii) Examples of the lower alkenyl include straight or branched chain alkenyl having 2 to 10 carbon atoms, for example, vinyl, allyl, 1-propenyl, butenyl, pentenyl, hexenyl, heptenyl, octenyl, nonenyl, decenyl and the like.
(iii) Examples of the lower alkynyl include straight or branched chain alkynyl having 2 to 10 carbon atoms, for example, ethynyl, propynyl, butynyl, pentynyl, hexynyl, heptynyl, octynyl, nonynyl, decynyl and the like.
(iv) Examples of the cycloalkyl include cycloalkyl having 3 to 8 carbon atoms, for example, cyclopropyl, cyclobutyl, cyclopentyl, cyclohexyl, cycloheptyl, cyclooctyl and the like.
(v) Examples of the aryl and the aryl moiety in the aryloxy include phenyl, naphthyl and the like.
(vi) Examples of the heterocyclic group and the heterocyclic group moiety of the heterocyclylcarbonyl include an aliphatic heterocyclic group, an aromatic heterocyclic group and the like. Examples of the aliphatic heterocyclic group include a 5- or 6-membered monocyclic aliphatic heterocyclic group containing at least one atom selected from a nitrogen atom, an oxygen atom and a sulfur atom, and a bicyclic or tricyclic condensed aliphatic heterocyclic group comprising 3- to 8-membered rings and containing at least one atom selected from a nitrogen atom, an oxygen atom and a sulfur atom and the like, for example, azetidinyl, tetrahydrothienyl, tetrahydrothiopyranyl, imidazolidinyl, pyrrolidinyl, oxazolinyl, dioxolanyl, piperidino, piperidinyl, piperazinyl, morpholino, morpholinyl, thiomorpholinyl, homopiperidinyl, homopiperazinyl, tetrahydropyridinyl, tetrahydroquinolinyl, tetrahydroisoquinolinyl, tetrahydrofuranyl, tetrahydropyranyl, dihydrobenzofuranyl, pyranyl and the like. Examples of the aromatic heterocyclic group include a 5- or 6-membered monocyclic aromatic heterocyclic group containing at least one atom selected from a nitrogen atom, an oxygen atom and a sulfur atom, and a bicyclic or tricyclic condensed aromatic heterocyclic group comprising 3- to 8-membered rings and containing at least one atom selected from a nitrogen atom, an oxygen atom and a sulfur atom, and the like, for example, furyl, thienyl, pyrrolyl, pyrazolyl, imidazolyl, triazolyl, tetrazolyl, thiazolyl, isothiazolyl, oxazolyl, isoxazolyl, thiadiazolyl, oxadiazolyl, pyridyl, pyrazinyl, pyrimidinyl, pyridazinyl, indolyl, isoindolyl, indazolyl, benzoxazolyl, benzothienyl, benzimidazolyl, benzothiazolyl, benzotriazolyl, purinyl, quinolyl, isoquinolyl, quinazolinyl, phthalazinyl, quinoxalinyl, naphthylidinyl, benzodiazepinyl, phenothiazinyl, benzopyranyl, cinnolinyl, and the like.
(vii) Examples of the heterocyclic group formed together with the adjacent nitrogen atom include an aliphatic heterocyclic group containing at least one nitrogen atom, and the like. Said aliphatic heterocyclic group containing at least one nitrogen atom may contain an oxygen atom, a sulfur atom or another nitrogen atom, and examples thereof include, for example, 1-pyrrolyl, pyrrolidinyl, imidazolyl, morpholino, thiomorpholino, pyrazolidinyl, piperidino, piperazinyl, homopiperazinyl, aziridinyl, azetidinyl, azolidinyl, perhydroazepinyl, perhydroazocinyl, tetrahydroquinolinyl, tetrahydroisoquinolinyl, indolyl, isoindolyl, 1,3-dihydroisoindolyl, pyrrolidonyl, succinimidyl, glutarimidyl, piperidonyl, 1,2-thiazan-2-yl, 1,2-thiazepan-2-yl, and the like.
(viii) The substituent in the substituted lower alkyl, the substituted lower alkenyl, the substituted lower alkynyl, the substituted cycloalkyl, the substituted lower alkoxy, the substituted lower alkanoyl, the substituted lower alkoxycarbonyl, the substituted lower alkylcarbamoyl, the substituted di-(lower alkyl)carbamoyl and the substituted lower alkylsulfonyl may be the same or different in number of 1 to substitutable number, preferably 1 to 3 substituent(s), and includes halogen, hydroxy, oxo, nitro, azido, cyano, carboxy, substituted or unsubstituted cycloalkyl {the substituent (a) in said substituted cycloalkyl may be the same or different in number of 1 to 3 substituent(s), and includes
   halogen, hydroxy, oxo, amino, nitro, azido, cyano, carboxy,
   substituted or unsubstituted lower alkoxy (the substituent (b) in said substituted lower alkoxy may be the same or different in number of 1 to 3 substituent(s), and includes
   halogen, hydroxy, oxo, amino, nitro, azido, cyano, carboxy, lower alkoxy, hydroxy-substituted lower alkoxy, lower alkoxy-substituted lower alkoxy, amino-substituted lower alkoxy, lower alkylamino, di-(lower alkyl)amino, hydroxy-substituted lower alkylamino, lower alkoxy-substituted lower alkylamino, amino-substituted lower alkylamino, aralkyloxy, aryl, aryloxy, a heterocyclic group, and the like),
   substituted or unsubstituted lower alkylthio (the substituent in the substituted lower alkylthio has the same meaning as that of the substituent (b) in the aforementioned substituted lower alkoxy),
   substituted or unsubstituted lower alkanoyl (the substituent in the substituted lower alkanoyl has the same meaning as that of the substituent (b) in the aforementioned substituted lower alkoxy),
   -NR²⁵R²⁶ [wherein R²⁵ and R²⁶ are the same or different, and represent
   a hydrogen atom, substituted or unsubstituted lower alkyl (the substituent in the substituted lower alkyl has the same meaning as that of the substituent (b) in the aforementioned substituted lower alkoxy), substituted or unsubstituted lower alkanoyl (the substituent in the substituted lower alkanoyl has the same meaning as that of the substituent (b) in the aforementioned substituted lower alkoxy), aralkyl, aryl, or a heterocyclic group, or
   R²⁵ and R²⁶ are combined together with the adjacent nitrogen atom to form a substituted or unsubstituted heterocyclic group (the substituent in the heterocyclic group formed together with the adjacent nitrogen atom has the same meaning as that of the substituent (b) in the aforementioned lower alkoxy)], aryl, a heterocyclic group, and the like},
   substituted or unsubstituted aryl (the substituent in the substituted aryl has the same meaning as that of the after-mentioned substituent (xi) in the substituted aryl), a substituted or unsubstituted heterocyclic group (the substituent in the substituted heterocyclic group has the same meaning as that of the after-mentioned substituent (xii) in the substituted heterocyclic group),
   -NR²⁷R²⁸ <wherein R²⁷ and R²⁸ are the same or different, and represent
   a hydrogen atom, hydroxy, amino,
   substituted or unsubstituted lower alkoxy (the substituent in the substituted lower alkoxy has the same meaning as that of the aforementioned substituent (a) in the substituted cycloalkyl),
   substituted or unsubstituted lower alkylamino (the substituent in the substituted lower alkylamino has the same meaning as that of the aforementioned substituent (a) in the substituted cycloalkyl),
   substituted or unsubstituted di-(lower alkyl)amino (the substituent in said substituted di-(lower alkyl)amino has the same meaning as that of the aforementioned substituent (a) in the substituted cycloalkyl), substituted or unsubstituted lower alkyl {the substituent (c) in said substituted lower alkyl may be 1 to 3 substituent(s), and includes
   halogen, hydroxy, oxo, nitro, azido, cyano, carboxy,
   substituted or unsubstituted cycloalkyl (the substituent in the substituted cycloalkyl has the same meaning as that of the aforementioned substituent (a) in the substituted cycloalkyl),
   substituted or unsubstituted aryl (the substituent in the substituted aryl has the same meaning as that of the after-mentioned substituent (xi) in the substituted aryl),
   a substituted or unsubstituted heterocyclic group (the substituent in the substituted heterocyclic group has the same meaning as that of the after-mentioned substituent (xii) in the substituted heterocyclic group), substituted or unsubstituted lower alkoxy (the substituent in the substituted lower alkoxy has the same meaning as that of the aforementioned substituent (a) in the substituted cycloalkyl),
   substituted or unsubstituted lower alkylthio (the substituent in the substituted lower alkylthio has the same meaning as that of the aforementioned substituent (a) in the substituted cycloalkyl),
   substituted or unsubstituted lower alkanoyl (the substituent in the substituted lower alkanoyl has the same meaning as that of the aforementioned substituent (a) in the substituted cycloalkyl),
   substituted or unsubstituted lower alkoxycarbonyl (the substituent in said substituted lower alkoxycarbonyl has the same meaning as that of the aforementioned substituent (a) in the substituted cycloalkyl),
   -O(CH₂CH₂O)ₚR²⁹ (wherein p is an integer of 1 to 15, and R²⁹ represents a hydrogen atom, or lower alkyl),
   -NR³⁰R³¹ [wherein R³⁰ and R³¹ are the same or different, and represent a hydrogen atom, hydroxy, amino, lower alkylamino, di-(lower alkyl)amino,
   substituted or unsubstituted lower alkoxy (the substituent in the substituted lower alkoxy has the same meaning as that of the aforementioned substituent (a) in the substituted cycloalkyl),
   substituted or unsubstituted lower alkyl (the substituent in the substituted lower alkyl has the same meaning as that of the aforementioned substituent (a) in the substituted cycloalkyl),
   substituted or unsubstituted lower alkenyl (the substituent in the substituted lower alkenyl has the same meaning as that of the aforementioned substituent (a) in the substituted cycloalkyl),
   substituted or unsubstituted lower alkynyl (the substituent in the substituted lower alkynyl has the same meaning as that of the aforementioned substituent (a) in the substituted cycloalkyl),
   substituted or unsubstituted cycloalkyl (the substituent in the substituted cycloalkyl has the same meaning as that of the aforementioned substituent (a) in the substituted cycloalkyl),
   substituted or unsubstituted aryl (the substituent in the substituted aryl has the same meaning as that of the after-mentioned substituent (xi) in the substituted aryl),
   a substituted or unsubstituted heterocyclic group (the substituent in the substituted heterocyclic group has the same meaning as that of the after-mentioned substituent (xii) in the substituted heterocyclic group), substituted or unsubstituted lower alkanoyl (the substituent in the substituted lower alkanoyl has the same meaning as that of the aforementioned substituent (a) in the substituted cycloalkyl), substituted or unsubstituted lower alkoxycarbonyl (the substituent in said substituted lower alkoxycarbonyl has the same meaning as that of the aforementioned substituent (a) in the substituted cycloalkyl), substituted or unsubstituted lower alkylsulfonyl (the substituent in the substituted lower alkylsulfonyl has the same meaning as that of the aforementioned substituent (a) in the substituted cycloalkyl), substituted or unsubstituted aroyl (the substituent in the substituted aroyl has the same meaning as that of the after-mentioned substituent (xi) in the substituted aryl),
   substituted or unsubstituted aryloxycarbonyl (the substituent in said substituted aryloxycarbony has the same meaning as that of the after-mentioned substituent (xi) in the substituted aryl), or
   substituted or unsubstituted aralkyl (the substituent in the substituted aralkyl has the same meaning as that of the after-mentioned substituent (xi) in the substituted aryl), or
   R³⁰ and R³¹ are combined together with the adjacent nitrogen atom to form a substituted or unsubstituted heterocyclic group (the substituent in the heterocyclic group formed together with the adjacent nitrogen atom has the same meaning as that of the after-mentioned substituent (xii) in the substituted heterocyclic group)],
   -CONR³²R³³ (wherein R³² and R³³have the same meanings as those of the aforementioned R³⁰ and R³¹, respectively),
   -SO₂R³⁴ [wherein R³⁴ represents
   substituted or unsubstituted lower alkyl (the substituent in the substituted lower alkyl has the same meaning as that of the aforementioned substituent (a) in the substituted cycloalkyl),
   substituted or unsubstituted lower alkenyl (the substituent in the substituted lower alkenyl has the same meaning as that of the aforementioned substituent (a) in the substituted cycloalkyl),
   substituted or unsubstituted lower alkynyl (the substituent in the substituted lower alkynyl has the same meaning as that of the aforementioned substituent (a) in the substituted cycloalkyl),
   substituted or unsubstituted cycloalkyl (the substituent in the substituted cycloalkyl has the same meaning as that of the aforementioned substituent (a) in the substituted cycloalkyl),
   substituted or unsubstituted aryl (the substituent in the substituted aryl has the same meaning as that of the after-mentioned substituent (xi) in the substituted aryl),
   a substituted or unsubstituted heterocyclic group (the substituent in the substituted heterocyclic group has the same meaning as that of the after-mentioned substituent (xii) in the substituted heterocyclic group), substituted or unsubstituted lower alkoxy (the substituent in the substituted lower alkoxy has the same meaning as that of the aforementioned substituent (a) in the substituted cycloalkyl), or -NR35R36 (wherein R³⁵ and R³⁶ have the same meanings as those of the aforementioned R³⁰ and R³¹, respectively)],
   -N⁺R³⁷R³⁸R³⁹X· (wherein R³⁷ and R³⁸ are the same or different, and represent lower alkyl, or R³⁷ and R³⁸ are combined together with the adjacent nitrogen atom to form a heterocyclic group, R³⁹ represents lower alkyl, and X represents halogen), and the like},
   substituted or unsubstituted lower alkenyl (the substituent in the substituted lower alkenyl has the same meaning as that of the aforementioned substituent (c) in the substituted lower alkyl),
   substituted or unsubstituted lower alkynyl (the substituent in the substituted lower alkynyl has the same meaning as that of the aforementioned substituent (c) in the substituted lower alkyl),
   substituted or unsubstituted cycloalkyl (the substituent in the substituted cycloalkyl has the same meaning as that of the aforementioned substituent (c) in the substituted lower alkyl),
   substituted or unsubstituted aryl (the substituent in the substituted aryl has the same meaning as that of the after-mentioned substituent (xi) in the substituted aryl),
   a substituted or unsubstituted heterocyclic group (the substituent in the substituted heterocyclic group has the same meaning as that of the after-mentioned substituent (xii) in the substituted heterocyclic group), -COR⁴⁰ [wherein R⁴⁰ represents
   substituted or unsubstituted lower alkyl (the substituent in the substituted lower alkyl has the same meaning as that of the aforementioned substituent (c) in the substituted lower alkyl),
   substituted or unsubstituted lower alkenyl (the substituent in the substituted lower alkenyl has the same meaning as that of the aforementioned substituent (c) in the substituted lower alkyl),
   substituted or unsubstituted lower alkynyl (the substituent in the substituted lower alkynyl has the same meaning as that of the aforementioned substituent (c) in the substituted lower alkyl),
   substituted or unsubstituted cycloalkyl (the substituent in the substituted cycloalkyl has the same meaning as that of the aforementioned substituent (c) in the substituted lower alkyl),
   substituted or unsubstituted aryl (the substituent in the substituted aryl has the same meaning as that of the after-mentioned substituent (xi) in the substituted aryl),
   a substituted or unsubstituted heterocyclic group (the substituent in the substituted heterocyclic group has the same meaning as that of the after-mentioned substituent (xii) in the substituted heterocyclic group), substituted or unsubstituted lower alkoxy (the substituent in the substituted lower alkoxy has the same meaning as that of the aforementioned substituent (c) in the substituted lower alkyl),
   substituted or unsubstituted aryloxy (the substituent in the substituted aryloxy has the same meaning as that of the after-mentioned substituent (xi) in the substituted aryl), or
   substituted or unsubstituted heterocyclyloxy (the substituent in the substituted heterocyclyloxy has the same meaning as that of the after-mentioned substituent (xiii) in the substituted heterocyclic ring)],
   -CONR⁴¹R⁴² (wherein R⁴¹ and R⁴² have the same meanings as those of the aforementioned R³⁰ and R³¹, respectively), or
   -SO₂R⁴³ [wherein R⁴³ represents
   substituted or unsubstituted lower alkyl (the substituent in the substituted lower alkyl has the same meaning as that of the aforementioned substituent (c) in the substituted lower alkyl),
   substituted or unsubstituted lower alkenyl (the substituent in the substituted lower alkenyl has the same meaning as that of the aforementioned substituent (c) in the substituted lower alkyl),
   substituted or unsubstituted lower alkynyl (the substituent in the substituted lower alkynyl has the same meaning as that of the aforementioned substituent (c) in the substituted lower alkyl),
   substituted or unsubstituted cycloalkyl (the substituent in the substituted cycloalkyl has the same meaning as that of the aforementioned substituent (c) in the substituted lower alkyl),
   substituted or unsubstituted aryl (the substituent in the substituted aryl has the same meaning as that of the after-mentioned substituent (xi) in the substituted aryl),
   substituted or unsubstituted heterocyclic group (the substituent in the substituted heterocyclic group has the same meaning as that of the after-mentioned substituent (xii) in the substituted heterocyclic group), substituted or unsubstituted lower alkoxy (the substituent in the substituted lower alkoxy has the same meaning as that of the aforementioned substituent (c) in the substituted lower alkyl), or
   -NR44R45 (wherein R⁴⁴ and R⁴⁵ have the same meanings as those of the aforementioned R³⁰ and R³¹, respectively)], or
   R²⁷ and R²⁸ are combined together with the adjacent nitrogen atom to form a substituted or unsubstituted heterocyclic group (the substituent in the heterocyclic group formed together with the adjacent nitrogen atom has the same meaning as that of the after-mentioned substituent (xii) in the substituted heterocyclic group)>,
   -CONR⁴⁶R⁴⁷ (wherein R⁴⁶ and R⁴⁷ have the same meanings as those of the aforementioned R²⁷ and R²⁸, respectively),
   -COR⁴⁸ [wherein R⁴⁸ represents
   substituted or unsubstituted lower alkyl (the substituent in the substituted lower alkyl has the same meaning as that of the aforementioned substituent (c) in the substituted lower alkyl),
   substituted or unsubstituted lower alkenyl (the substituent in the substituted lower alkenyl has the same meaning as that of the aforementioned substituent (c) in the substituted lower alkyl),
   substituted or unsubstituted lower alkynyl (the substituent in the substituted lower alkynyl has the same meaning as that of the aforementioned substituent (c) in the substituted lower alkyl),
   substituted or unsubstituted cycloalkyl (the substituent in the substituted cycloalkyl has the same meaning as that of the aforementioned substituent (c) in the substituted lower alkyl),
   substituted or unsubstituted aryl (the substituent in the substituted aryl has the same meaning as that of the after-mentioned substituent (xi) in the substituted aryl), or
   a substituted or unsubstituted heterocyclic group (the substituent in the substituted heterocyclic group has the same meaning as that of the after-mentioned substituent (xii) in the substituted heterocyclic group)],
   -COOR⁴⁹ (wherein R⁴⁹ has the same meaning as that of the aforementioned R⁴⁸),
   -SO₂R⁵⁰ [wherein R⁵⁰ represents
   substituted or unsubstituted lower alkyl (the substituent in the substituted lower alkyl has the same meaning as that of the aforementioned substituent (c) in the substituted lower alkyl),
   substituted or unsubstituted lower alkenyl (the substituent in the substituted lower alkenyl has the same meaning as that of the aforementioned substituent (c) in the substituted lower alkyl),
   substituted or unsubstituted lower alkynyl (the substituent in the substituted lower alkynyl has the same meaning as that of the aforementioned substituent (c) in the substituted lower alkyl),
   substituted or unsubstituted cycloalkyl (the substituent in the substituted cycloalkyl has the same meaning as that of the aforementioned substituent (c) in the substituted lower alkyl),
   substituted or unsubstituted aryl (the substituent in the substituted aryl has the same meaning as that of the after-mentioned substituent (xi) in the substituted aryl),
   a substituted or unsubstituted heterocyclic group (the substituent in the substituted heterocyclic group has the same meaning as that of the after-mentioned substituent (xii) in the substituted heterocyclic group), substituted or unsubstituted lower alkoxy (the substituent in the substituted lower alkoxy has the same meaning as that of the aforementioned substituent (c) in the substituted lower alkyl), or
   -NR⁵¹R⁵² (wherein R⁵¹ and R⁵² have the same meanings as those of the aforementioned R³⁰ and R³¹, respectively)],
   -OR⁵³ [wherein R⁵³ represents
   substituted or unsubstituted lower alkyl (the substituent in the substituted lower alkyl has the same meaning as that of the aforementioned substituent (c) in the substituted lower alkyl),
   substituted or unsubstituted lower alkenyl (the substituent in the substituted lower alkenyl has the same meaning as that of the aforementioned substituent (c) in the substituted lower alkyl),
   substituted or unsubstituted lower alkynyl (the substituent in the substituted lower alkynyl has the same meaning as that of the aforementioned substituent (c) in the substituted lower alkyl),
   substituted or unsubstituted cycloalkyl (the substituent in the substituted cycloalkyl has the same meaning as that of the aforementioned substituent (c) in the substituted lower alkyl),
   substituted or unsubstituted aryl (the substituent in the substituted aryl has the same meaning as that of the after-mentioned substituent (xi) in the substituted aryl),
   a substituted or unsubstituted heterocyclic group (the substituent in the substituted heterocyclic group has the same meaning as that of the after-mentioned substituent (xii) in the substituted heterocyclic group), -COR⁵⁴ (wherein R⁵⁴ has the same meaning as that of the aforementioned R⁵⁰), -SO₂R⁵⁵ (wherein R⁵⁵ has the same meaning as that of the aforementioned R⁵⁰), or -SiR⁵⁶R⁵⁷R⁵⁸ (wherein R⁵⁶, R⁵⁷ and R⁵⁸ are the same or different, and represent a hydrogen atom, hydroxy, lower alkyl, or lower alkoxy)],
   -SR⁵⁹ (wherein R⁵⁹ has the same meaning as that of the aforementioned R⁵³),
   -N⁺R⁶⁰R⁶¹R⁶²X ¹⁻ (wherein R⁶⁰, R⁶¹, R⁶² and X¹ have the same meanings as those of the aforementioned R³⁷, R³⁸, R³⁹ and X, respectively), and the like.

Herein, the lower alkyl moiety in the lower alkyl, the lower alkoxy, the lower alkylthio, the lower alkylamino, the di-(lower alkyl)amino, the lower alkoxycarbonyl, the lower alkanoyl, the lower alkoxy-substituted lower alkoxy, the lower alkoxy-substituted lower alkylamino, and the lower alkylsulfonyl, the lower alkenyl, the lower alkynyl, and the cycloalkyl have the same meanings as those of the aforementioned lower alkyl (i), lower alkenyl (ii), lower alkynyl (iii), and cycloalkyl (iv), respectively, and the alkylene moiety in the hydroxy-substituted lower alkoxy, the amino-substituted lower alkoxy, the lower alkoxy-substituted lower alkoxy, the hydroxy-substituted lower alkylamino, the amino-substituted lower alkylamino, and the lower alkoxy-substituted lower alkylamino has the same meaning as that of the aforementioned lower alkyl (i) from which one hydrogen atom is removed. Two of the lower alkyl moieties in the di-(lower alkyl)amino may be the same or different. Also herein, the aryl moiety in the aryl, the aryloxy, the aryloxycarbonyl, and the aroyl, the heterocyclic group moiety in the heterocyclic group and the heterocyclyloxy, and the heterocyclic group formed together with the adjacent nitrogen atom have the same meanings as those of the aforementioned aryl (v), heterocyclic group (vi) and the heterocyclic group formed together with the adjacent nitrogen atom (vii), respectively, and examples of the aralkyl moiety (ix) in the aralkyl and the aralkyloxy mentioned here include aralkyl having 7 to 15 carbon atoms, for example, benzyl, phenethyl, benzhydryl, naphthylmethyl and the like. The halogen (x) means each atom of fluorine, chlorine, bromine and iodine.
(xi) The substituent in the substituted aryl and substituted aryloxy may be the same or different in number of 1 to 3 substituent(s), and includes halogen, hydroxy, nitro, cyano, azido, methylenedioxy,
   substituted or unsubstituted lower alkyl (the substituent in the substituted lower alkyl has the same meaning as that of the aforementioned substituent (a) in the cycloalkyl),
   substituted or unsubstituted lower alkenyl (the substituent in the substituted lower alkenyl has the same meaning as that of the aforementioned substituent (a) in the cycloalkyl),
   substituted or unsubstituted lower alkynyl (the substituent in the substituted lower alkynyl has the same meaning as that of the aforementioned substituent (a) in the cycloalkyl),
   substituted or unsubstituted cycloalkyl (the substituent in the substituted cycloalkyl has the same meaning as that of the aforementioned substituent (a) in the cycloalkyl), substituted or unsubstituted aryl (the substituents (d) in said substituted aryl may be the same or different in number of 1 to 3 substituent(s), and includes
   halogen, hydroxy, amino, nitro, azido, cyano, carboxy, lower alkoxy, lower alkylthio, lower alkylamino, di-(lower alkyl)amino, lower alkanoyl, lower alkanoyloxy, lower alkanoylamino, methylenedioxy, aryl, a heterocyclic group, and the like),
   a substituted or unsubstituted heterocyclic group (the substituent in the substituted heterocyclic group has the same meaning as that of the aforementioned substituent (d) in the substituted aryl),
   -COR⁶³ [wherein R⁶³ represents
   a hydrogen atom,
   substituted or unsubstituted lower alkyl (the substituent in the substituted lower alkyl has the same meaning as that of the aforementioned substituent (a) in the cycloalkyl),
   substituted or unsubstituted lower alkenyl (the substituent in the substituted lower alkenyl has the same meaning as that of the aforementioned substituent (a) in the cycloalkyl),
   substituted or unsubstituted lower alkynyl (the substituent in the substituted lower alkynyl has the same meaning as that of the aforementioned substituent (a) in the cycloalkyl),
   substituted or unsubstituted cycloalkyl (the substituent in the substituted cycloalkyl has the same meaning as that of the aforementioned substituent (a) in the cycloalkyl),
   substituted or unsubstituted aryl (the substituent in the substituted aryl has the same meaning as that of the aforementioned substituent (d) in the substituted aryl), or
   a substituted or unsubstituted heterocyclic group (the substituent in the substituted heterocyclic group has the same meaning as that of the aforementioned substituent (d) in the substituted aryl)],
   -COOR⁶⁴ (wherein R⁶⁴ has the same meaning as that of the aforementioned R⁶³),
   -OR⁶⁵ [wherein R⁶⁵ represents
   substituted or unsubstituted lower alkyl (the substituent in the substituted lower alkyl has the same meaning as that of the aforementioned substituent (a) in the cycloalkyl),
   substituted or unsubstituted lower alkenyl (the substituent in the substituted lower alkenyl has the same meaning as that of the aforementioned substituent (a) in the cycloalkyl),
   substituted or unsubstituted lower alkynyl (the substituent in the substituted lower alkynyl has the same meaning as that of the aforementioned substituent (a) in the cycloalkyl),
   substituted or unsubstituted cycloalkyl (the substituent in the substituted cycloalkyl has the same meaning as that of the aforementioned substituent (a) in the cycloalkyl),
   substituted or unsubstituted lower alkanoyl (the substituent in the substituted lower alkanoyl has the same meaning as that of the aforementioned substituent (a) in the cycloalkyl),
   tri-(lower alkyl)silyl,
   substituted or unsubstituted aryl (the substituent in the substituted aryl has the same meaning as that of the aforementioned substituent (d) in the substituted aryl),
   substituted or unsubstituted aralkyl (the substituent in the substituted aralkyl has the same meaning as that of the aforementioned substituent (d) in the substituted aryl), or
   a substituted or unsubstituted heterocyclic group (the substituent in the substituted heterocyclic group has the same meaning as that of the aforementioned substituent (d) in the substituted aryl)],
   -SR⁶⁶ (wherein R⁶⁶ has the same meaning as that of the aforementioned R⁶⁵),
   -NR⁶⁷R⁶⁸ [wherein R⁶⁷ and R⁶⁸ are the same or different, and represent a hydrogen atom, hydroxy,
   substituted or unsubstituted lower alkyl (the substituent in the substituted lower alkyl has the same meaning as that of the aforementioned substituent (a) in the cycloalkyl),
   substituted or unsubstituted lower alkenyl (the substituent in the substituted lower alkenyl has the same meaning as that of the aforementioned substituent (a) in the cycloalkyl),
   substituted or unsubstituted lower alkynyl (the substituent in the substituted lower alkynyl has the same meaning as that of the aforementioned substituent (a) in the cycloalkyl),
   substituted or unsubstituted cycloalkyl (the substituent in the substituted cycloalkyl has the same meaning as that of the aforementioned substituent (a) in the cycloalkyl),
   substituted or unsubstituted lower alkoxy (the substituent in the substituted lower alkoxy has the same meaning as that of the aforementioned substituent (a) in the cycloalkyl),
   substituted or unsubstituted lower alkanoyl (the substituent in the substituted lower alkanoyl has the same meaning as that of the aforementioned substituent (a) in the cycloalkyl),
   substituted or unsubstituted lower alkylsulfonyl (the substituent in the substituted lower alkylsulfonyl has the same meaning as that of the aforementioned substituent (a) in the cycloalkyl),
   substituted or unsubstituted aryl (the substituent in the substituted aryl has the same meaning as that of the aforementioned substituent (d) in the substituted aryl),
   substituted or unsubstituted aralkyl (the substituent in the substituted aralkyl has the same meaning as that of the aforementioned substituent (d) in the substituted aryl),
   substituted or unsubstituted aroyl (the substituent in the substituted aroyl has the same meaning as that of the aforementioned substituent (d) in the substituted aryl), or
   a substituted or unsubstituted heterocyclic group (the substituent in the substituted heterocyclic group has the same meaning as that of the aforementioned substituent (d) in the substituted aryl), or
   R⁶⁷ and R⁶⁸ are combined together with the adjacent nitrogen atom to form a substituted or unsubstituted heterocyclic group (the substituent in the heterocyclic group formed together with the adjacent nitrogen atom has the same meaning as that of the aforementioned substituent (d) in the aryl)],
   -CONR⁶⁹R⁷⁰(wherein R⁶⁹ and R⁷⁰ have the same meanings as those of the aforementioned R⁶⁷ and R⁶⁸, respectively),
   -SO₂R⁷¹ [wherein R⁷¹ represents,
   substituted or unsubstituted lower alkyl (the substituent in the substituted lower alkyl has the same meaning as that of the aforementioned substituent (a) in the cycloalkyl),
   substituted or unsubstituted lower alkenyl (the substituent in the substituted lower alkenyl has the same meaning as that of the aforementioned substituent (a) in the cycloalkyl),
   substituted or unsubstituted lower alkynyl (the substituent in the substituted lower alkynyl has the same meaning as that of the aforementioned substituent (a) in the cycloalkyl),
   substituted or unsubstituted cycloalkyl (the substituent in the substituted cycloalkyl has the same meaning as that of the aforementioned substituent (a) in the cycloalkyl),
   substituted or unsubstituted lower alkoxy (the substituent in the substituted lower alkoxy has the same meaning as that of the aforementioned substituent (a) in the cycloalkyl),
   substituted or unsubstituted aryl (the substituent in the substituted aryl has the same meaning as that of the aforementioned substituent (d) in the substituted aryl),
   substituted or unsubstituted aralkyl (the substituent in the substituted aralkyl has the same meaning as that of the aforementioned substituent (d) in the substituted aryl),
   substituted or unsubstituted aroyl (the substituent in the substituted aroyl has the same meaning as that of the aforementioned substituent (d) in the substituted aryl),
   a substituted or unsubstituted heterocyclic group (the substituent in the substituted heterocyclic group has the same meaning as that of the aforementioned substituent (d) in the substituted aryl), or
   -NR⁷²R⁷³ (wherein R⁷² and R⁷³ have the same meanings as those of the aforementioned R⁶⁷ and R⁶⁸, respectively)], and the like.

Herein, the lower alkyl moiety in the lower alkyl, the lower alkoxy, the lower alkylthio, the lower alkylamino, the di-(lower alkyl)amino, the tri-(lower alkyl)silyl, the lower alkanoyl, the lower alkanoyloxy, the lower alkanoylamino, and the lower alkylsulfonyl, the lower alkenyl, the lower alkynyl, the cycloalkyl, and the halogen have the same meanings as those of the aforementioned lower alkyl (i), lower alkenyl (ii), lower alkynyl (iii), cycloalkyl (iv), and halogen (x), respectively, and two of the lower alkyl moieties in the di-(lower alkyl)amino and three of the lower alkyl moieties in the tri-(lower alkyl)silyl may be the same or different, respectively. Also herein, the aryl moiety in the aryl and the aroyl, the heterocyclic group, the heterocyclic group formed together with the adjacent nitrogen atom, and the aralkyl have the same meanings as those of the aforementioned aryl (v), heterocyclic group (vi), heterocyclic group formed together with the adjacent nitrogen atom (vii), and aralkyl (ix), respectively.
(xii) The substituent in the substituted heterocyclic group, the substituted heterocyclylcarbonyl group and the substituted heterocyclic group formed together with the adjacent nitrogen atom includes oxo and the like as well as the groups mentioned in the definition of the aforementioned substituent (xi) in the substituted aryl.

Example of the pharmacologically acceptable salt of Compound (I) include pharmacologically acceptable acid addition salts, metal salts, ammonium salts, organic amine addition salts, amino acid addition salts and the like. Examples of the pharmacologically acceptable acid addition salt of Compound (I) include an inorganic acid addition salt such as hydrochloride, sulfate and phosphate, an organic acid addition salt such as acetate, maleate, fumarate and citrate, and the like. Examples of the pharmacologically acceptable metal salt include an alkali metal salt such as a sodium salt and a potassium salt, an alkaline-earth metal salt such as a magnesium salt and a calcium salt, an aluminium salt, a zinc salt and the like. Examples of the pharmacologically acceptable ammonium salt include a salt of ammonium, tetramethylammonium or the like. Examples of the pharmacologically acceptable organic amine addition salt include an addition salt of morpholine, piperidine or the like. Examples of the pharmacologically acceptable amino acid addition salt include an addition salt of lysine, glycine, phenylalanine, aspartic acid, glutamic acid or the like.

Next, the methods of preparing the Compound (I) are described as follows.

In the preparing methods as shown below, when the defined group changes under the conditions of the method carried out, or is inappropriate for carrying out the methods, the desired compound can be obtained by using the protection and deprotection methods which are ordinarily used in the organic synthetic chemistry [e.g., Protective Groups in Organic Synthesis, T. W. Greene, John Wiley & Sons Inc. (1981)] and the like. In addition, the order of the steps for introducing a substituent and the like may be changed, if necessary.

Compound (I) can be prepared according to the following preparing methods.

### Preparing method 1

Compound (I) can be prepared from Compound (III) and Compound (IV) via Compound (V) by known methods [e.g., J. Bangladesh Chem. Soc., Vol. 5, p.127 (1992); J. Org. Chem., Vol. 45, p.1473 (1980), Patent of East Germany No. 243930, and the like], or the methods similar to the known methods. Herein, the starting materials, Compounds (III), (IV), (VIa) and (VIb) can be prepared as commercial products, or can be prepared by known methods [e.g., methods described in Shin-Jikken-Kagaku-Koza Vol. 14, p.751 (Maruzen, 1978); Shin-Jikken-Kagaku-Koza Vol. 14, p.1621 (Maruzen, 1978); Shin-Jikken-Kagaku-Koza Vol. 14, p.1104 and p.1120 (Maruzen, 1978) and the like], or the methods similar to the known methods. 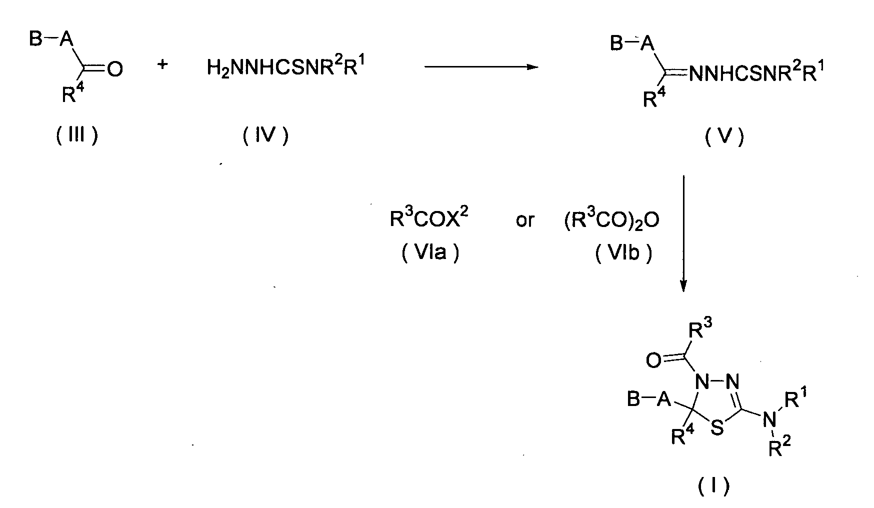 (wherein R¹, R², R³, R⁴, A and B have the same meanings as those mentioned above, respectively, and X² represents a chlorine atom, or a bromine atom).

### Preparing method 2

Among Compound (I), Compound (Ia) wherein R² is -COR⁵ (wherein R⁵ has the same meaning as that mentioned above), and R³ corresponds to R⁵ in R² can also be prepared from Compound (III) and Compound (IVa) via Compound (Va) by known methods [e.g., J. Bangladesh Chem. Soc., Vol. 5, p.127 (1992); J. Org. Chem., Vol. 45, p.1473 (1980), Patent of East Germany No. 243930, and the like], or the methods similar to the known methods. The starting compounds, Compounds (III), (IVa), (VIIa) and (VIIb), can be prepared as commercial products, or can be prepared by known methods [e.g., methods described in Shin-Jikken-Kagaku-Koza Vol. 14, p.751 (Maruzen, 1978); Shin-Jikken-Kagaku-Koza Vol. 14, p.1621 (Maruzen, 1978); Shin-Jikken-Kagaku-Koza Vol. 14, p. 1104 and p.1120 (Maruzen, 1978) and the like], or the methods similar to the known methods: 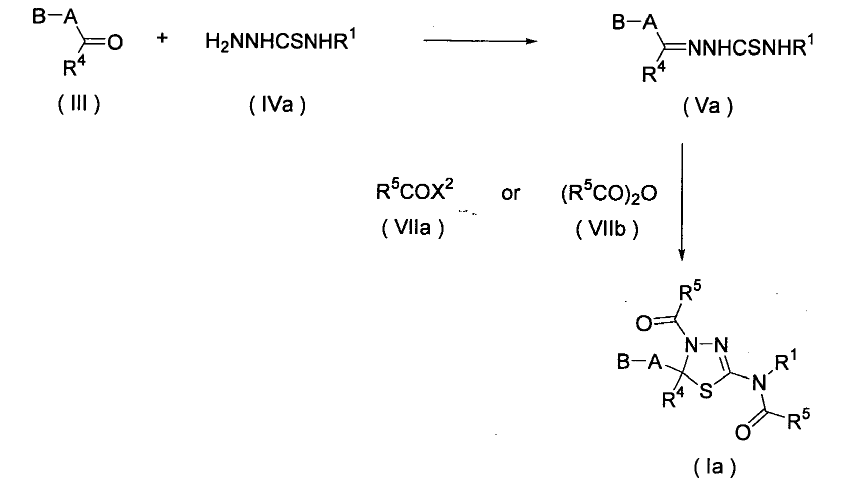 (wherein R¹, R⁴, R⁵, X², A and B have the same meanings as those mentioned above, respectively).

### Preparing method 3

Among Compound (I), Compound (Ib) wherein R² is -COR⁵ (wherein R⁵ has the same meaning as that mentioned above) can also be prepared in accordance with the following step: 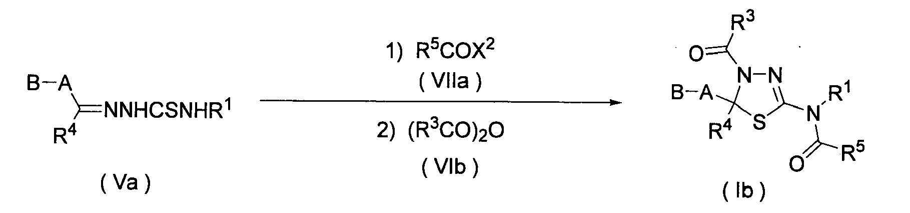 (wherein R¹, R³, R⁴, R⁵, A, B and X² have the same meanings as those mentioned above, respectively) .

Compound (Ib) can be obtained by the reaction of Compound (Va) obtained in the preparing method 1 or 2 with Compound (VIIa) in an inert solvent, for example, acetone, dimethylformamide (DMF) and the like, in the presence of an appropriate base such as 2,6-di-tert-butyl-4-methylpyridine, generally at a temperature between -78°C and 100°C, preferably at a temperature between -10°C and 30°C, for 5 minutes to 24 hours, and then the following reaction with Compound (VIb) for 10 to 48 hours after addition of an appropriate base such as pyridine. Compound (VIIa), Compound (VIb), the appropriate base used in the first step, and the appropriate base used in the following step are preferably used in amounts of 1 to 5 equivalents, 1 to 5 equivalents, 0.5 to 2 equivalents, and 1 to 5 equivalents, respectively, to Compound (Va).

### Preparing method 4

Among Compound (I), Compound (Ic) or (Id) wherein R² is -COR⁵ (wherein R⁵ has the same meaning as that mentioned above), A is -(CH₂)ₙ- (wherein n has the same meaning as that mentioned above), and B is tert-butoxycarbonylamino can be prepared from Compound (VIII) in the same manner as those in the preparing methods 1 to 3. The starting compound, Compound (VIII), can be prepared by known methods [e.g., the methods described in, for example, J. Med. Chem., Vol. 41, p.591 (1998); Angew. Chem. Int. Ed., Vol. 40, p.3458 (2001) and the like], or the methods similar to the known methods: 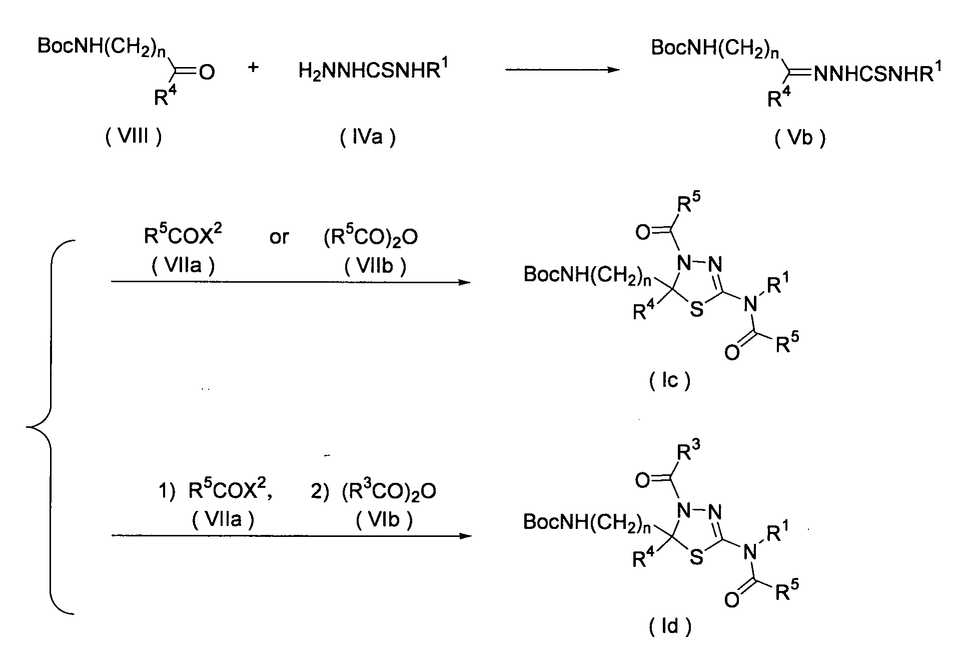 (wherein n, R¹, R³, R⁴, R⁵ and X² have the same meanings as those mentioned above, respectively, and Boc represents tert-butoxycarbonyl.)

### Preparing method 5

Among Compound (I), Compound (If) wherein A is -(CH₂)ₙ- (wherein n has the same meaning as that mentioned above), and B is NH₂ can also be prepared by treatment of Compound (Ie) obtained in the preparing methods 1 to 4 with the deprotection condition ordinarily used in the organic synthetic chemistry, for example, those by the methods described in Protective Groups in Organic Synthesis, T.W. Greene, John Wiley & Sons Inc., 1981 and the like, or the methods similar to the thereof: 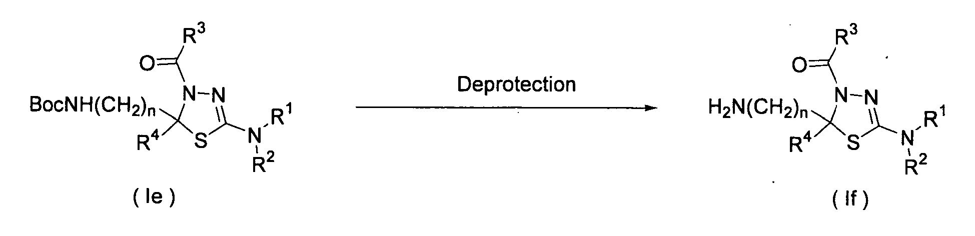 (wherein n, R¹, R², R³, R⁴, and Boc have the same meanings as those mentioned above, respectively.)

### Preparing method 6

Among Compound (I), Compound (Ig) wherein A is -(CH₂)ₙ- (wherein n has the same meaning as that mentioned above), and B is -NHCOR⁸ (wherein R⁸ has the same meaning as that mentioned above) or -NHCOR¹⁶ (wherein R¹⁶ has the same meaning as that mentioned above) can be prepared from Compound (If) obtained in the preparing methods 1 to 3 or 5 in accordance with the following step: 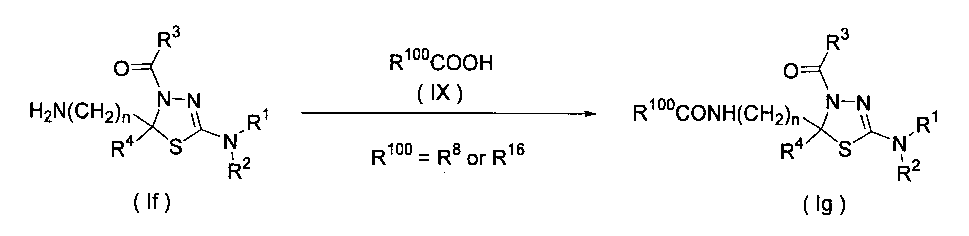 (wherein n, R¹, R², R³ and R⁴ have the same meanings as those mentioned above, respectively, and R¹⁰⁰ represents R⁸ or R¹⁶, which has the same meanings as that mentioned above.)

Compound (Ig) can be prepared by the reaction of Compound (If) with Compound (IX) in an inert solvent such as DMF in the presence of an appropriate condensing agent such as 1-ethyl-3-(3'-dimethylaminopropyl)carbodiimide hydrochloride and an appropriate activating agent such as 1-hydroxybenzotriazole monohydrate generally at a temperature between -78°C and 100°C, preferably at a temperature between 0°C and 50°C, for 5 minutes to 48 hours. Compound (IX), the appropriate condensing agent, and the appropriate activating agent are preferably used in amounts of 1 to 10 equivalents to Compound (If), respectively.

### Preparing method 7

Among Compound (I), Compound (Ih) wherein A is -(CH₂)ₙ- (wherein n has the same meaning as that mentioned above), and B is NR⁶R⁷ (wherein R⁶ and R⁷ have the same meanings as those mentioned above, respectively) or NR¹⁴R¹⁵ (wherein R¹⁴ and R¹⁵ have the same meanings as those mentioned above, respectively) can also be prepared from Compound (X) via Compound (XI) prepared in the same manner as those in the preparing methods 1 to 3 in accordance with the following step. The starting compound, Compound (X), can be prepared as a commercial product, or can be prepared by known methods [e.g., methods described in Shin-Jikken-Kagaku-Koza Vol. 14, p.1000 (Maruzen, 1978) and the like], or the methods similar to the known methods: 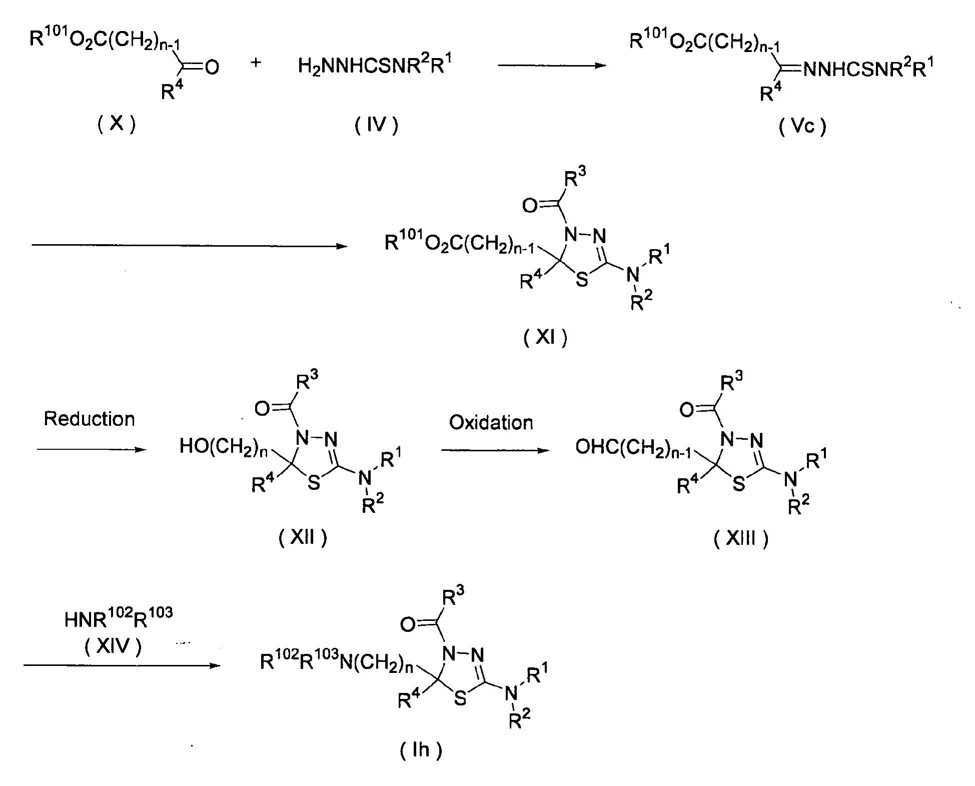 (wherein n, R¹, R², R³ and R⁴ have the same meanings as those mentioned above, respectively, R¹⁰¹ represents lower alkyl such as methyl and ethyl, and R¹⁰² and R¹⁰³ have the same meanings as those of R⁶ and R⁷, or R¹⁴ and R¹⁵ mentioned above, respectively.)

Compound (XII) can be prepared by treatment of Compound (XI) in an inert solvent such as tetrahydrofuran (THF), toluene and hexane in the presence of an appropriate reducing agent such as diisobutylaluminum hydride at a temperature between -78°C and 100°C, preferably at a temperature between -78°C and 30°C, for 5 minutes to 80 hours. The appropriate reducing agent is preferably used in an amount of 1 to 10 equivalents to Compound (XI).

Compound (XIII) can be prepared by treatment of Compound (XII) prepared above in an inert solvent such as dichloromethane, 1,2-dichloroethane and toluene in the presence of an appropriate oxidizing agent such as pyridinium dichromate at a temperature between -78°C and 100°C, preferably at a temperature between 0°C and 50°C, for 5 minutes to 72 hours. The appropriate oxidizing agent is preferably used in an amount of 1 to 10 equivalents to Compound (XII).

Compound (Ih) can be obtained by the reaction of Compound (XIII) prepared above with Compound (XIV) in an inert solvent such as dichloromethane, 1,2-dichloroethane and toluene in the presence of an appropriate reducing agent such as triacetoxy sodium borohydride and an appropriate acid such as acetic acid at a temperature between -78°C and 100°C, preferably at a temperature between 0°C and 50°C, for 5 minutes to 48 hours. Compound (XIV), the appropriate acid and the appropriate reducing agent are preferably used in amounts of 1 to 10 equivalents to Compound (XIII), respectively.

### Preparing method 8

Among Compound (I), Compound (Ie) wherein A is -(CH₂)ₙ- (wherein n has the same meaning as that mentioned above), and B is tert-butoxycarbonylamino can also be prepared in accordance with the following step: 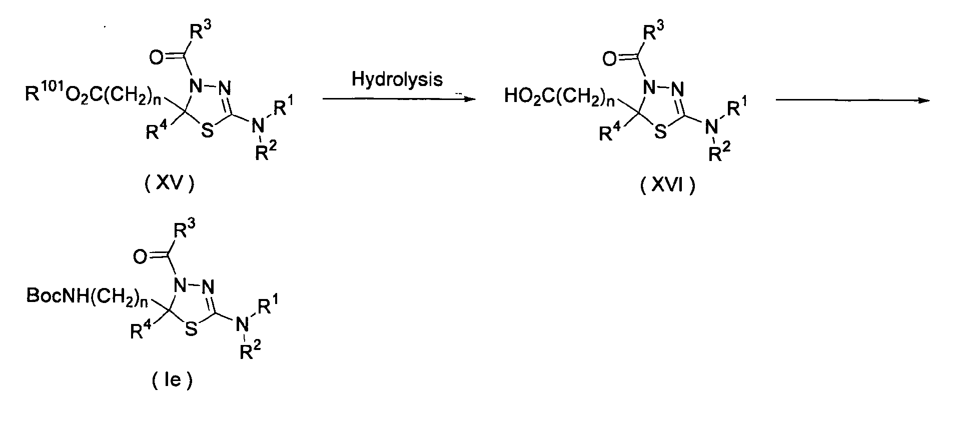 (wherein n, R¹, R², R³, R⁴, R¹⁰¹ and Boc have the same meanings as those mentioned above, respectively.)

Compound (XVI) can be prepared by treatment of Compound (XV) prepared in a manner similar to that in the preparing method 7 in an appropriate solvent containing water such as 1,4-dioxane/water in the presence of an appropriate base such as sodium hydroxide at a temperature between -10°C and 100°C for 5 minutes to 48 hours. The appropriate base is used in an amount of 0.3 to 100 equivalents to Compound (XV).

Compound (Ie) can be prepared by the reaction of Compound (XVI) prepared above with diphenylphosphoryl azide in tert-butanol in the presence of an appropriate base such as triethylamine generally at a temperature between -78°C and 140°C, preferably at a temperature between 0°C and 120°C, for 5 minutes to 48 hours. The appropriate base and diphenylphosphoryl azide are preferably used in amounts of 0.5 to 10 equivalents and 1 to 10 equivalents to Compound (XVI), respectively.

### Preparing method 9

Among Compound (I), Compound (Ii) wherein R² is a hydrogen atom can also be prepared in accordance with the following step: 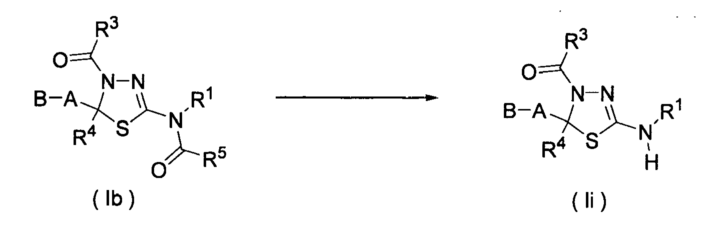 (wherein R¹, R³, R⁴, R⁵, A and B have the same meanings as those mentioned above, respectively).

Compound (Ii) can be prepared by treatment of Compound (Ib) prepared in the preparing methods 1 to 8 in an appropriate solvent in the presence of 1 to 200 equivalents, preferably 1 to 10 equivalents, of an appropriate base at a temperature between -10°C and the boiling point of the solvent used for 5 minutes to 24 hours.

Examples of the appropriate solvent include, for example, methanol, ethanol, tert-butanol, acetonitrile, dichloromethane, chloroform, ethyl acetate, THF, dioxane, toluene, xylene, DMF, N-methylpyrrolidone (NMP), pyridine, water and the like, and they can be used alone or as a mixture. Examples of the appropriate base include, for example, sodium hydride, sodium hydroxide, potassium hydroxide, lithium hydroxide, potassium carbonate, hydrazine monohydrate and the like.

As an alternative method, Compound (Ii) can also be prepared by treatment of Compound (Ib) in an appropriate solvent in the presence of 1 to 200 equivalents of an appropriate reducing agent, and if necessary, an appropriate additive, at a temperature between -10°C and 100°C for 5 minutes to 24 hours.

Examples of the appropriate solvent include, for example, methanol, ethanol, tert-butanol, acetonitrile, dichloromethane, THF, dioxane, toluene, xylene, water and the like, and they can be used alone or as a mixture. Examples of the appropriate reducing agent include, for example, sodium borohydride, triacetoxy sodium borohydride and the like, and examples of the appropriate additive include ceric chloride heptahydrate, hydrochloric acid-sodium acetate buffer and the like.

### Preparing method 10

Among Compound (I), Compound (Ik) wherein R¹ and R² are combined to form a substituted or unsubstituted heterocyclic group together with the adjacent nitrogen atom can be prepared in accordance with the following Steps 10-1 and 10-2: 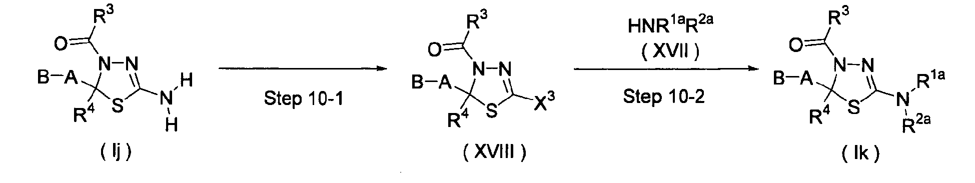 [wherein R³, R⁴, A and B have the same meanings as those mentioned above, respectively, X³ represents a chlorine atom, a bromine atom, or an iodine atom, and R^{1a} and R^{2a} are combined to form a substituted or unsubstituted heterocyclic group together with the adjacent nitrogen atom (said heterocyclic group has the same meaning as the aforementioned heterocyclic group formed together with the adjacent nitrogen atom (vii), and the substituent in said substituted heterocyclic group has the same meaning as the aforementioned substituent (xii) in the heterocyclic group).]

### Step 10-1

Compound (XVIII) can be prepared from Compound (Ij) prepared in the preparing methods 1 or 5 to 9 by the methods described in for example, Chem.

Commun., Vol. 8, p.873 (1998) and the like, or the methods similar thereto.

### Step 10-2

Compound (Ik) can be prepared by the reaction of Compound (XVIII) prepared in Step 10-1 mentioned above with 1 to 200 equivalents, preferably 2 to 50 equivalents of Compound (XVII), without solvent or in an inert solvent at a temperature between -10°C and 200°C for 5 minutes to 24 hours.

Examples of the inert solvent include, for example, acetonitrile, dichloromethane, chloroform, ethyl acetate, THF, dioxane, toluene, xylene, DMF, NMP, pyridine and the like, and they can be used alone or as a mixture.

Compound (VII) can be prepared as a commercial product, or can be prepared by the methods described in Shin-Jikken-Kagaku-Koza Vol. 14, p.1332 (Maruzen, 1978) and the like, or the methods similar to thereof.

As an alternative method, among Compound (Ik), Compound (In) wherein R^{1a} and R^{2a} are combined to form -CO(CH₂)_{q}- (wherein q represents an integer of 2 to 7) can also be prepared in accordance with Steps 10-3 and 10-4 mentioned below: 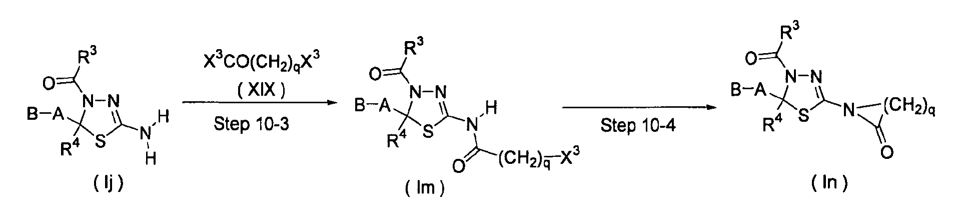 (wherein q, R³, R⁴, X³, A and B have the same meanings as those mentioned above, respectively.)

### Step 10-3

Compound (Im) can be prepared by the reaction of Compound (Ij) prepared in the preparing methods 1 or 5 to 9 with 1 to 30 equivalents of Compound (XIX) without solvent or in an appropriate solvent, if necessary, in the presence of 1 to 30 equivalents of an appropriate base, at a temperature between -30°C and 150°C for 5 minutes to 48 hours.

Examples of the appropriate solvent include, for example, dichloromethane, acetonitrile, toluene, ethyl acetate, pyridine, THF, DMF, and the like. Examples of the appropriate base include, for example, pyridine, triethylamine, diisopropylethylamine, potassium carbonate, potassium hydroxide, and the like.

### Step 10-4

Compound (In) can be prepared from Compound (Im) prepared in Step 10-3 mentioned above by the methods described in, for example, Shin-Jikken-Kagaku-Koza Vol. 14, p.1174 (Maruzen, 1978) and the like, or the methods similar thereto.

### Preparing method 11

Among Compound (I), Compound (Ip) wherein A is -(CH₂)ₙ- (wherein n has the same meaning as that mentioned above), and B is NHCONR⁹R¹⁰ (wherein R⁹ and R¹⁰ have the same meanings as those mentioned above, respectively) or NHCONR¹⁷R¹⁸ (wherein R¹⁷ and R¹⁸ have the same meanings as those mentioned above, respectively) can also be prepared in accordance with Steps 11-1 and 12-2 mentioned below: 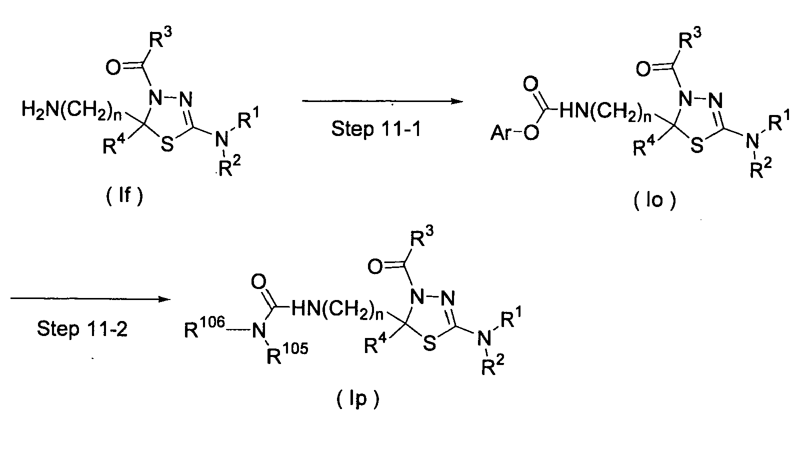 (wherein R¹, R², R³, R⁴ and n have the same meanings as those mentioned above, respectively, Ar represents phenyl, phenyl substituted with one or two nitro groups or phenyl substituted with one to three chlorine atoms, and R¹⁰⁵ and R¹⁰⁶ have the same meanings as those of R⁹ and R¹⁰, or R¹⁷ and R¹⁸ mentioned above, respectively.)

### Step 11-1

Compound (Io) can be prepared by the reaction of Compound (If) prepared in the preparing methods 1 to 3, 5, 9 or 10 with 1 to 30 equivalents of ArOCOCl (wherein Ar has the same meaning as that mentioned above) in an appropriate solvent, if necessary, in the presence of 1 to 30 equivalents of an appropriate base, at a temperature between -30°C and the boiling point of the solvent used for 5 minutes to 48 hours.

Examples of the appropriate solvent include, for example, dichloromethane, acetonitrile, toluene, ethyl acetate, pyridine, THF, DMF, and the like. Examples of the appropriate base include, for example, pyridine, triethylamine, diisopropylethylamine, potassium carbonate, potassium hydroxide, and the like. Examples of ArOCOC1 (wherein Ar has the same meaning as that mentioned above) include, for example, phenyl chloroformate, 4-nitrophenyl chloroformate, 2-nitrophenyl chloroformate, 2,4-dinitrophenyl chloroformate, 2,4-dichlorophenyl chloroformate, and the like

### Step 11-2

Compound (Ip) can be prepared by the reaction of Compound (Io) prepared in Step 11-1 mentioned above with 1 to 200 equivalents of a compound NHR¹⁰⁵R¹⁰⁶ (wherein R¹⁰⁵ and R¹⁰⁶ have the same meanings as those mentioned above, respectively) without solvent or in an appropriate solvent, if necessary, in the presence of 1 to 30 equivalents of an appropriate base, at a temperature between -30°C and 150°C for 5 minutes to 48 hours.

Examples of the appropriate solvent include, for example, dichloromethane, acetonitrile, toluene, ethyl acetate, pyridine, THF, DMF, and the like. Examples of the appropriate base include, for example, pyridine, triethylamine, diisopropylethylamine, potassium carbonate, potassium hydroxide, and the like.

### Preparing method 12

Among Compound (I), Compound (It) wherein A is -(CH₂)ₙ- (wherein n has the same meaning as that mentioned above), and B is ·WR¹¹ (wherein W represents an oxygen atom or a sulfur atom, and R¹¹ has the same meanings as that mentioned above) can also be prepared in accordance with the following step: 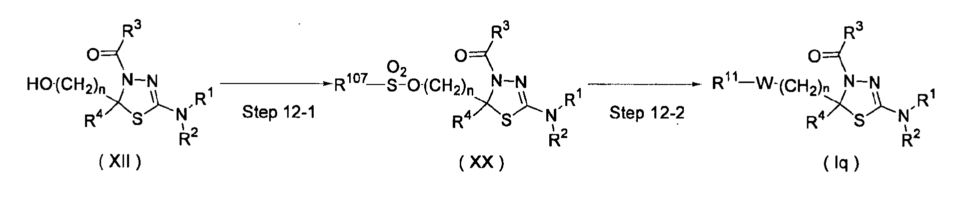 (wherein R¹, R², R³, R⁴, R¹¹ and n have the same meanings as those mentioned above, respectively, R¹⁰⁷ represents methyl, ethyl, isopropyl, phenyl, or p-tolyl, and W represents an oxygen atom or a sulfur atom.)

### Step 12-1

Compound (XX) can be prepared by the reaction of Compound (XII) prepared in the preparing method 7 with 1 to 30 equivalents of R¹⁰⁷SO₂Cl (wherein R¹⁰⁷ has the same meaning as that mentioned above) or (R¹⁰⁷SO₂)₂O (wherein R¹⁰⁷ has the same meaning as that mentioned above) in an appropriate solvent, if necessary, in the presence of 1 to 30 equivalents of an appropriate base, at a temperature between -30°C and 150°C for 5 minutes to 48 hours.

Examples of the appropriate solvent include, for example, dichloromethane, acetonitrile, toluene, ethyl acetate, pyridine, THF, DMF, and the like. Examples of the appropriate base include, for example, pyridine, triethylamine, diisopropylethylamine, potassium carbonate, potassium hydroxide, and the like.

### Step 12-2

Compound (Iq) can be prepared by the reaction of Compound (XX) prepared in Step 12-1 mentioned above with 1 to 200 equivalents of R¹¹WH (wherein R¹¹ and W have the same meanings as those mentioned above, respectively) in an appropriate solvent, if necessary, in the presence of 1 to 30 equivalents of an appropriate base, at a temperature between -30°C and 150°C for 5 minutes to 48 hours.

Examples of the appropriate solvent include, for example, dichloromethane, acetonitrile, toluene, ethyl acetate, pyridine, THF, DMF, and the like. Examples of the appropriate base include, for example, pyridine, triethylamine, diisopropylethylamine, potassium carbonate, potassium hydroxide, and the like.

In Compound (I), conversion of the functional groups contained in R¹, R², R³, R⁴, A or B can be carried out by the other known methods [e.g., Comprehensive Organic Transformations, R. C. Larock (1989) and the like], or the methods similar to the known methods, as well as by the aforementioned steps.

Compound (I) having the desired functional group at the desired position can be prepared by carrying out the aforementioned methods in appropriate combination.

The intermediates and the desired compounds in the aforementioned preparation methods can be isolated and purified by conducting separation and purification methods ordinarily used in the organic synthetic chemistry such as filtration, extraction, washing, drying, concentration, recrystallization, various chromatography and the like. The intermediates can also be subjected to the next reaction without particular purification.

Among Compounds (I), stereoisomers such as regioisomers, geometrical isomers, optical isomers, tautomers and the like may be existed, and including these isomers, all possible isomers and the mixtures thereof fall within the scope of the present invention.

To obtain a salt of Compound (I), when Compound (I) is obtained as a salt form, it may be purified as it is. When Compound (I) is obtained as a free form, it may be dissolved or suspended in an appropriate solvent, and added an appropriate acid or base to form a salt and then be isolated and purified.

In addition, Compound (I) or a pharmacologically acceptable salt thereof may exist in the form of adducts with water or various solvents, which also fall within the scope of the present invention.

Specific examples of Compound (I) obtained by the present invention are shown in Tables 1 to 8. However, the compounds of the present invention are not limited to these examples.

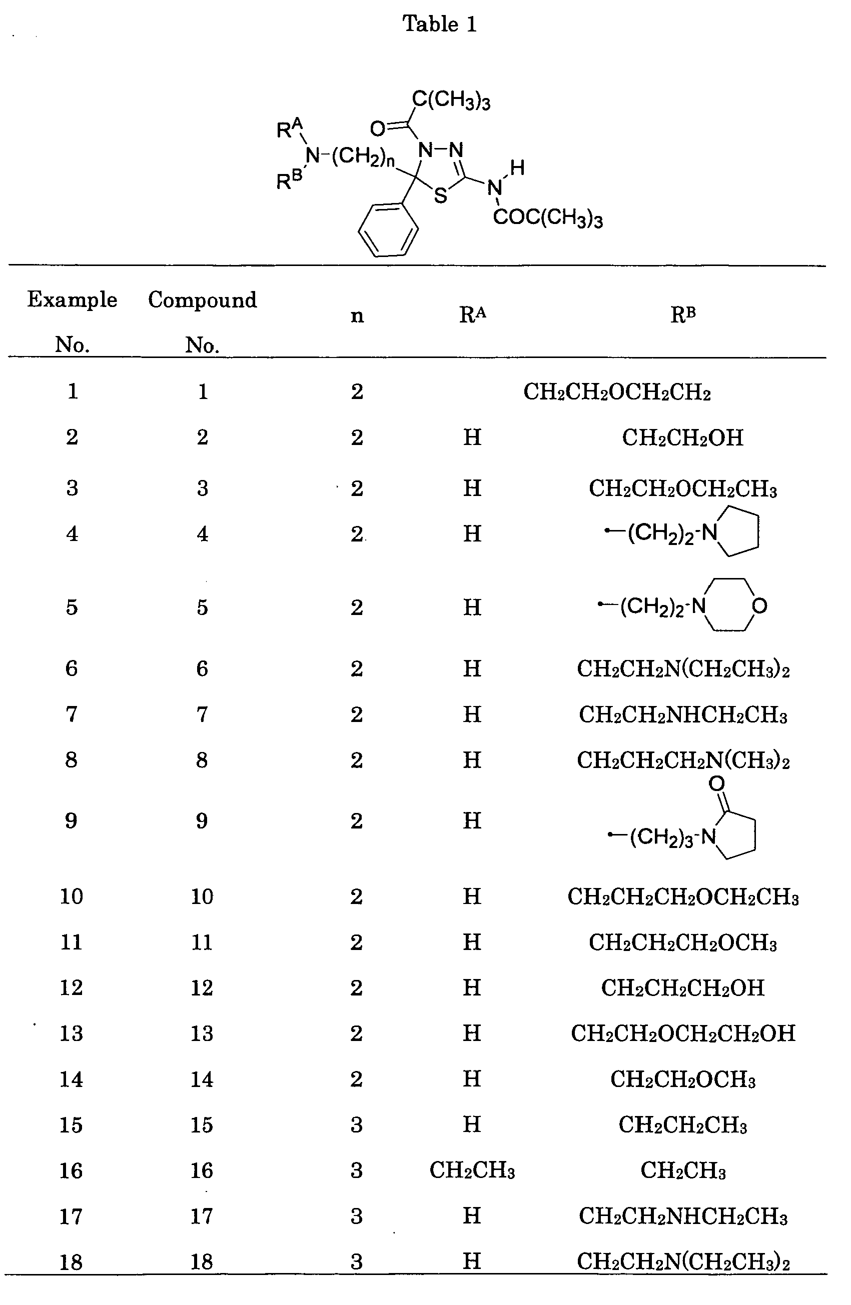

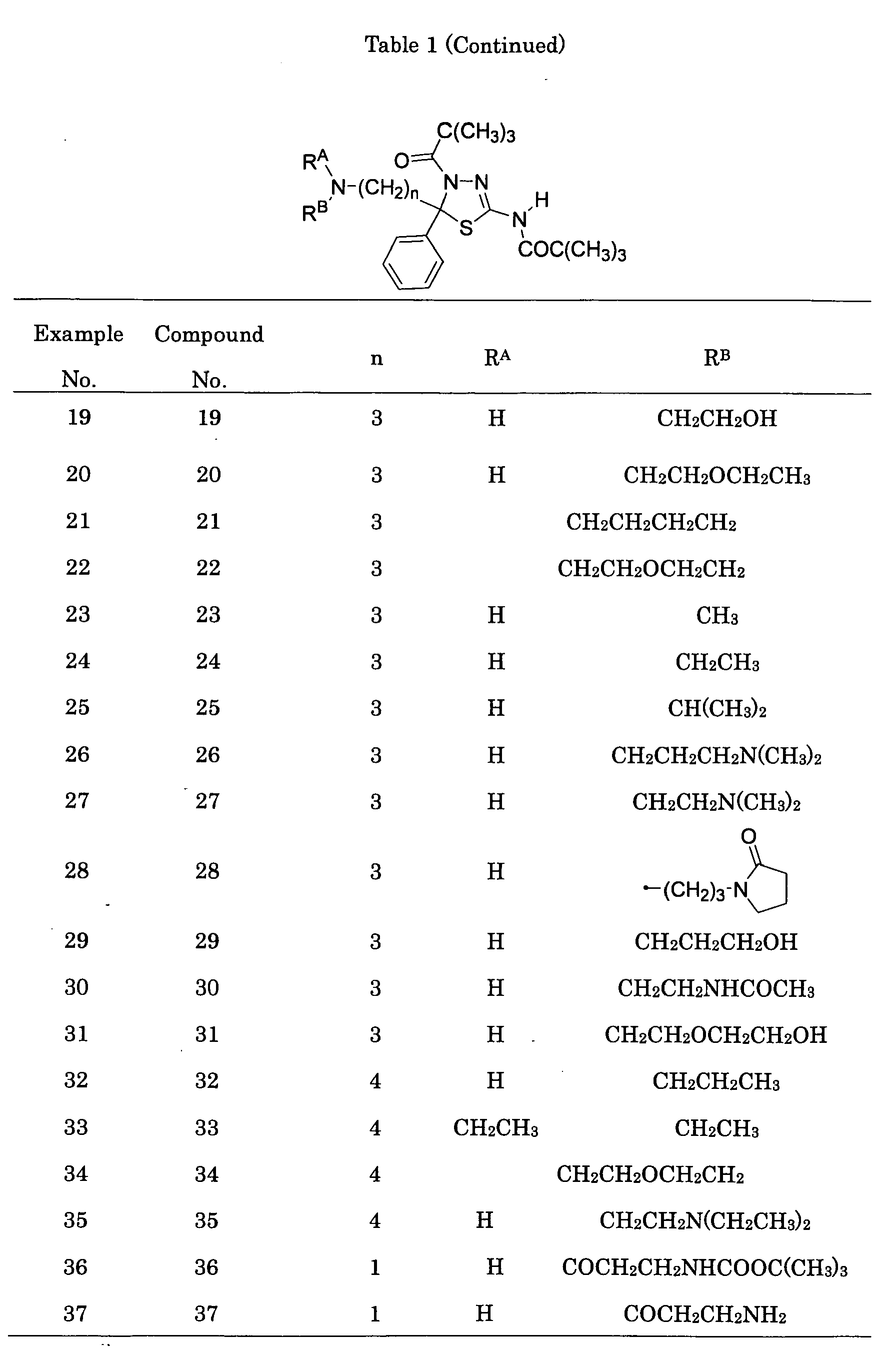

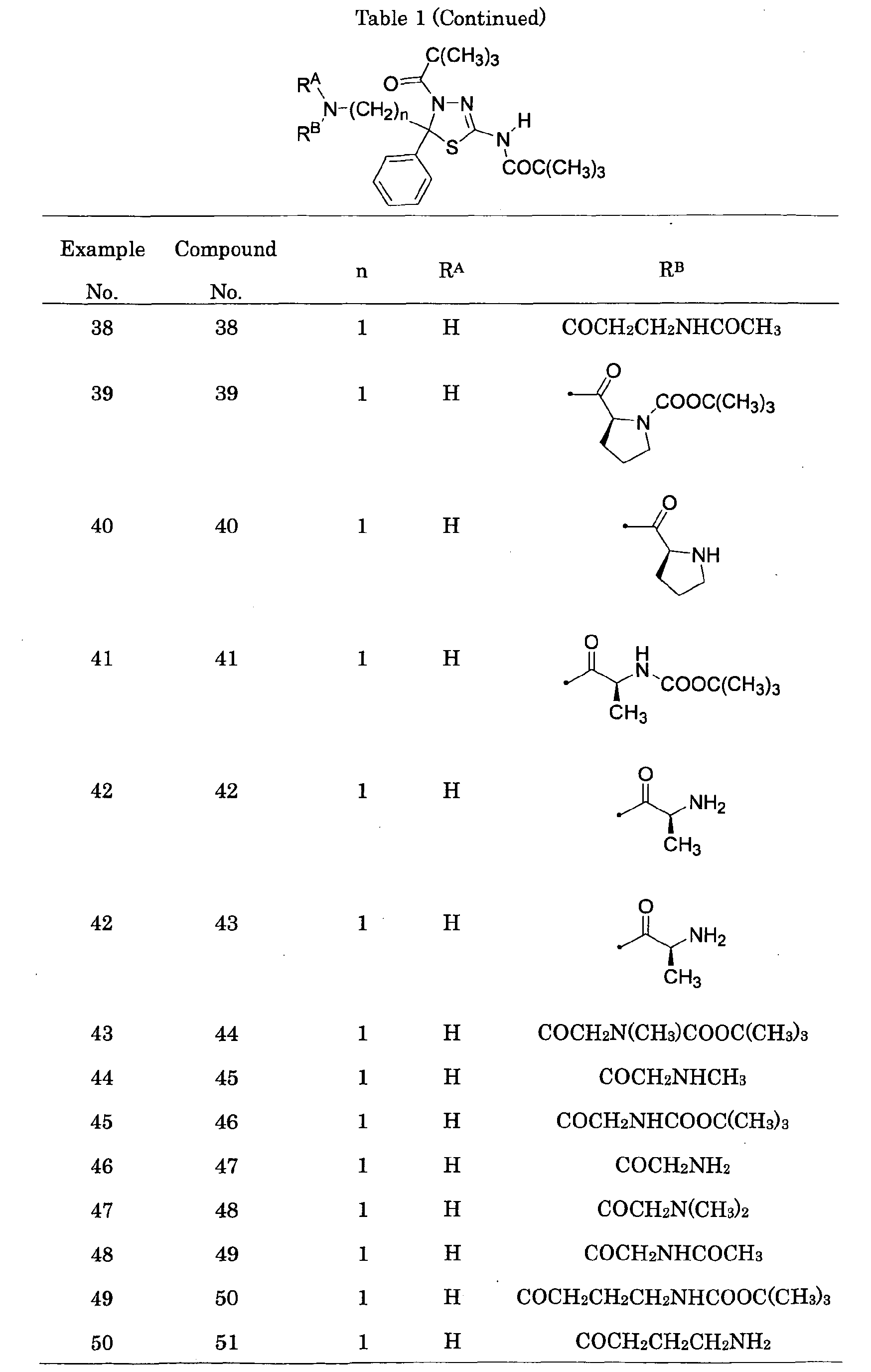

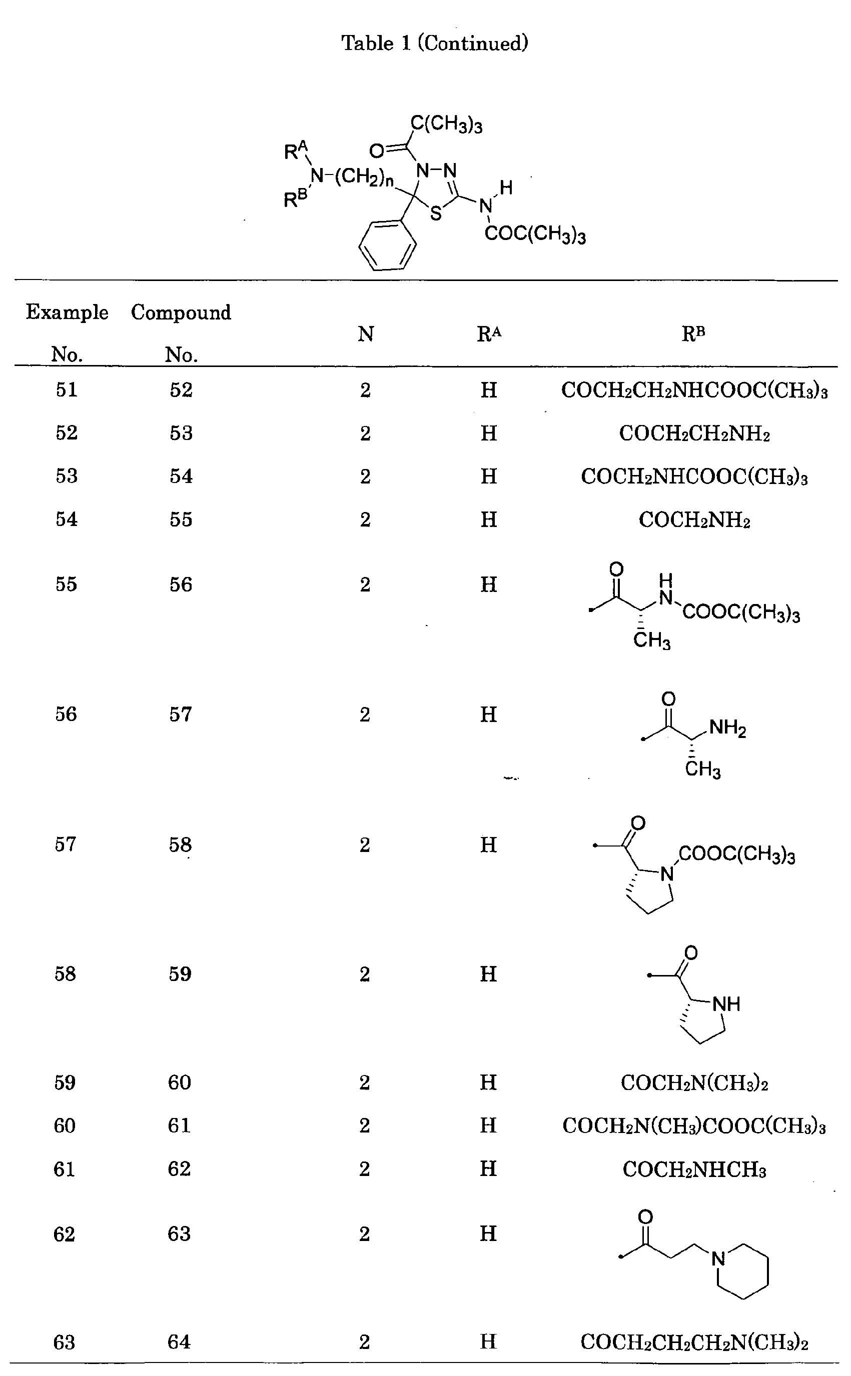

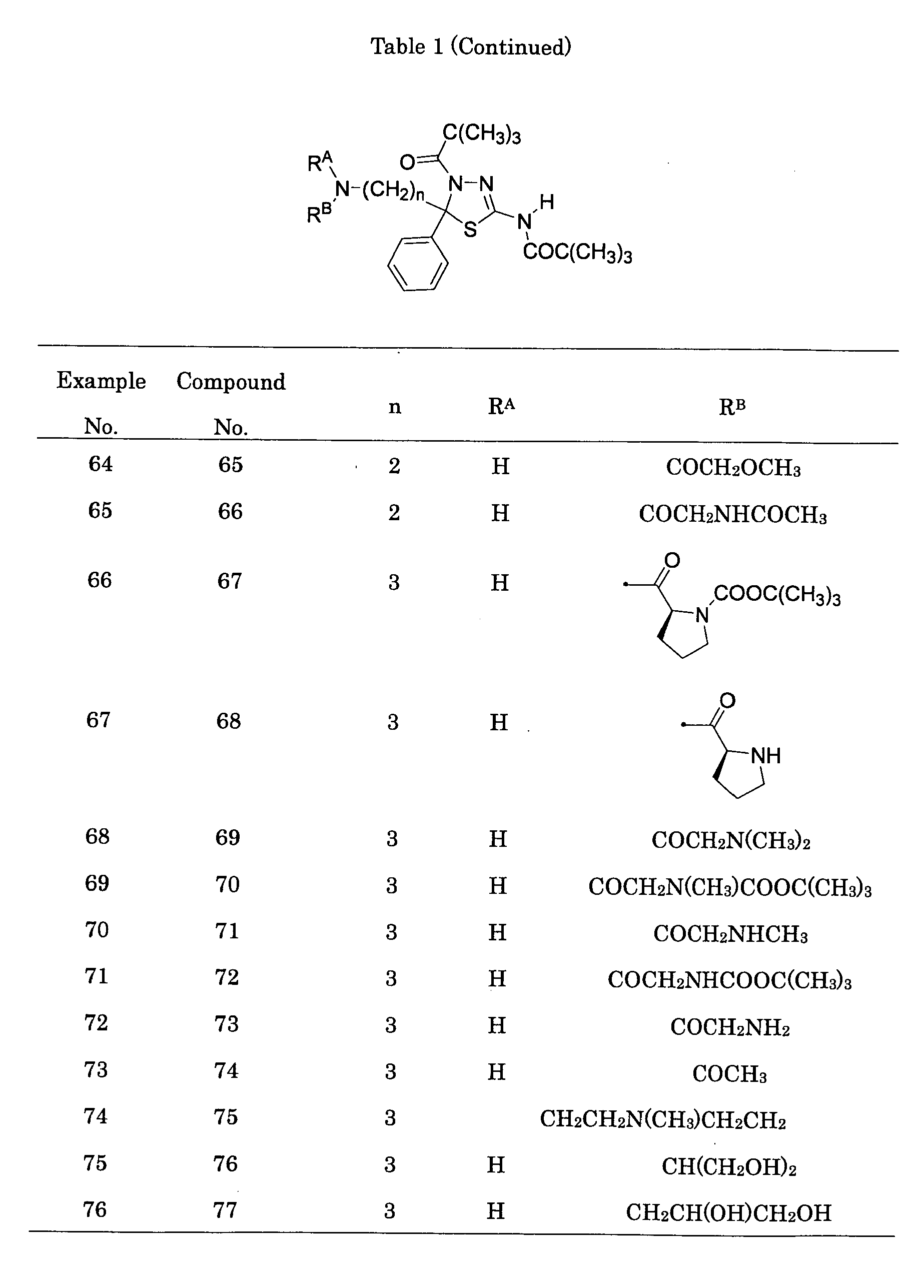

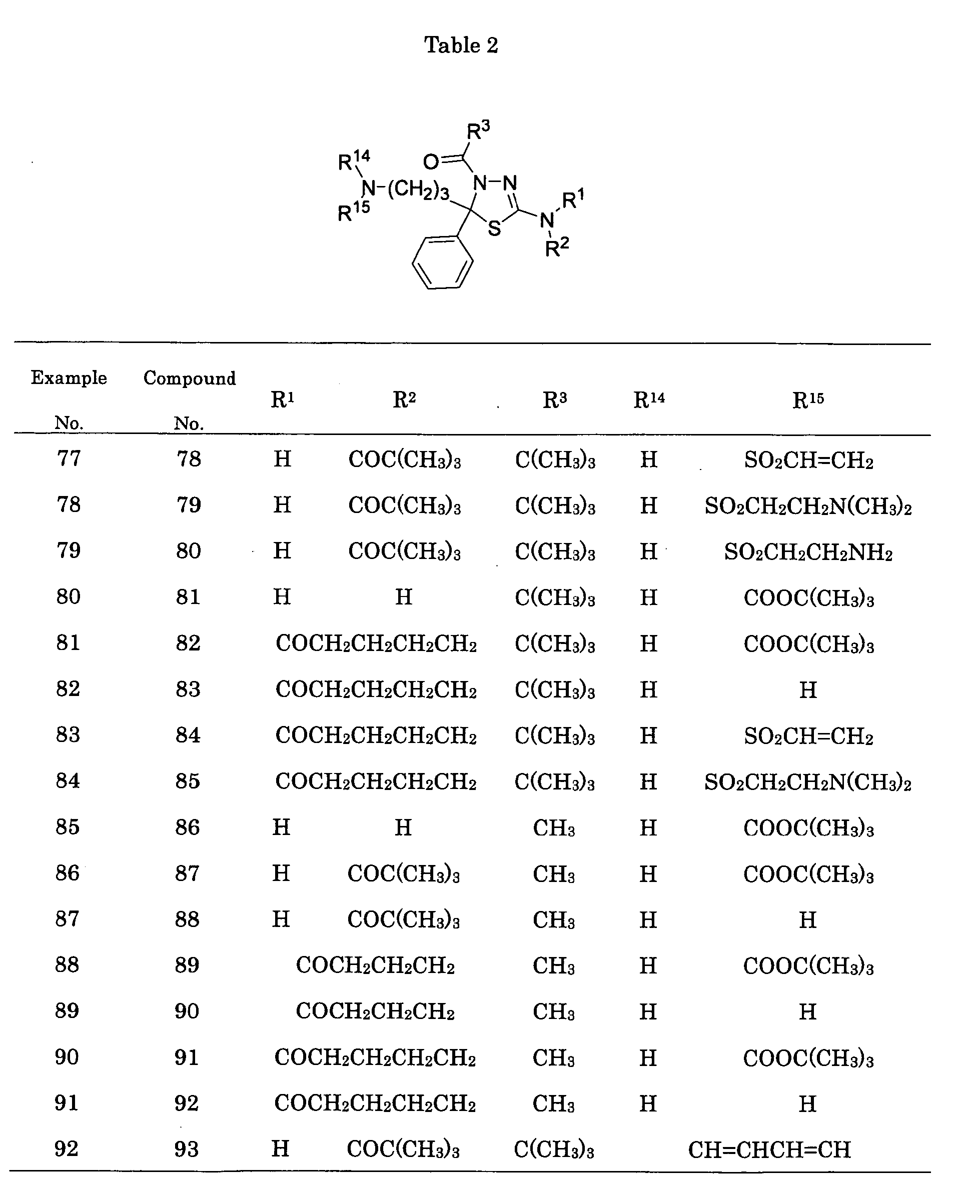

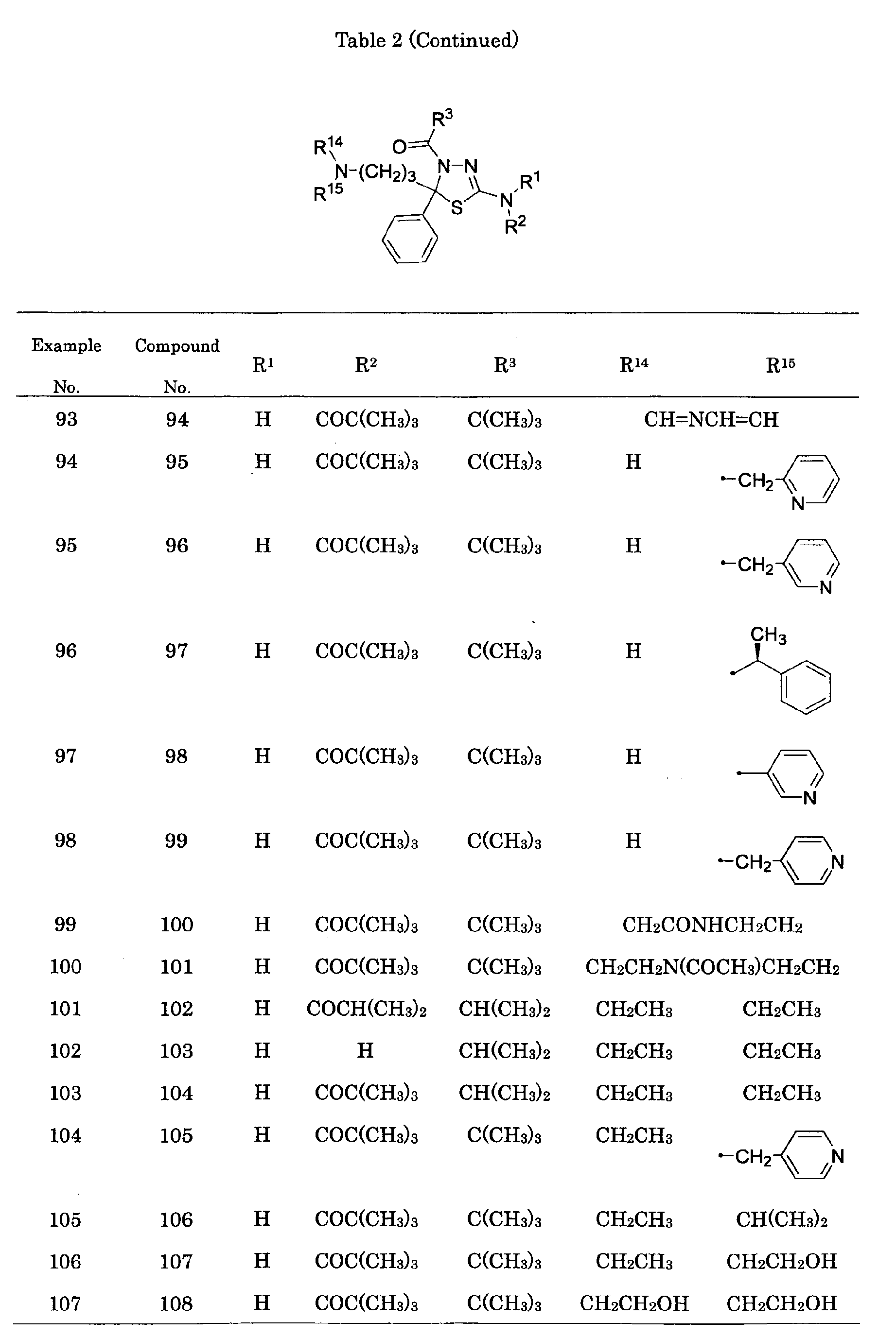

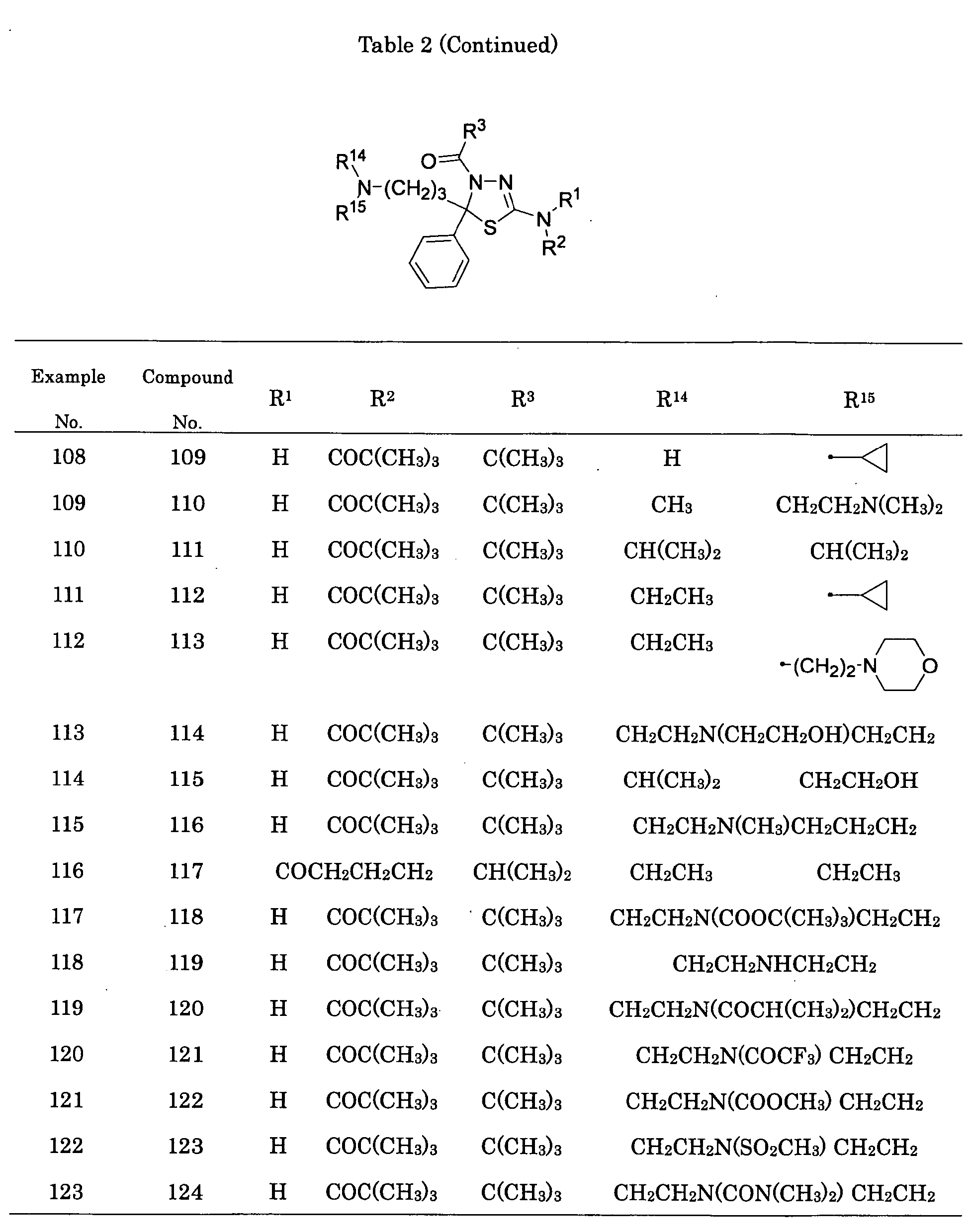

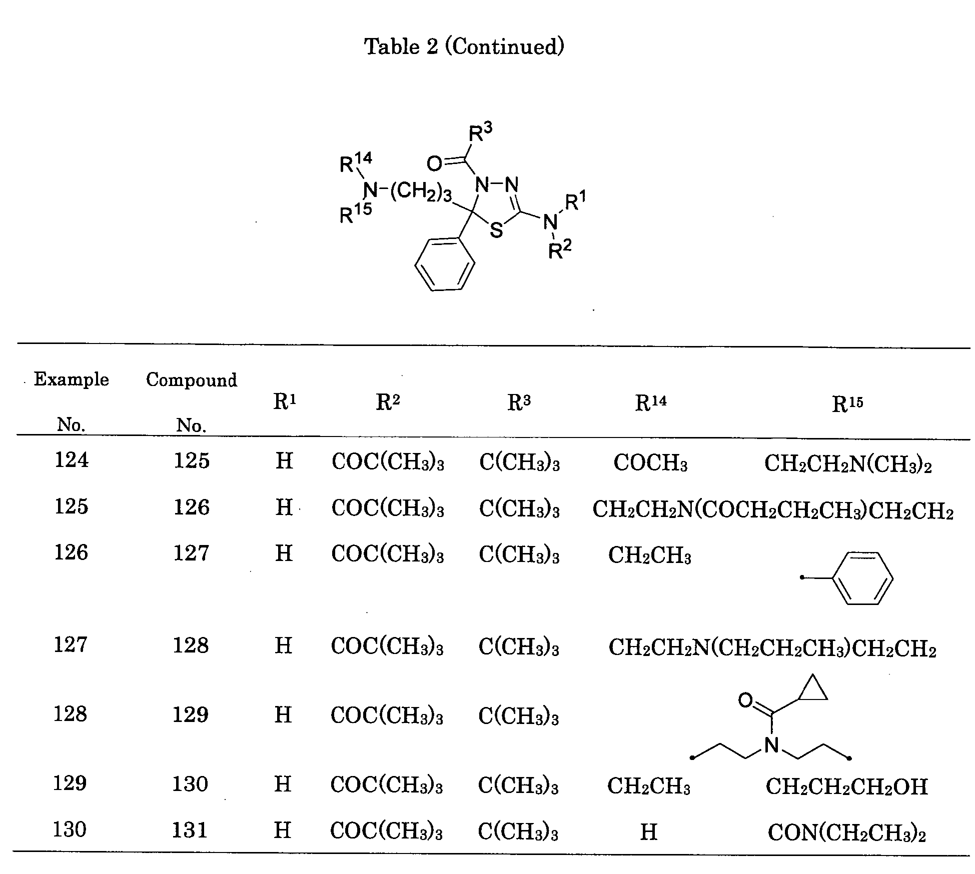

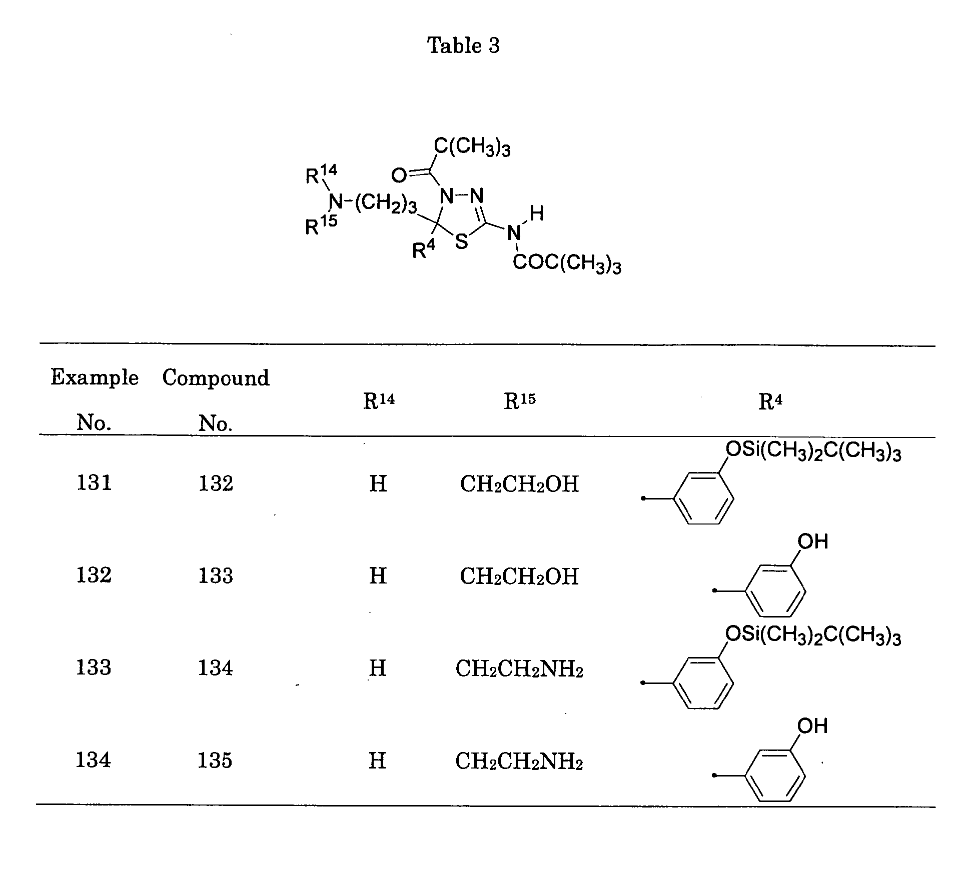

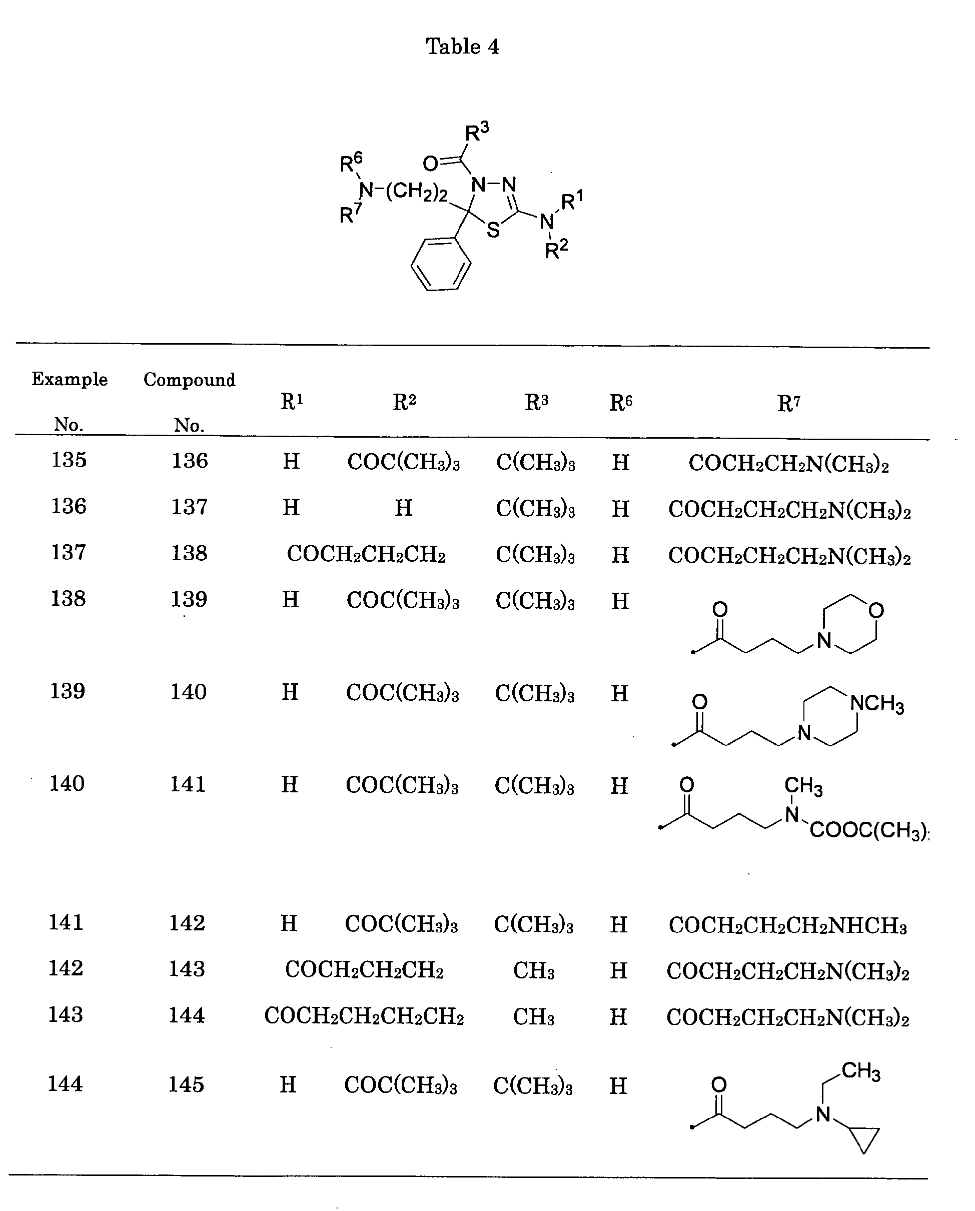

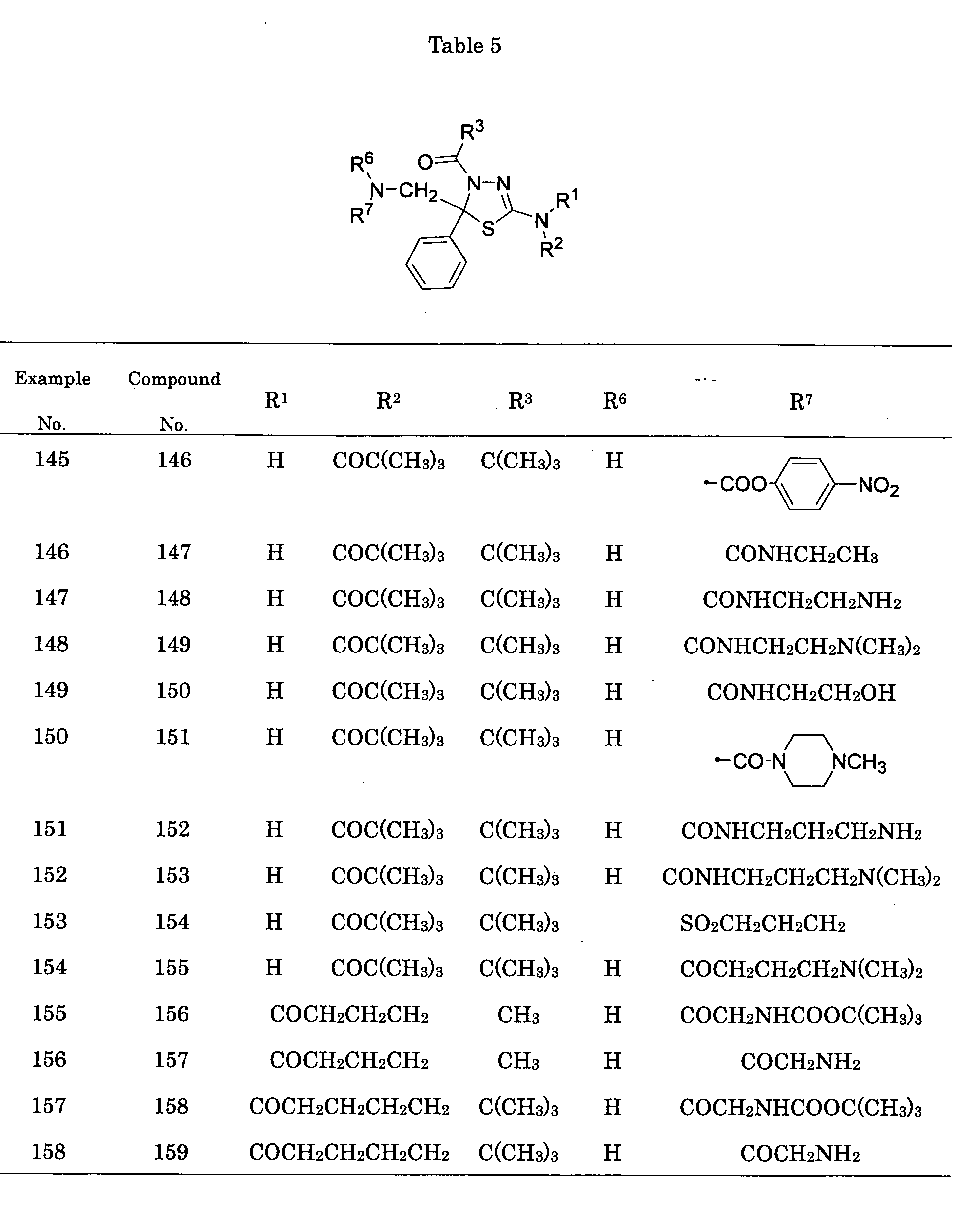

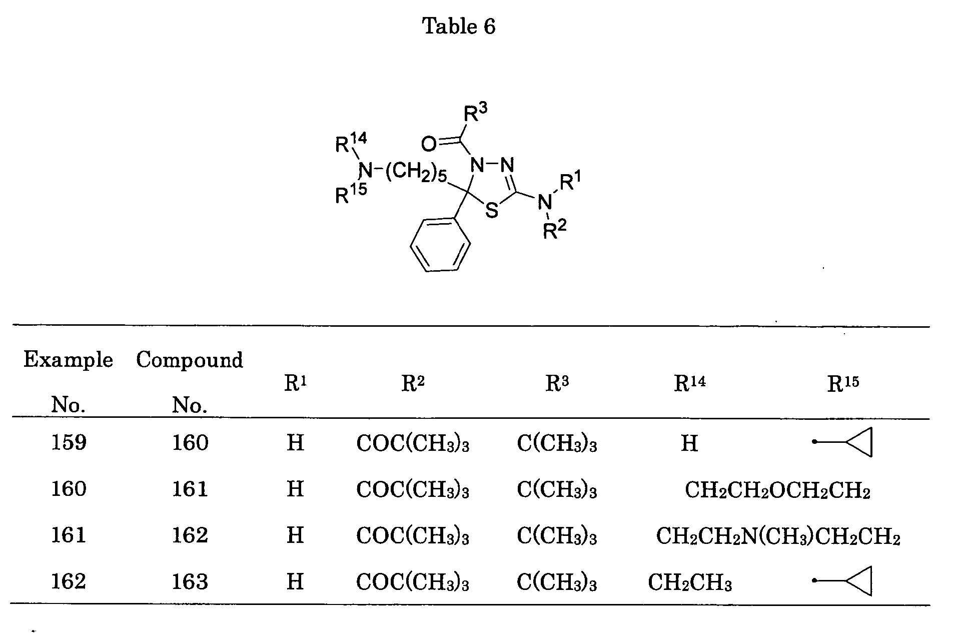

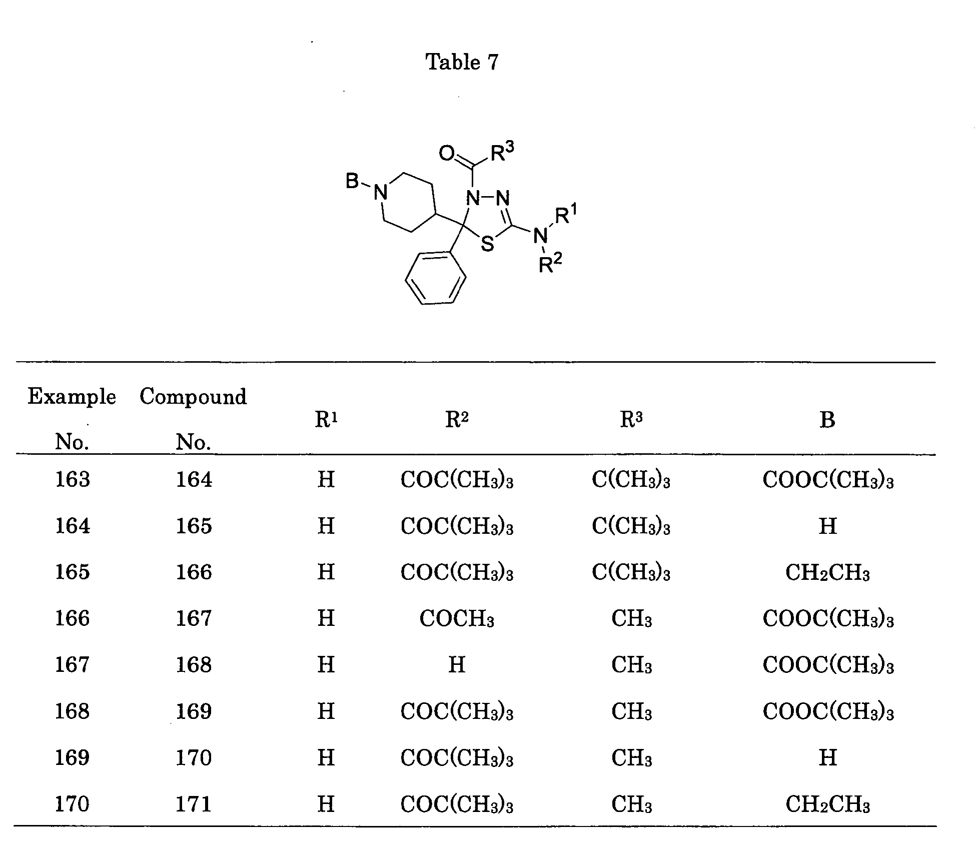

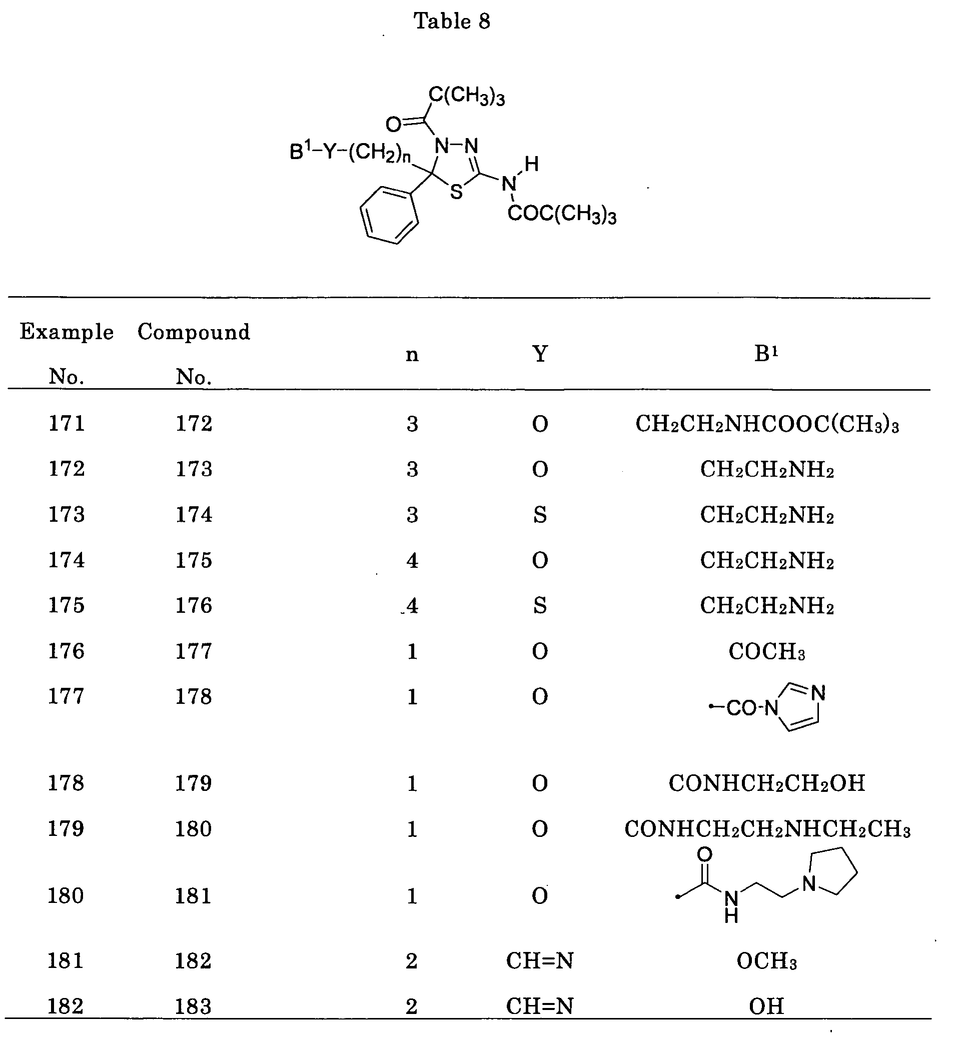

Pharmacological activities of typical Compound (I) will be specifically explained by the following test examples.

### Test example 1: Antiproliferative activity against HCT 116 human colon carcinoma cells

HCT 116 cells (ATCC No.: CCL-247) were placed on a 96-well microtiter plate (Nunc, 167008) at a density of 1x10³ cells/well. The plate was incubated in a 5% CO₂ incubator at 37°C for 24 hours, and then to the plate was added test compounds diluted stepwise to 100 mL/well in total, and the plate was further incubated in a 5% CO₂ incubator at 37°C for 72 hours. To the culture medium, the XTT (sodium 3'-[1-(phenylaminocarbonyl)-3,4-tetrazolium]-bis(4-methoxy-6-nitro)benzenesulfonic acid hydrate) labeling mixture (Roche Diagnostics, 1465015) was dispensed in 50 mL/well portions, then the plate was incubated in a 5% CO₂ incubator at 37°C for 1 hour, and the absorbance was measured at 490 nm and 655 nm with a microplate spectrophotometer (Bio-Rad, Model 550). The inhibitory activity against cell proliferation was shown as GI₅₀, a concentration of compound at which induces 50% inhibition of cell proliferation.
GI₅₀ calculation method: The value (difference in absorbance) was calculated by subtracting the absorbance at 655 nm from the absorbance at 490 nm of each well. The difference in absorbance obtained from the cells untreated with a test compound was defined as 100%, and compared with the difference in absorbance obtained from the cells treated with the solution of the compound in the known concentration, and thereby the concentration of the compound of 50% inhibition against cell proliferation was calculated to obtain GI₅₀.

Compound 9 showed the antiproliferative activity, and the GI₅₀ value was 65 nmol/L. Also, compounds 10, 59, 76, 85, 96, 122, 144, 174, and 181 had the GI₅₀ value less than 10 µ mol/L.

### Test Example 2: Eg5 enzyme inhibition test (1)

A full length recombinant human Eg5 protein is prepared by referring to the literature [Cell, Vol. 83, p.1159 (1995)]. The *Spodoptera frugiperda* (Sf) 9 insect cells are infected with a baculovirus expressing a full length human Eg5 protein fused with a His tag at the N-terminus, and cultured. Then the culture medium is centrifuged to collect cell pellets. The cell pellets are suspended in a buffer, and the suspension is centrifuged to recover the supernatant. The supernatant is passed through a nickel agarose column to obtain the Eg5 protein fused with a His tag at the N-terminus as a partially purified sample.

Measurement of the ATPase activity of Eg5 is carried out by referring to the literature [EMBO Journal, Vol. 13, p.751 (1994); Proc. Natl. Acad. Sci. USA, Vol. 89, p.4884 (1992)]. A reaction solution is prepared which consisted of 25 mmol/L piperazine N,N'-bis(ethanesulfonate) (PIPES)/KOH (pH 6.8), 1 mmol/L ethylene glycol-bis(2-aminoethyl ether)tetraacetic acid (EGTA), 2 mmol/L MgCl₂, 1 mmol/L dithiothreitol (DTT), 100 µg/mL bovine serum albumin (BSA), 5 µmol/L paclitaxel, 25 µg/mL tubulin (Cytoskeleton, Catalog No. TL238), 200 µmol/L MESG substrate (2-amino-6-mercapto-7-methylpurine riboside) (Molecular Probes, Catalog Number E-6646), 1 U/mL purine nucleoside phosphorylase (Molecular Probe, Catalog No. E-6646) and the partially purified sample of full length human Eg5. The reaction solution containing serially diluted test compound is added to each well of a 96-well plate. The enzymatic reaction is performed at 30°C for 30 minutes. Absorbance at 360 nm is measured using a plate reader (Molecular Device, SpectraMax 340PC³⁸⁴) as an index of the ATPase activity. The absorbance observed in the presence of Eg5 and absence of the test compound is defined 100%, and the absorbance observed in the absence of both Eg5 and the test compound is defined 0%. The relative activity is calculated to determine the IC₅₀ value.

The inhibitory activity against Eg5 enzyme of Compound (I) can be confirmed by the test mentioned above.

### Test Example 3: Eg5 enzyme inhibition test (2)

A recombinant human Eg5 motor domain protein was prepared by referring to the literature [Biochemistry, Vol. 35, p.2365 (1996)]. A plasmid expressing the motor domain of human Eg5 was constructed, and transformed into *Escherichia coli* BL21 (DE3). The transformant was cultured at 25°C, and when the OD₆₀₀ reached 0.74, isopropyl-β-D-thiogalactoside was added at a final concentration of 0.5 mmol/L. The transformant was further cultured for 4 hours, and then the culture medium was centrifuged to collect the cells. The cells were suspended in a buffer and ultrasonicated, and then the sonicated solution was centrifuged to recover the supernatant. The supernatant was purified by cation exchange column chromatography to obtain a partially purified sample. Furthermore, the partially purified sample was purified by gel filtration column chromatography to obtain a finally purified sample.

Measurement of the ATPase activity of Eg5 was carried out by referring to the literatures [EMBO Journal, Vol. 13, p.751 (1994); Proc. Natl. Acad. Sci. USA, Vol. 89, p.4884 (1992)]. The following two kinds of solutions were prepared: Solution A consisting of 25 mmol/L piperazine N,N'-bis(ethanesulfonate) (PIPES)/KOH (pH 6.8), 1 mmol/L ethylene glycol-bis(2-aminoethyl ether)tetraacetic acid (EGTA), 2 mmol/L MgCl₂, 1 mmol/L dithiothreitol (DTT), 5 µmol/L paclitaxel, 167 µg/mL bovine serum albumin (BSA), 41.7 µg/mL tubulin (Cytoskeleton, Catalog No. TL238), 333 µmol/L MESG substrate (2-amino-6-mercapto-7-methylpurine riboside) (Molecular Probes, Catalog Number E-6646), 1.67 U/mL purine nucleoside phosphorylase (Molecular Probe, Catalog No. E-6646) and 1.33 µg/mL of the human Eg5 motor domain purified sample, and Solution B consisting of 25 mmol/L piperazine N,N'-bis(ethanesulfonate) (PIPES)/KOH (pH 6.8), 1 mmol/L ethylene glycol-bis(2-aminoethyl ether)tetraacetic acid (EGTA), 2 mmol/L MgCl₂, 1 mmol/L dithiothreitol (DTT), 5 µmol/L paclitaxel and 2.5 mmol/L ATP. Solution A was dispensed into each well of a 96-well plate as 45 µL portions. Solution B was used to serially dilute a test compound. The diluted test compound solutions in a volume of 30 µL were mixed with Solution A added beforehand in each well of the 96-well plate to start the enzymatic reaction. The enzymatic reaction was performed at 30°C for 30 minutes. Absorbance at 360 nm, which serves as an index of the ATPase activity, was measured using a plate reader (Molecular Device, SpectraMax 340PC³⁸⁴). The absorbance observed in the presence of Eg5 and absence of the test compound was defined 100%, and the absorbance observed in the absence of both Eg5 and the test compound was defined 0%. The relative activity was calculated to calculate IC₅₀ value.

Compounds 3, 9, 23, 29, 59, 73, 76, 83, 85, 88, 90, 96, 122, 144, and 181 inhibited the ATPase activity of Eg5 in a concentration-dependent manner, and IC₅₀ values of the compounds were found to be 5 µmol/L or lower.

Compound (I) or a pharmaceutically acceptable salt thereof can be administered alone. However, usually, Compound (I) or a pharmaceutically acceptable salt thereof is preferably provided in various pharmaceutical preparations. Furthermore, these pharmaceutical preparations are used for animals and humans.

The pharmaceutical preparations according to the present invention may comprise Compound (I) or a pharmaceutically acceptable salt thereof alone as an active ingredient. Alternatively, the pharmaceutical preparations may comprise a mixture of Compound (I) or a pharmaceutically acceptable salt thereof with any effective ingredient used for another treatment. Furthermore, these pharmaceutical preparations are prepared by mixing the active ingredient(s) with one or more pharmaceutically acceptable carrier(s) and then employing any method well-known in the technical field of pharmaceutics.

As for administration routes, it is preferred to select the most effective route of administration. Examples of the administration routes include oral administration and parenteral administration such as intravenous administration and the like.

As for the dosage form, for example, tablets, injections and the like are included.

For example, the tablet suitable for oral administration can be prepared with, for example, excipients such as lactose and mannitol; disintegrants such as starch; lubricants such as magnesium stearate; binders such as hydroxypropylcellulose; surfactants such as a fatty acid ester; plasticizers such as glycerol; and the like.

Preparations suitable for parenteral administration preferably comprise a sterilized aqueous preparation containing the active compound and being isotonic to blood of a recipient. For example, when an injection is prepared, a solution for injection is prepared by using a carrier consisting of a salt solution, glucose solution, a mixture of salt solution and glucose solution, or the like.

Also in these parenteral preparations, one or more kinds of auxiliary components selected from excipients, disintegrants, lubricants, binders, surfactants, plasticizers, diluents which are exemplified for the oral administration, preservatives, flavors and the like may be added.

Compound (I) or a pharmacologically acceptable salt thereof is generally administered systemically or locally in the form of an oral or parenteral preparation when used for the aforementioned purpose. The dose and the frequency of administration may vary depending on the administration form, the age and body weight of a patient, nature and severity of the condition to be treated, and the like. When oral administration is performed, generally 0.01 to 1,000 mg/kg, preferably 0.05 to 500 mg/kg per single administration for an adult may be administered once a day or a few times a day. When parenteral administration such as intravenous administration is performed, 0.001 to 1,000 mg/kg, preferably 0.01 to 300 mg/kg, per single administration for an adult may be administered once a day or a few times a day, or may be continuously administered intravenously for 1 to 24 hours a day. However, the dose and the frequency of administration may vary depending on the aforementioned various conditions and the like.

### Best Mode for Carrying out the Invention

The present invention will be explained in detail with reference to the following examples and reference examples.

The spectra of proton nuclear magnetic resonance (¹H NMR) used in Examples and Reference Examples were measured at 270 or 300 MHz, and exchangeable hydrogen may not always be clearly observed depending on the compound and the measurement conditions. For the descriptions of the multiplicity of signals, those generally applied are used, and the symbol "br" represents an apparent broad signal.

### Example 1 (Compound 1)

In a manner similar to that in Reference Example 4, Compound 1 (55.5 mg, 91%) was obtained from Compound c (51.3 mg, 0.132 mmol) prepared in Reference Example 3, acetic acid (0.0460 mL, 0.804 mmol), morpholine (0.0580 mL, 0.665 mmol) and triacetoxy sodium borohydride (117 mg, 0.553 mmol).
APCI-MS m/z: 461 (M+H)⁺.

### Example 2 (Compound 2)

In a manner similar to that in Reference Example 4, Compound 2 (24.5 mg, 44%) was obtained from Compound c (50.3 mg, 0.129 mmol) prepared in Reference Example 3, acetic acid (0.0440 mL, 0.769 mmol), 2-aminoethanol (0.0400 mL, 0.663 mmol) and triacetoxy sodium borohydride (116 mg, 0.546 mmol).
APCI-MS m/z: 435 (M+H)⁺.

### Example 3 (Compound 3)

In a manner similar to that in Reference Example 4, Compound 3 (46.9 mg, 79%) was obtained from Compound c (50.4 mg, 0.129 mmol) prepared in Reference Example 3, acetic acid (0.0440 mL, 0.769 mmol), 2-ethoxyethylamine (0.0680 mL, 0.648 mmol) and triacetoxy sodium borohydride (121 mg, 0.572 mmol).
APCI-MS m/z: 463 (M+H)⁺.

### Example 4 (Compound 4)

In a manner similar to that in Reference Example 4, Compound 4 (18.5 mg, 29%) was obtained from Compound c (51.1 mg, 0.131 mmol) prepared in Reference Example 3, acetic acid (0.0460 mL, 0.804 mmol), N-(2-aminoethyl)pyrrolidine (0.0830 mL, 0.661 mmol) and triacetoxy sodium borohydride (139 mg, 0.656 mmol).
APCI-MS m/z: 488 (M+H)⁺.

### Example 5 (Compound 5)

In a manner similar to that in Reference Example 4, Compound 5 (61.2 mg, 89%) was obtained from Compound c (53.1 mg, 0.136 mmol) prepared in Reference Example 3, acetic acid (0.0470 mL, 0.821 mmol), N-(2-aminoethyl)morpholine (0.0890 mL, 0.684 mmol) and triacetoxy sodium borohydride (148 mg, 0.697 mmol).
APCI-MS m/z: 504 (M+H)⁺.

### Example 6 (Compound 6)

In a manner similar to that in Reference Example 4, Compound 6 (36.9 mg, 56%) was obtained from Compound c (52.0 mg, 0.134 mmol) prepared in Reference Example 3, acetic acid (0.0460 mL, 0.804 mmol), N,N-diethylethylenediamine (0.0940 mL, 0.669 mmol) and triacetoxy sodium borohydride (121 mg, 0.570 mmol).
APCI-MS m/z: 490 (M+H)⁺.

### Example 7 (Compound 7)

In a manner similar to that in Reference Example 4, Compound 7 (29.7 mg, 46%) was obtained from Compound c (54.5 mg, 0.140 mmol) prepared in Reference Example 3, acetic acid (0.0800 mL, 1.40 mmol), N-ethylethylenediamine (0.0740 mL, 0.703mmol) and triacetoxy sodium borohydride (134 mg, 0.632 mmol).
FAB-MS m/z: 462 (M+H)⁺.

### Example 8 (Compound 8)

In a manner similar to that in Reference Example 4, Compound 8 (22.9 mg, 34%) was obtained from Compound c (54.5 mg, 0.140 mmol) prepared in Reference Example 3, acetic acid (0.0800 mL, 1.400 mmol), N,N-dimethyl-1,3-propanediamine (0.0880 mL, 0.699 mmol) and triacetoxy sodium borohydride (131 mg, 0.620 mmol).
FAB-MS m/z: 476 (M+H)⁺.

### Example 9 (Compound 9)

In a manner similar to that in Reference Example 4, Compound 9 (49.1 mg, 77%) was obtained from Compound c (48.3 mg, 0.124 mmol) prepared in Reference Example 3, acetic acid (0.0480 mL, 0.839 mmol), 1-(3-aminopropyl)-2-pyrrolidinone (0.0870 mL, 0.620 mmol) and triacetoxy sodium borohydride (125 mg, 0.590 mmol).
APCI-MS m/z: 516 (M+H)⁺.

### Example 10 (Compound 10)

In a manner similar to that in Reference Example 4, Compound 10 (54.6 mg, 88%) was obtained from Compound c (50.7 mg, 0.130 mmol) prepared in Reference Example 3, acetic acid (0.0450 mL, 0.786 mmol), 3-ethoxypropylamine (0.0780 mL, 0.651 mmol) and triacetoxy sodium borohydride (125 mg, 0.588 mmol).
APCI-MS m/z: 477 (M+H)⁺.

### Example 11 (Compound 11)

In a manner similar to that in Reference Example 4, Compound 11 (48.8 mg, 81%) was obtained from Compound c (50.9 mg, 0.131 mmol) prepared in Reference Example 3, acetic acid (0.0450 mL, 0.786 mmol), 3-methoxypropylamine (0.0670 mL, 0.657 mmol) and triacetoxy sodium borohydride (130 mg, 0.611 mmol).
APCI-MS m/z: 463 (M+H)⁺.

### Example 12 (Compound 12)

In a manner similar to that in Reference Example 4, Compound 12 (87.1 mg, 75%) was obtained from Compound c (101 mg, 0.259 mmol) prepared in Reference Example 3, acetic acid (0.0900 mL, 1.57 mmol), 3-amino-1-propanol (0.100 mL, 1.31 mmol) and triacetoxy sodium borohydride (248 mg, 1.17 mmol).
APCI-MS m/z: 449 (M+H)⁺.

### Example 13 (Compound 13)

In a manner similar to that in Reference Example 4, Compound 13 (85.4 mg, 68%) was obtained from Compound c (102 mg, 0.262 mmol) prepared in Reference Example 3, acetic acid (0.0900 mL, 1.57 mmol), 2-(2-aminoethoxy)ethanol (0.131 mL, 1.31 mmol) and triacetoxy sodium borohydride (243 mg, 1.15 mmol).
APCI-MS m/z: 479 (M+H)⁺.

### Reference Example 14 (Compound 14)

In a manner similar to that in Reference Example 4, Compound 14 (50.3 mg, 87%) was obtained from Compound c (50.3 mg, 0.129 mmol) prepared in Reference Example 3, acetic acid (0.0450 mL, 0.786 mmol), 2-methoxyethylamine (0.0570 mL, 0.656 mmol) and triacetoxy sodium borohydride (119 mg, 0.561 mmol).

APCI-MS m/z: 449 (M+H)⁺.

### Example 15 (Compound 15)

In a manner similar to that in Reference Example 4, Compound 15 (29.3 mg, 38%) was obtained from Compound g (69.0 mg, 0.171 mmol) prepared in Reference Example 8, acetic acid (0.0587 mL, 1.03 mmol), n-propylamine (0.0703 mL, 0.855 mmol) and triacetoxy sodium borohydride (187 mg, 0.882 mmol).
APCI-MS m/z: 447 (M+H)⁺.

### Example 16 (Compound 16)

In a manner similar to that in Reference Example 4, Compound 16 (47.4 mg, 62%) was obtained from Compound g (66.5 mg, 0.165 mmol) prepared in Reference Example 8, acetic acid (0.0587 mL, 1.03 mmol), diethylamine (0.0886 mL, 0.855 mmol) and triacetoxy sodium borohydride (175 mg, 0.824 mmol).
APCI-MS m/z: 461 (M+H)⁺.

### Example 17 (Compound 17)

In a manner similar to that in Reference Example 4, Compound 17 (23.6 mg, 39%) was obtained from Compound g (51.6 mg, 0.128 mmol) prepared in Reference Example 8, acetic acid (0.0730 mL, 1.28 mmol), N-ethylethylenediamine (0.0670 mL, 0.636 mmol) and triacetoxy sodium borohydride (114 mg, 0.537 mmol).
APCI-MS m/z: 476 (M+H)⁺.

### Example 18 (Compound 18)

In a manner similar to that in Reference Example 4, Compound 18 (50.5 mg, 77%) was obtained from Compound g (52.5 mg, 0.130 mmol) prepared in Reference Example 8, acetic acid (0.0740 mL, 1.29 mmol), N,N-diethylethylenediamine (0.0910 mL, 0.648 mmol) and triacetoxy sodium borohydride (114 mg, 0.539 mmol).
APCI-MS m/z: 504 (M+H)⁺.

### Example 19 (Compound 19)

In a manner similar to that in Reference Example 4, Compound 19 (20.3 mg, 36%) was obtained from Compound g (51.2 mg, 0.127 mmol) prepared in Reference Example 8, acetic acid (0.0440 mL, 0.769 mmol), 2-aminoethanol (0.0380 mL, 0.630 mmol) and triacetoxy sodium borohydride (121 mg, 0.570 mmol).
APCI-MS m/z: 449 (M+H)⁺.

### Example 20 (Compound 20)

In a manner similar to that in Reference Example 4, Compound 20 (41.5 mg, 68%) was obtained from Compound g (51.8 mg, 0.128 mmol) prepared in Reference Example 8, acetic acid (0.0440 mL, 0.758 mmol), 2-ethoxyethylamine (0.0670 mL, 0.639 mmol) and triacetoxy sodium borohydride (123 mg, 0.581 mmol).
APCI-MS m/z: 477 (M+H)⁺.

### Example 21 (Compound 21)

In a manner similar to that in Reference Example 4, Compound 21 (55.7 mg, 96%) was obtained from Compound g (51.4 mg, 0.127 mmol) prepared in Reference Example 8, acetic acid (0.0440 mL, 0.758 mmol), pyrrolidine (0.0530 mL, 0.636 mmol) and triacetoxy sodium borohydride (117 mg, 0.551 mmol).
APCI-MS m/z: 459 (M+H)⁺.

### Example 22 (Compound 22)

In a manner similar to that in Reference Example 4, Compound 22 (55.2 mg, 91%) was obtained from Compound g (51.7 mg, 0.128 mmol) prepared in Reference Example 8, acetic acid (0.0440 mL, 0.758 mmol), morpholine (0.0560 mL, 0.642 mmol) and triacetoxy sodium borohydride (133 mg, 0.628 mmol).
APCI-MS m/z: 475 (M+H)⁺.

### Example 23 (Compound 23)

In a manner similar to that in Reference Example 4, Compound 23 (31.4 mg, 52%) was obtained from Compound g (57.6 mg, 0.143 mmol) prepared in Reference Example 8, acetic acid (0.0587 mL, 1.03 mmol), a 40% solution of methylamine in methanol (0.0838 mL, 0.855 mmol) and triacetoxy sodium borohydride (176 mg, 0.831 mmol).
APCI-MS m/z: 419 (M+H)⁺.

### Example 24 (Compound 24)

In a manner similar to that in Reference Example 4, Compound 24 (38.2 mg, 61%) was obtained from Compound g (58.0 mg, 0.144 mmol) prepared in Reference Example 8, acetic acid (0.0587 mL, 1.03 mmol), a 70% solution of ethylamine in water (0.0707 mL, 0.855 mmol) and triacetoxy sodium borohydride (180 mg, 0.850 mmol).
APCI-MS m/z: 433 (M+H)⁺.

### Example 25 (Compound 25)

In a manner similar to that in Reference Example 4, Compound 25 (46.5 mg, 81%) was obtained from Compound g (52.1 mg, 0.129 mmol) prepared in Reference Example 8, acetic acid (0.0587 mL, 1.03 mmol), 2-aminopropane (0.0728 mL, 0.855 mmol) and triacetoxy sodium borohydride (175 mg, 0.828 mmol).
APCI-MS m/z: 447 (M+H)⁺.

### Example 26 (Compound 26)

In a manner similar to that in Reference Example 4, Compound 26 (24.4 mg, 37%) was obtained from Compound g (55.0 mg, 0.136 mmol) prepared in Reference Example 8, acetic acid (0.0800 mL, 1.40 mmol), N,N-dimethyl-1,3-propanediamine (0.0880 mL, 0.699 mmol) and triacetoxy sodium borohydride (137 mg, 0.646 mmol).
APCI-MS m/z: 490 (M+H)⁺.

### Example 27 (Compound 27)

In a manner similar to that in Reference Example 4, Compound 27 (40.3 mg, 64%) was obtained from Compound g (53.5 mg, 0.133 mmol) prepared in Reference Example 8, acetic acid (0.0800 mL, 1.40 mmol), N,N-dimethylethylenediamine (0.0780 mL, 0.711 mmol) and triacetoxy sodium borohydride (131 mg, 0.620 mmol).
APCI-MS m/z: 476 (M+H)⁺.

### Example 28 (Compound 28)

In a manner similar to that in Reference Example 4, Compound 28 (56.3 mg, 80%) was obtained from Compound g (53.3 mg, 0.132 mmol) prepared in Reference Example 8, acetic acid (0.0500 mL, 0.873 mmol), 1-(3-aminopropyl)-2-pyrrolidone (0.0930 mL, 0.663 mmol) and triacetoxy sodium borohydride (131 mg, 0.617 mmol).
APCI-MS m/z: 530 (M+H)⁺.

### Example 29 (Compound 29)

In a manner similar to that in Reference Example 4, Compound 29 (38.3 mg, 66%) was obtained from Compound g (50.7 mg, 0.126 mmol) prepared in Reference Example 8, acetic acid (0.0500 mL, 0.873 mmol), 3-amino-1-propanol (0.0480 mL, 0.628 mmol) and triacetoxy sodium borohydride (138 mg, 0.652 mmol).
APCI-MS m/z: 463 (M+H)⁺.

### Example 30 (Compound 30)

In a manner similar to that in Reference Example 4, Compound 30 (30.3 mg, 49%) was obtained from Compound g (50.6 mg, 0.125 mmol) prepared in Reference Example 8, acetic acid (0.0500 mL, 0.873 mmol), N-acetylethylenediamine (80.0 mg, 0.783 mmol) and triacetoxy sodium borohydride (134 mg, 0.632 mmol).
APCI-MS m/z: 490 (M+H)⁺.

### Example 31 (Compound 31)

In a manner similar to that in Reference Example 4, Compound 31 (44.6 mg, 73%) was obtained from Compound g (50.1 mg, 0.124 mmol) prepared in Reference Example 8, acetic acid (0.0500 mL, 0.873 mmol), 2-(2-aminoethoxy)ethanol (0.0630 mL, 0.628 mmol) and triacetoxy sodium borohydride (130 mg, 0.612 mmol).
APCI-MS m/z: 493 (M+H)⁺.

### Example 32

In a manner similar to that in Reference Example 4, Compound 32 (30.0 mg, 93%) was obtained from Compound j (29.4 mg, 0.0704 mmol) prepared in Reference Example 11, acetic acid (0.0450 mL, 0.786 mmol), n-propylamine (0.0538 mL, 0.654 mmol) and triacetoxy sodium borohydride (162 mg, 0.762 mmol).
ESI-MS m/z: 459 (M-H)⁻.

### Example 33 (Compound 33)

In a manner similar to that in Reference Example 4, Compound 33 (28.5 mg, 49%) was obtained from Compound j (50.8 mg, 0.122 mmol) prepared in Reference Example 11, acetic acid (0.0420 mL, 0.734 mmol), diethylamine (0.0630 mL, 0.609 mmol) and triacetoxy sodium borohydride (107 mg, 0.503 mmol).
APCI-MS m/z: 475 (M+H)⁺.

### Example 34 (Compound 34)

In a manner similar to that in Reference Example 4, Compound 34 (48.8 mg, 82%) was obtained from Compound j (51.0 mg, 0.122 mmol) prepared in Reference Example 11, acetic acid (0.0420 mL, 0.734 mmol), morpholine (0.0530 mL, 0.608 mmol) and triacetoxy sodium borohydride (112 mg, 0.530 mmol).
APCI-MS m/z: 489 (M+H)⁺.

### Example 35 (Compound 35)

In a manner similar to that in Reference Example 4, Compound 35 (23.3 mg, 42%) was obtained from Compound j (45.0 mg, 0.108 mmol) prepared in Reference Example 11, acetic acid (0.0370 mL, 0.638 mmol), N,N-diethylethylenediamine (0.0760 mL, 0.541 mmol) and triacetoxy sodium borohydride (97.0 mg, 0.455 mmol).
APCI-MS m/z: 518 (M+H)⁺.

### Example 36 (Compound 36)

Trifluoroacetate of Compound m (116 mg, 0.235 mmol) prepared in Reference Example 13 was dissolved in DMF (4 mL). To the solution was added N-tert-butoxycarbonyl-β-alanine (127 mg, 0.671 mmol), 1-hydroxybenzotriazole (163 mg, 1.07 mmol) and 1-ethyl-3-(3'-dimethylaminopropyl)carbodiimide hydrochloride (0.100 mL, 0.644 mmol), and the mixture was stirred at room temperature for 10 hours. To the reaction mixture was added saturated aqueous sodium hydrogencarbonate (30 mL), and the mixture was extracted with ethyl acetate. The organic layer was washed with brine, and then dried over anhydrous sodium sulfate, and the solvent was evaporated under reduced pressure. The residue was purified by preparative thin layer chromatography (hexane/ethyl acetate = 1/3) to give Compound 36 (93.3 mg, 75%).
APCI-MS m/z: 548 (M+H)⁺.

### Example 37 (Compound 37)

Compound 36 (79.0 mg, 0.149 mmol) was dissolved in dichloromethane (5 mL). To this solution was added trifluoroacetic acid (0.5 mL), and the mixture was stirred at room temperature for 3 hours. Then, the reaction mixture was evaporated under reduced pressure. The residue was purified by preparative thin layer chromatography (chloroform/methanol/concentrated aqueous ammonia = 100/10/1) to give Compound 37 (61.0 mg, 91%).
APCI-MS m/z: 448 (M+H)⁺.

### Example 38 (Compound 38)

Compound 37 (43.6 mg, 0.0974 mmol) was dissolved in acetonitrile (5 mL). To the solution was added 4-dimethylaminopyridine (26.6 mg, 0.218 mmol) and acetic anhydride (0.0368 mL, 0.389 mmol), and the mixture was stirred at room temperature for 12 hours. To the reaction mixture was added saturated aqueous sodium hydrogencarbonate (30 mL), and the mixture was extracted with ethyl acetate. The organic layer was washed with brine, and then dried over anhydrous sodium sulfate, and the solvent was evaporated under reduced pressure. The residue was purified by preparative thin layer chromatography (chloroform/methanol = 20/1) to give Compound 38 (12.7 mg, 27%).
APCI-MS m/z: 488 (M-H)⁻.

### Example 39 (Compound 39)

In a manner similar to that in Example 36, Compound 39 (140 mg, 100%) was obtained from trifluoroacetate of Compound m (107 mg, 0.218 mmol) prepared in Reference Example 13, N-tert-butoxycarbonyl-L-proline (148 mg, 0.687 mmol), 1-hydroxybenzotriazole (209 mg, 1.35 mmol) and 1-ethyl-3-(3'-dimethylaminopropyl)carbodiimide hydrochloride (0.120 mL, 0.784 mmol).
APCI-MS m/z: 572 (M-H)⁻.

### Example 40 (Compound 40)

In a manner similar to that in Example 37, Compound 40 (76.9 mg, 82%) was obtained from Compound 39 (114 mg, 0.199 mmol) and trifluoroacetic acid (0.5 mL).
APCI-MS m/z 474 (M+H)⁺.

### Example 41 (Compound 41)

In a manner similar to that in Example 36, Compound 41 (110 mg, 92%) was obtained from trifluoroacetate of Compound m (107 mg, 0.218 mmol) prepared in Reference Example 13, N-tert-butoxycarbonyl-L-alanine (130 mg, 0.686 mmol), 1-hydroxybenzotriazole (209 mg, 1.35 mmol) and 1-ethyl-3-(3'-dimethylaminopropyl)carbodiimide hydrochloride (0.120 mL, 0.784 mmol).
APCI-MS m/z: 546 (M-H)⁻.

### Example 42 (Compounds 42 and 43)

In a manner similar to that in Example 37, each of Compound 42 (27.9 mg, 45%) and Compound 43 (25.1 mg, 41%) was obtained as a diastereomer from Compound 41 (75.7 mg, 0.138 mmol) and trifluoroacetic acid (0.5 mL).
Compound 42 APCI-MS m/z: 448 (M+H)⁺.
Compound 43 APCI-MS m/z: 448 (M+H)⁺.

### Example 43 (Compound 44)

In a manner similar to that in Example 36, Compound 44 (106 mg, 88%) was obtained from trifluoroacetate of Compound m (107 mg, 0.218 mmol) prepared in Reference Example 13, N-tert-butoxycarbonyl-N-methylglycine (138 mg, 0.727 mmol), 1-hydroxybenzotriazole (209 mg, 1.35 mmol) and 1-ethyl-3-(3'-dimethylaminopropyl)carbodiimide hydrochloride (0.120 mL, 0.784 mmol).
APCI-MS m/z: 546 (M-H)⁻.

### Example 44 (Compound 45)

In a manner similar to that in Example 37, Compound 45 (61.4 mg, 97%) was obtained from Compound 44 (77.8 mg, 0.142 mmol) and trifluoroacetic acid (0.5 mL).
APCI-MS m/z: 448 (M+H)⁺.

### Example 45 (Compound 46)

In a manner similar to that in Example 36, Compound 46 (58.9 mg, 51%) was obtained from trifluoroacetate of Compound m (107 mg, 0.218 mmol) prepared in Reference Example 13, N-tert-butoxycarbonylglycine (121 mg, 0.688 mmol), 1-hydroxybenzotriazole (209 mg, 1.35 mmol) and 1-ethyl-3-(3'-dimethylaminopropyl)carbodiimide hydrochloride (0.120 mL, 0.784 mmol).
APCI-MS m/z: 532 (M-H)⁻.

### Example 46 (Compound 47)

In a manner similar to that in Example 37, Compound 47 (36.6 mg, 100%) was obtained from Compound 46 (39.8 mg, 0.0750 mmol) and trifluoroacetic acid (0.5 mL).
APCI-MS m/z: 434 (M+H)⁺.

### Example 47 (Compound 48)

In a manner similar to that in Example 36, Compound 48 (68.7 mg, 68%) was obtained from trifluoroacetate of Compound m (107 mg, 0.218 mmol) prepared in Reference Example 13, N,N-dimethylglycine (80.1 mg, 0.777 mmol), 1-hydroxybenzotriazole (209 mg, 1.35 mmol) and 1-ethyl-3-(3'-dimethylaminopropyl)carbodiimide hydrochloride (0.120 mL, 0.784 mmol).
APCI-MS m/z: 460 (M-H)⁻.

### Example 48 (Compound 49)

In a manner similar to that in Example 36, Compound 49 (87.1 mg, 89%) was obtained from trifluoroacetate of Compound m (101 mg, 0.206 mmol) prepared in Reference Example 13, N-acetylglycine (112 mg, 0.956 mmol), 1-hydroxybenzotriazole (215 mg, 1.40 mmol) and 1-ethyl-3-(3'-dimethylaminopropyl)carbodiimide hydrochloride (0.120 mL, 0.784 mmol).
APCI-MS m/z: 474 (M-H)⁻.

### Example 49 (Compound 50)

In a manner similar to that in Example 36, Compound 50 (44.4 mg, 36%) was obtained from trifluoroacetate of Compound m (107 mg, 0.218 mmol) prepared in Reference Example 13, N-tert-butoxycarbonyl-γ-aminobutyric acid (129 mg, 0.633 mmol), 1-hydroxybenzotriazole (209 mg, 1.35 mmol) and 1-ethyl-3-(3'-dimethylaminopropyl)carbodiimide hydrochloride (0.120 mL, 0.784 mmol).

### Example 50 (Compound 51)

In a manner similar to that in Example 40, Compound 51 (18.0 mg, 61%) was obtained from Compound 50 (36.0 mg, 0.0640 mmol) prepared in Example 49 and trifluoroacetic acid (0.3 mL).
APCI-MS m/z: 462 (M+H)⁺.

### Example 51 (Compound 52)

In a manner similar to that in Example 36, Compound 52 (87.5 mg, 75%) was obtained from trifluoroacetate of Compound o (105 mg, 0.208 mmol) prepared in Reference Example 15, N-tert-butoxycarbonyl-β-alanine (122 mg, 0.643 mmol), 1-hydroxybenzotriazole (169 mg, 1.10 mmol) and 1-ethyl-3-(3'-dimethylaminopropyl)carbodiimide hydrochloride (0.120 mL, 0.784 mmol).
APCI-MS m/z: 562 (M+H)⁺.

### Example 52 (Compound 53)

In a manner similar to that in Example 37, trifluoroacetate of Compound 53 (59.7 mg, 86%) was obtained from Compound 52 (67.9 mg, 0.121 mmol) prepared in Example 51 and trifluoroacetic acid (0.5 mL).
APCI-MS m/z: 462 (M+H)⁺.

### Example 53 (Compound 54)

In a manner similar to that in Example 36, Compound 54 (80.5 mg, 72%) was obtained from trifluoroacetate of Compound o (103 mg, 0.203 mmol) prepared in Reference Example 15, N-tert-butoxycarbonylglycine (116 mg, 0.664 mmol), 1-hydroxybenzotriazole (190 mg, 1.23 mmol) and 1-ethyl-3-(3'-dimethylaminopropyl)carbodiimide hydrochloride (0.120 mL, 0.784 mmol).
APCI-MS m/z: 548 (M+H)⁺.

### Example 54 (Compound 55)

In a manner similar to that in Example 37, Compound 55 (52.0 mg, 94%) was obtained from Compound 54 (67.6 mg, 0.123 mmol) prepared in Example 53 and trifluoroacetic acid (0.5 mL).
APCI-MS m/z: 448 (M+H)⁺.

### Example 55 (Compound 56)

In a manner similar to that in Example 36, Compound 56 (66.3 mg, 59%) was obtained from trifluoroacetate of Compound o (101 mg, 0.201 mmol) prepared in Reference Example 15, N-tert-butoxycarbonyl-L-alanine (128 mg, 0.678 mmol), 1-hydroxybenzotriazole (168 mg, 1.08 mmol) and 1-ethyl-3-(3'-dimethylaminopropyl)carbodiimide hydrochloride (0.120 mL, 0.784 mmol).
APCI-MS m/z: 560 (M-H)⁻.

### Example 56 (Compound 57)

In a manner similar to that in Example 37, Compound 57 (37.2 mg, 83%) was obtained from Compound 56 (54.3 mg, 0.0970 mmol) prepared in Example 55 and trifluoroacetic acid (0.5 mL).
APCI-MS m/z: 462 (M+H)⁺.

### Example 57 (Compound 58)

In a manner similar to that in Example 36, Compound 58 (94.7 mg, 82%) was obtained from trifluoroacetate of Compound o (102 mg, 0.202 mmol) prepared in Reference Example 15, N-tert-butoxycarbonyl-L-proline (140 mg, 0.651 mmol), 1-hydroxybenzotriazole (173 mg, 1.11 mmol) and 1-ethyl-3-(3'-dimethylaminopropyl)carbodiimide hydrochloride (0.120 mL, 0.784 mmol).
APCI-MS m/z: 588 (M+H)⁺.

### Example 58 (Compound 59)

In a manner similar to that in Example 37, Compound 59 (51.2 mg, 88%) was obtained from Compound 58 (70.5 mg, 0.120 mmol) prepared in Example 57 and trifluoroacetic acid (0.5 mL).
APCI-MS m/z: 488 (M+H)⁺.

### Example 59 (Compound 60)

In a manner similar to that in Example 36, Compound 60 (76.4 mg, 78%) was obtained from trifluoroacetate of Compound o (104 mg, 0.206 mmol) prepared in Reference Example 15, N,N-dimethylglycine (73.3 mg, 0.711 mmol), 1-hydroxybenzotriazole (183 mg, 1.18 mmol) and 1-ethyl-3-(3'-dimethylaminopropyl)carbodiimide hydrochloride (0.120 mL, 0.784 mmol).
APCI-MS m/z: 476 (M+H)⁺.

### Example 60 (Compound 61)

In a manner similar to that in Example 36, Compound 61 (75.4 mg, 66%) was obtained from trifluoroacetate of Compound o (102 mg, 0.202 mmol) prepared in Reference Example 15, N-tert-butoxycarbonyl-N-methylglycine (122 mg, 0.645 mmol), 1-hydroxybenzotriazole (165 mg, 1.06 mmol) and 1-ethyl-3-(3'-dimethylaminopropyl)carbodiimide hydrochloride (0.120 mL, 0.784 mmol).
APCI-MS m/z: 562 (M+H)⁺.

### Example 61 (Compound 62)

In a manner similar to that in Example 37, Compound 62 (45.9 mg, 90%) was obtained from Compound 61 (61.8 mg, 0.110 mmol) prepared in Example 60 and trifluoroacetic acid (0.5 mL).
APCI-MS m/z: 462 (M+H)⁺.

### Example 62 (Compound 63)

In a manner similar to that in Example 36, Compound 63 (75.1 mg, 69%) was obtained from trifluoroacetate of Compound o (103 mg, 0.204 mmol) prepared in Reference Example 15, 3-piperidinopropionic acid (103 mg, 0.656 mmol), 1-hydroxybenzotriazole (199 mg, 1.28 mmol) and 1-ethyl-3-(3'-dimethylaminopropyl)carbodiimide hydrochloride (0.120 mL, 0.784 mmol).
APCI-MS m/z: 530 (M+H)⁺.

### Example 63 (Compound 64)

In a manner similar to that in Example 36, Compound 64 (61.3 mg, 60%) was obtained from trifluoroacetate of Compound o (102 mg, 0.202 mmol) prepared in Reference Example 15, N,N-dimethyl-γ-aminobutyric acid (119 mg, 0.710 mmol), 1-hydroxybenzotriazole (178 mg, 1.15 mmol) and 1-ethyl-3-(3'-dimethylaminopropyl)carbodiimide hydrochloride (0.120 mL, 0.784 mmol).
APCI-MS m/z: 504 (M+H)⁺.

### Example 64 (Compound 65)

In a manner similar to that in Example 36, Compound 65 (63.8 mg, 67%) was obtained from trifluoroacetate of Compound o (103 mg, 0.205 mmol) prepared in Reference Example 15, methoxyacetic acid (0.0500 mL, 0.652 mmol), 1-hydroxybenzotriazole (192 mg, 1.24 mmol) and 1-ethyl-3-(3'-dimethylaminopropyl)carbodiimide hydrochloride (0.120 mL, 0.784 mmol).
APCI-MS m/z: 463 (M+H)⁺.

### Example 65 (Compound 66)

In a manner similar to that in Example 36, Compound 66 (71.5 mg, 65%) was obtained from trifluoroacetate of Compound o (113 mg, 0.224 mmol) prepared in Reference Example 15, N-acetylglycine (101 mg, 0.858 mmol), 1-hydroxybenzotriazole (209 mg, 1.37 mmol) and 1-ethyl-3-(3'-dimethylaminopropyl)carbodiimide hydrochloride (0.120 mL, 0.784 mmol).
APCI-MS m/z: 488 (M-H)⁻.

### Example 66 (Compound 67)

In a manner similar to that in Example 36, Compound 67 (108 mg, 100%) was obtained from hydrochloride of Compound r (77.0 mg, 0.175 mmol) prepared in Reference Example 18, N-tert-butoxycarbonyl-L-proline (108 mg, 0.502 mmol), 1-hydroxybenzotriazole (187 mg, 1.21 mmol) and 1-ethyl-3-(3'-dimethylaminopropyl)carbodiimide hydrochloride (0.120 mL, 0.784 mmol).
APCI-MS m/z: 602 (M+H)⁺.

### Example 67 (Compound 68)

In a manner similar to that in Example 37, Compound 68 (70.9 mg, 93%) was obtained from Compound 67 (91.6 mg, 0.152 mmol) prepared in Example 66 and trifluoroacetic acid (0.5 mL).
APCI-MS m/z: 502 (M+H)⁺.

### Example 68 (Compound 69)

In a manner similar to that in Example 36, Compound 69 (81.5 mg, 100%) was obtained from hydrochloride of Compound r (74.4 mg, 0.152 mmol) prepared in Reference Example 18, N,N-dimethylglycine (94.9 mg, 0.920 mmol), 1-hydroxybenzotriazole (204 mg, 1.32 mmol) and 1-ethyl-3-(3'-dimethylaminopropyl)carbodiimide hydrochloride (0.120 mL, 0.784 mmol).
APCI-MS m/z: 490 (M+H)⁺.

### Example 69 (Compound 70)

In a manner similar to that in Example 36, Compound 70 (103 mg, 100%) was obtained from hydrochloride of Compound r (75.8 mg, 0.172 mmol) prepared in Reference Example 18, N-tert-butoxycarbonyl-N-methylglycine (95.0 mg, 0.502 mmol), 1-hydroxybenzotriazole (198 mg, 1.28 mmol) and 1-ethyl-3-(3'-dimethylaminopropyl)carbodiimide hydrochloride (0.120 mL, 0.784 mmol).
APCI-MS m/z: 576 (M+H)⁺.

### Example 70 (Compound 71)

In a manner similar to that in Example 37, Compound 71 (64.0 mg, 90%) was obtained from Compound 70 (86.2 mg, 0.150 mmol) prepared in Example 69 and trifluoroacetic acid (0.5 mL).
APCI-MS m/z: 476 (M+H)⁺.

### Example 71 (Compound 72)

In a manner similar to that in Example 36, Compound 72 (96.9 mg, 99%) was obtained from hydrochloride of Compound r (76.8 mg, 0.174 mmol) prepared in Reference Example 18, N-tert-butoxycarbonylglycine (97.7 mg, 0.558 mmol), 1-hydroxybenzotriazole (187 mg, 1.21 mmol) and 1-ethyl-3-(3'-dimethylaminopropyl)carbodiimide hydrochloride (0.120 mL, 0.784 mmol).
APCI-MS m/z: 562 (M+H)⁺.

### Example 72 (Compound 73)

In a manner similar to that in Example 37, Compound 73 (61.1 mg, 92%) was obtained from Compound 72 (80.9 mg, 0.144 mmol) prepared in Example 71 and trifluoroacetic acid (0.5 mL).
APCI-MS m/z: 462 (M+H)⁺.

### Example 73 (Compound 74)

In a manner similar to that in Example 38, Compound 74 (45.6 mg, 90%) was obtained from hydrochloride of Compound r (50.3 mg, 0.114 mmol) prepared in Reference Example 18, 4-dimethylaminopyridine (33.7 mg, 0.276 mmol) and acetic anhydride (0.0500 mL, 0.529 mmol).
APCI-MS m/z: 447 (M+H)⁺.

### Example 74 (Compound 75)

In a manner similar to that in Reference Example 4, Compound 75 (52.4 mg, 85%) was obtained from Compound g (51.3 mg, 0.127 mmol) prepared in Reference Example 8, acetic acid (0.0440 mL, 0.769 mmol), 1-methyl-piperazine (0.0710 mL, 0.635 mmol) and triacetoxy sodium borohydride (119 mg, 0.563 mmol).
APCI-MS m/z: 488 (M+H)⁺.

### Example 75 (Compound 76)

In a manner similar to that in Reference Example 4, Compound 76 (18.0 mg, 18%) was obtained from Compound g (86.0 mg, 0.213 mmol) prepared in Example 8, acetic acid (0.0730 mL, 1.28 mmol), 2-amino-1,3-propanediol (100 mg, 1.102 mmol) and triacetoxy sodium borohydride (202 mg, 0.952 mmol).
APCI-MS m/z: 479 (M+H)⁺.

### Example 76 (Compound 77)

In a manner similar to that in Reference Example 4, Compound 77 (21.9 mg, 23%) was obtained from Compound g (81.4 mg, 0.202 mmol) prepared in Example 8, acetic acid (0.0730 mL, 1.28 mmol), 3-amino-1,2-propanediol (88.2 mg, 0.968 mmol) and triacetoxy sodium borohydride (202 mg, 0.952 mmol).
APCI-MS m/z: 479 (M+H)⁺.

### Example 77 (Compound 78)

### Step 1

N-tert-Butoxycarbonyl-γ-aminobutyric acid (10 g, 49.2 mmol) was dissolved in THF (100 mL). To the solution was added N,N'-carbonyldiimidazole (14.3 g, 59.0 mmol), and the mixture was stirred at room temperature for 30 minutes. Then, to the reaction mixture was added N,O-dimethylhydroxyamine hydrochloride (6.2 g, 64.0 mmol), and the mixture was stirred at room temperature for 12 hours. To the reaction mixture was added water, and the mixture was extracted with ethyl acetate. The organic layer was washed with brine, and then dried over anhydrous sodium sulfate. The solvent was evaporated under reduced pressure to give 1-(tert-butoxycarbonylamino)-3-(N-methoxy-N-methylcarbamoyl)propane (9.55 g, 80%).
APCI-MS m/z: 247 (M+H)⁺.

### Step 2

1-(tert-Butoxycarbonylamino)-3-(N-methoxy-N-methylcarbamoyl)propane obtained above was dissolved in THF (300 mL). To this solution was added a 2.0 mol/L solution of isopropyl magnesium chloride in THF (18.4 mL, 36.8 mmol) at -10°C, and the mixture was stirred at the same temperature for 15 minutes. Then, to the reaction mixture was added a 2.0 mol/L solution of phenyl magnesium chloride in THF (21.3 mL, 42.7 mmol) at -10°C, and the mixture was stirred at room temperature for 3 hours. To the reaction mixture was added acetic acid (5.6 mL), and the mixture was concentrated under reduced pressure. To the residue was added water, and the mixture was extracted with ethyl acetate. The organic layer was washed with brine, and then dried over anhydrous sodium sulfate. The solvent was evaporated under reduced pressure, and the residue was purified by silica gel column chromatography (hexane/ethyl acetate = 6/1→4/1) to give 4-(tert-butoxycarbonylamino)butyrophenone (3.95 g, 39%).
APCI-MS m/z: 264 (M+H)⁺.

### Step 3

4-(tert-Butoxycarbonylamino)butyrophenone obtained above was dissolved in a mixed solvent of methanol (80 mL) and distilled water (20 mL). To this solution was added thiosemicarbazide hydrochloride (3.80 g, 30.0 mmol), and the mixture was stirred at room temperature for 5 hours. The reaction mixture was concentrated under reduced pressure. To the residue was added water, and the mixture was extracted with ethyl acetate. The organic layer was washed with brine, and then dried over anhydrous sodium sulfate, and the solvent was evaporated under reduced pressure. The residue was reslurried in hexane-diethyl ether to give 4-(tert-butoxycarbonylamino)butyrophenone=thiosemicarbazone (3.86 g, 76%).
APCI-MS m/z: 337 (M+H)⁺.

### Step 4

4-(tert-Butoxycarbonylamino)butyrophenone=thiosemicarbazone (1.69 g, 5.02 mmol) obtained above was dissolved in dichloromethane (50 mL). To the solution was added pyridine (3.30 mL, 40.2 mmol) and trimethylacetyl chloride (3.1 mL, 25.1 mmol), and the mixture was stirred at room temperature for 12 hours. To the reaction mixture was added 1 mol/L hydrochloric acid, and the mixture was stirred at room temperature for 1 hour, and then extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 4/1) to give Compound q (2.02 g, 80%) described in Reference Example 17.
APCI-MS m/z: 505 (M+H)⁺.

### Step 5

Compound q (0.674 g, 1.34 mmol) obtained above was dissolved in 4 mol/L hydrogen chloride-ethyl acetate (20 mL). The solution was stirred at room temperature for 30 minutes, and the reaction mixture was concentrated under reduced pressure. The residue was reslurried in diethyl ether to give hydrochloride of Compound r (574 mg, 98%) described in Reference Example 18.
ESI-MS m/z: 405 (M+H)⁺.

### Step 6

To hydrochloride of Compound r (450 mg, 1.02 mmol) obtained above was added dichloromethane (40 mL) and triethylamine (2.5 mL, 17.7 mmol), and the mixture was stirred. Then, to the mixture was added 2-chloro-1-ethanesulfonyl chloride (0.74 mL, 7.07 mmol), and the mixture was stirred at room temperature for 30 minutes. To the reaction mixture was added water, and the mixture was extracted with chloroform. The organic layer was washed with brine, and then dried over anhydrous sodium sulfate, and the solvent was evaporated under reduced pressure. The residue was purified by silica gel column chromatography (hexane/ethyl acetate = 3/2→1/1) to give Compound 78 (169 mg, 34%).
APCI-MS m/z: 495 (M+H)⁺.

### Example 78 (Compound 79)

Compound 78 (490 mg, 0.991 mmol) prepared in Example 77 was dissolved in acetonitrile (5 mL), methanol (5 mL) and saturated aqueous sodium hydrogencarbonate (5 mL). To the solution was added dimethylamine hydrochloride (808 mg, 9.91 mmol), and the mixture was stirred at room temperature for 15 minutes. To the reaction mixture was added water, and the mixture was extracted with ethyl acetate. The organic layer was washed with brine, and then dried over anhydrous sodium sulfate, and the solvent was evaporated under reduced pressure. The residue was purified by preparative thin layer chromatography (chloroform/methanol/triethylamine = 90/10/0.1). The resulting crude product was reslurried in 4 mol/L hydrogen chloride-ethyl acetate (20 mL) and diethyl ether to give Compound 79 (206 mg, 36%) as hydrochloride.
APCI-MS m/z: 540 (M+H)⁺.

### Example 79 (Compound 80)

Compound 78 (505 mg, 1.02 mmol) prepared in Example 77 was dissolved in 7 mol/L ammonia - methanol (100 mL), and the solution was stirred at room temperature for 12 hours. The reaction mixture was concentrated under reduced pressure, and the residue was purified by silica gel column chromatography (chloroform/methanol/triethylamine = 6/1/0.35). The resulting colorless powder was dissolved in 10% hydrogen chloride - methanol (10 mL). To the solution was added diethyl ether (50 mL), and the resulting crystals were collected by filtration, and dried to give Compound 80 (235 mg, 43%).
APCI-MS m/z: 512 (M+H)⁺.

### Example 80 (Compound 81)

Compound q (0.81 g, 1.60 mmol) prepared in Reference Example 17 was dissolved in tert-butanol (35 mL). To the solution was added 1 mol/L hydrochloric acid in 1 mol/L sodium acetate buffer (pH = 3, 12 mL) and sodium borohydride (0.60 g, 16.0 mmol), and the mixture was stirred at 60°C for 15 minutes. To the reaction mixture was added acetic acid (2.7 mL), and the mixture was concentrated under reduced pressure. To the residue was added water, and the mixture was extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 3/1→1/1) to give Compound 81 (323 mg, 48%).
APCI-MS m/z: 421 (M+H)⁺.

### Example 81 (Compound 82)

Compound 81 (323 mg, 0.768 mmol) prepared in Example 80 was dissolved in dichloromethane (10 mL). To the solution was added pyridine (0.230 mL, 2.69 mmol) and 5-bromovaleryl chloride (0.206 mL, 1.54 mmol), and the mixture was stirred at room temperature for 30 minutes. To the reaction mixture was added water, and the mixture was extracted with ethyl acetate. The organic layer was washed with brine, and then dried over anhydrous sodium sulfate, and the solvent was evaporated under reduced pressure. The residue was dissolved in dimethyl sulfoxide (DMSO) (10 mL). To the solution was added sodium acetate (0.315 mg, 3.84 mmol), and the mixture was stirred at room temperature for 24 hours. To the reaction mixture was added water, and the mixture was extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 3/2) to give Compound 82 (0.386 g, 99%).
APCI-MS m/z: 503 (M+H)⁺.

### Example 82 (Compound 83)

In a manner similar to that in Step 5 of Example 77, Compound 83 (0.217 g, 64%) was obtained from Compound 82 (0.386 g, 0.768 mmol) prepared in Example 81 and 4 mol/L hydrogen chloride-ethyl acetate (10 mL).
APCI-MS m/z: 403 (M+H)⁺.

### Example 83 (Compound 84)

In a manner similar to that in Step 6 of Example 77, Compound 84 (0.205 mg, 99%) was obtained from Compound 83 (185 mg, 0.417 mmol) prepared in Example 82, triethylamine (0.290 mL, 2.11 mmol) and 2-chloro-1-ethanesulfonyl chloride (0.066 mL, 0.632 mmol).
APCI-MS m/z: 493 (M+H)⁺.

### Example 84 (Compound 85)

In a manner similar to that in Example 78, Compound 85 (0.177 mg, 77%) was obtained from Compound 84 (0.205 mg, 0.417 mmol) prepared in Example 83 and dimethylamine hydrochloride (0.348 g, 4.26 mmol).
APCI-MS m/z: 538 (M+H)⁺.

### Example 85 (Compound 86)

4-(tert-Butoxycarbonylamino)butyrophenone=thiosemicarbazone (0.968 mg, 4.09 mmol) prepared in Step 3 of Example 77 was dissolved in acetone (20 mL). To the solution was added pyridine (1.7 mL, 20.5 mmol) and acetic anhydride (1.9 mL, 20.5 mmol), and the mixture was stirred at room temperature for 24 hours. To the reaction mixture was added saturated aqueous sodium hydrogencarbonate, and the mixture was extracted with ethyl acetate. The organic layer was washed with brine, and then dried over anhydrous sodium sulfate, and the solvent was evaporated under reduced pressure. To the residue was added methanol (30 mL) and hydrazine monohydrate (20 mL), and the mixture was stirred at room temperature for 1 hour. To the reaction mixture was added 1 mol/L hydrochloric acid, and the mixture was extracted with ethyl acetate. The organic layer was washed with brine, and then dried over anhydrous sodium sulfate, and the solvent was evaporated under reduced pressure. The residue was reslurried in diisopropyl ether to give Compound 86 (0.910 mg, 59%).
APCI-MS m/z: 379 (M+H)⁺.

### Example 86 (Compound 87)

Compound 86 (0.334 g, 0.883 mmol) prepared in Example 85 was dissolved in dichloromethane (10 mL). To the solution was added pyridine (0.376 mL, 4.41 mmol) and trimethylacetyl chloride (0.326 mL, 2.65 mmol), and the mixture was stirred at room temperature for 12 hours. To the reaction mixture was added hydrochloric acid, and the mixture was stirred at room temperature for 1 hour. Then, the mixture was extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 3/2→1/1) to give Compound 87 (0.327 g, 80%).
APCI-MS m/z: 463 (M+H)⁺.

### Example 87 (Compound 88)

In a manner similar to that in Step 5 of Example 77, Compound 88 (214 mg, 77%) was obtained from Compound 87 (327 mg, 0.707 mmol) prepared in Example 86 and 4 mol/L hydrogen chloride-ethyl acetate.
¹H NMR (270 MHz, CDCl₃) δ (ppm): 1.24 (s, 9H), 2.17 (m, 2H), 2.28 (s, 3H), 2.39 (m, 1H), 3.03 (m, 2H), 3.23 (m, 1H), 7.21-7.45 (m, 5H).
APCI-MS m/z: 363 (M+H)⁺.

### Example 88 (Compound 89)

Compound 86 (299 mg, 0.790 mmol) prepared in Example 85 was dissolved in dichloromethane (8 mL). To the solution was added pyridine (0.202 mL, 2.37 mmol) and 4-bromobutyryl chloride (0.230 mL, 1.98 mmol), and the mixture was stirred at room temperature for 30 minutes. To the reaction mixture was added water, and the mixture was extracted with ethyl acetate. The organic layer was washed with brine, and then dried over anhydrous sodium sulfate, and the solvent was evaporated under reduced pressure. Then, the residue was dissolved in DMSO (3 mL), to the reaction mixture was added sodium acetate (0.324 mg, 3.95 mmol), and the mixture was stirred at room temperature for 24 hours. To the reaction mixture was added water, and the mixture was extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 1/1→2/3) to give Compound 89 (0.265g, 75%).
APCI-MS m/z: 447 (M+H)⁺.

### Example 89 (Compound 90)

In a manner similar to that in Step 5 of Example 77, Compound 90 (0.195 g, 86%) was obtained from Compound 89 (0.265 g, 0.593 mmol) prepared in Example 88 and 4 mol/L hydrogen chloride-ethyl acetate (10 mL).
APCI-MS m/z: 347 (M+H)⁺.

### Example 90 (Compound 91)

In a manner similar to that in Example 81, Compound 91 (0.267 g, 80%) was obtained from Compound 86 (274 mg, 0.724 mmol) prepared in Example 85, pyridine (0.185 mL, 2.17 mmol), 5-bromovaleryl chloride (0.242 mL, 1.81 mmol) and sodium acetate (0.324 mg, 3.95 mmol).
APCI-MS m/z: 461 (M+H)⁺.

### Example 91 (Compound 92)

In a manner similar to that in Step 5 of Example 77, Compound 92 (0.181 g, 79%) was obtained from Compound 91 (0.267 g, 0.580 mmol) prepared in Example 90 and 4 mol/L hydrogen chloride-ethyl acetate (10 mL).
APCI-MS m/z: 361 (M+H)⁺.

### Example 92 (Compound 93)

Pyrrole (0.0153 mL, 0.221 mmol) was dissolved in DMF (1 mL). To the solution was added sodium hydride (11.1 mg, 0.278 mmol), and the mixture was cooled to 0°C. Then, to the mixture was added Compound s (30.5 mg, 0.0631 mmol) prepared in Reference Example 19, and the mixture was stirred at room temperature for 14 hours. To the reaction mixture was added water and saturated aqueous sodium hydrogencarbonate, and the solution was extracted with chloroform. The organic layer was washed with brine, and then dried over anhydrous sodium sulfate, and the solvent was evaporated under reduced pressure. The resulting residue was purified by silica gel column chromatography (chloroform/methanol = 40/1) to give Compound 93 (0.0054 g, 19%).
APCI-MS m/z: 455 (M+H)⁺.

### Example 93 (Compound 94)

In a manner similar to that in Example 92, Compound 94 (12.4 mg, 40%) was obtained from Compound s (33.2 mg, 0.0686 mmol) prepared in Reference Example 19, imidazole (19.1 mg, 2.81 mmol) and sodium hydride (11.1 mg, 0.278 mmol).
APCI-MS m/z: 456 (M+H)⁺.

### Example 94 (Compound 95)

In a manner similar to that in Reference Example 4, Compound 95 (42.5 mg, 69%) was obtained from Compound g (50.0 mg, 0.124 mmol) prepared in Reference Example 8, acetic acid (0.090 mL, 1.57 mmol), 2-picolylamine (0.0650 mL, 0.968 mmol) and triacetoxy sodium borohydride (116 mg, 0.547 mmol).
APCI-MS m/z: 496 (M+H)⁺.

### Example 95 (Compound 96)

In a manner similar to that in Reference Example 4, Compound 96 (56.0 mg, 90%) was obtained from Compound g (50.6 mg, 0.125 mmol) prepared in Reference Example 8, acetic acid (0.0450 mL, 0.786 mmol), 3-picolylamine (0.0650 mL, 0.968 mmol) and triacetoxy sodium borohydride (128 mg, 0.603 mmol).
APCI-MS m/z: 496 (M+H)⁺.

### Example 96 (Compound 97)

In a manner similar to that in Reference Example 4, Compound 97 (50 mg, 40%) was obtained from Compound g (100 mg, 0.248 mmol) prepared in Reference Example 8, acetic acid (0.090 mL, 1.572 mmol), (S)-(-)-1-phenylethylamine (150 mg, 1.24 mmol) and triacetoxy sodium borohydride (210 mg, 0.992 mmol).
APCI-MS m/z: 509 (M+H)⁺.

### Example 97 (Compound 98)

In a manner similar to that in Reference Example 4, Compound 98 (96.7 mg, 80%) was obtained from Compound g (101 mg, 0.250 mmol) prepared in Reference Example 8, acetic acid (0.0900 mL, 1.57 mmol), 3-aminopyridine (138 mg, 1.47 mmol) and triacetoxy sodium borohydride (214 mg, 1.01 mmol).
APCI-MS m/z: 425 (M+H)⁺.

### Example 98 (Compound 99)

In a manner similar to that in Reference Example 4, Compound 99 (96.8 mg, 77%) was obtained from Compound g (102 mg, 0.253 mmol) prepared in Reference Example 8, acetic acid (0.090 mL, 1.572 mmol), 4-(aminomethyl)pyridine (136 mg, 1.26 mmol) and triacetoxy sodium borohydride (214 mg, 1.01 mmol).
APCI-MS m/z: 496 (M+H)⁺.

### Example 99 (Compound 100)

In a manner similar to that in Reference Example 4, Compound 100 (563 mg, 95%) was obtained from Compound g (490 mg, 1.21 mmol) prepared in Reference Example 8, acetic acid (0.420 mL, 7.34 mmol), piperazin-2-one (606 mg, 6.05 mmol) and triacetoxy sodium borohydride (1.02 g, 4.84 mmol).
APCI-MS m/z: 488 (M+H)⁺.

### Example 100 (Compound 101)

In a manner similar to that in Reference Example 4, Compound 101 (110 mg, 85%) was obtained from Compound g (100 mg, 0.248 mmol) prepared in Reference Example 8, acetic acid (0.0900 mL, 1.57 mmol), 1-acetylpiperazine (159 mg, 1.24 mmol) and triacetoxy sodium borohydride (210 mg, 0.992 mmol).
APCI-MS m/z: 516 (M+H)⁺.

### Example 101 (Compound 102)

In a manner similar to that in Reference Example 4, Compound 102 (0.236 g, 82%) was obtained from Compound v (0.249 g, 0.663 mmol) prepared in Reference Example 22, acetic acid (0.240 mL, 4.20 mmol), diethylamine (0.243 g, 3.32 mmol) and triacetoxy sodium borohydride (0.562 g, 2.65 mmol).
APCI-MS m/z: 433 (M+H)⁺.

### Example 102 (Compound 103)

Compound 102 (34.3 mg, 0.0793 mmol) prepared in Example 101 was dissolved in methanol (0.5 mL). To the solution was added cerium(III) chloride heptahydrate (29.5 mg, 0.0793 mmol) and sodium borohydride (30.0 mg, 0.793 mmol), and the mixture was stirred at room temperature for 1 hour. To the reaction mixture was added water, and the mixture was extracted with chloroform. The organic layer was dried over anhydrous sodium sulfate, and the solvent was evaporated under reduced pressure. The residue was purified by silica gel column chromatography (chloroform/methanol = 90/10) to give Compound 103 (15.1 mg, 52%).

### Example 103 (Compound 104)

Compound 103 (15.1 mg, 0.0415 mmol) prepared in Example 102 was dissolved in dichloromethane (0.5 mL), and the solution was cooled to 0°C. To this solution was added pyridine (0.0128 mL, 0.150 mmol) and trimethylacetyl chloride (0.014 mL, 0.125 mmol), and the mixture was stirred at room temperature for 15 hours. To the reaction mixture was added water, and the mixture was extracted with chloroform. The organic layer was dried over anhydrous sodium sulfate, and then the solvent was evaporated under reduced pressure. The residue was purified by preparative thin layer chromatography (chloroform/methanol = 90/10) to give Compound 104 (9.20 mg, 50%).
AP-Ms m/z: 447 (M+H)⁺.

### Example 104 (Compound 105)

In a manner similar to that in Reference Example 4, Compound 105 (51.4 mg, 79%) was obtained from Compound g (50.1 mg, 0.124 mmol) prepared in Reference Example 8, acetic acid (0.0450 mL, 0.785 mmol), 4-ethylaminomethylpyridine (84.4 mg, 0.620 mmol) and triacetoxy sodium borohydride (105 mg, 0.496 mmol).
AP-Ms m/z: 524 (M+ H)⁺.

### Example 105 (Compound 106)

In a manner similar to that in Reference Example 4, Compound 106 (0.0435 g, 74%) was obtained from Compound g (0.0502 g, 0.124 mmol) prepared in Reference Example 8, acetic acid (0.045 mL, 0.786 mmol), N-ethylisopropylamine (0.0542 g, 0.622 mmol) and triacetoxy sodium borohydride (0.105 g, 0.496 mmol).
APCI-MS m/z: 475 (M+H)⁺.

### Example 106 (Compound 107)

In a manner similar to that in Reference Example 4, Compound 107 (0.0572 g, 68%) was obtained from Compound g (0.0710 g, 0.176 mmol) prepared in Reference Example 8, acetic acid (0.062 mL, 1.08 mmol), 2-(ethylamino)ethanol (0.0784 g, 0.880 mmol) and triacetoxy sodium borohydride (0.149 g, 0.704 mmol).
APCI-MS m/z: 477 (M+H)⁺.

### Example 107 (Compound 108)

In a manner similar to that in Reference Example 4, Compound 108 (0.0708 g, 77%) was obtained from Compound g (0.0750 g, 0.186 mmol) prepared in Reference Example 8, acetic acid (0.065 mL, 1.13 mmol), diethanolamine (0.0978 g, 0.930 mmol) and triacetoxy sodium borohydride (0.158 g, 0.774 mmol).
APCI-MS m/z: 493 (M+H)⁺.

### Example 108 (Compound 109)

In a manner similar to that in Reference Example 4, Compound 109 (0.0772 g, 71%) was obtained from Compound g (0.0985 g, 0.244 mmol) prepared in Reference Example 8, acetic acid (0.090 mL, 1.57 mmol), cyclopropylamine (0.0700 g, 1.22 mmol) and triacetoxy sodium borohydride (0.207 g, 0.976 mmol).
APCI-MS m/z: 445 (M+1)⁺.

### Example 109 (Compound 110)

In a manner similar to that in Reference Example 4, Compound 110 (0.0523 g, 82%) was obtained from Compound g (0.0516 g, 0.128 mmol) prepared in Reference Example 8, acetic acid (0.0450 mL, 0.785 mmol), dimethylaminoethylmethylamine (0.0654 g, 0.640 mmol) and triacetoxy sodium borohydride (0.109 g, 0.512 mmol).
APCI-MS m/z: 490 (M+H)⁺.

### Example 110 (Compound 111)

In a manner similar to that in Reference Example 4, Compound 111 (0.0133 g, 22%) was obtained from Compound g (0.0507 g, 0.126 mmol) prepared in Reference Example 8, acetic acid (0.045 mL, 0.786 mmol), diisopropylamine (0.0637 g, 0.630 mmol) and triacetoxy sodium borohydride (0.107 g, 0.504 mmol).
APCI-MS m/z: 489 (M+H)⁺.

### Example 111 (Compound 112)

Compound 109 (0.0452 g, 0.102 mmol) prepared in Example 108 was dissolved in dichloroethane (2.0 mL). To the solution was added acetaldehyde (0.0225 g, 0.51 mmol), acetic acid (0.038 mL, 0.664 mmol) and triacetoxy sodium borohydride (0.152 g, 0.717 mmol), and the mixture was stirred at room temperature for 24 hours. To the reaction mixture was added saturated aqueous sodium hydrogencarbonate (3 mL) and water (3 mL), and the mixture was extracted with ethyl acetate. The organic layer was washed with water, and then dried over anhydrous sodium sulfate, and the solvent was evaporated under reduced pressure. The resulting residue was purified by preparative thin layer chromatography (chloroform/methanol = 9/1) to give Compound 112 (0.0283 g, 59%).
APCI-MS m/z: 473 (M+H)⁺.

### Example 112 (Compound 113)

In a manner similar to that in Reference Example 4, Compound 113 (0.0673 g, 69%) was obtained from Compound g (0.0760 g, 0.188 mmol) prepared in Reference Example 8, acetic acid (0.065 mL, 1.14 mmol), 4-(2-aminoethyl)morpholine (0.122 g, 0.937 mmol) and triacetoxy sodium borohydride (0.159 g, 0.752 mmol).
APCI-MS m/z: 518 (M+H)⁺.

### Example 113 (Compound 114)

In a manner similar to that in Reference Example 4, Compound 114 (0.0702 g, 73%) was obtained from Compound g (0.0750 g, 0.186 mmol) prepared in Reference Example 8, acetic acid (0.065 mL, 1.14 mmol), 1-(2-hydroxyethyl)piperazine (0.121 g, 0.930 mmol) and triacetoxy sodium borohydride (0.157 g, 0.744 mmol).
APCI-MS m/z: 518 (M+H)⁺.

### Example 114 (Compound 115)

In a manner similar to that in Reference Example 4, Compound 115 (0.0626 g, 69%) was obtained from Compound g (0.0748 g, 0.185 mmol) prepared in Reference Example 8, acetic acid (0.065 mL, 1.14 mmol), 2-(isopropylamino)ethanol (0.0954 g, 0.925 mmol) and triacetoxy sodium borohydride (0.157 g, 0.740 mmol).
APCI-MS m/z: 491 (M+H)⁺.

### Example 115 (Compound 116)

In a manner similar to that in Reference Example 4, Compound 116 (0.0597 g, 63%) was obtained from Compound g (0.0760 g, 0.188 mmol) prepared in Reference Example 8, acetic acid (0.065 mL, 1.14 mmol), 1-methylhomopiperazine (0.102 g, 0.940 mmol) and triacetoxy sodium borohydride (0.159 g, 0.752 mmol).
APCI-MS m/z: 502 (M+H)⁺.

### Example 116 (Compound 117)

In a manner similar to that in Example 81, Compound 117 (0.0108 g, 22%) was obtained from Compound 103 (0.0421 g, 0.116 mmol) prepared in Example 102, pyridine (0.0135 mL, 0.167 mmol), 4-bromobutyryl chloride (0.0161 mL, 0.139 mmol) and sodium acetate (0.324 mg, 3.95 mmol).
APCI-MS m/z: 431 (M+1)⁺.

### Example 117 (Compound 118)

In a manner similar to that in Reference Example 4, Compound 118 (0.975 g, 99%) was obtained from Compound g (0.691 g, 1.70 mmol) prepared in Reference Example 8, acetic acid (0.600 mL, 10.5 mmol), 1-(tert-butoxycarbonyl)piperazine (1.58 g, 8.48 mmol) and triacetoxy sodium borohydride (1.44 g, 6.79 mmol).
APCI-MS m/z: 574 (M+H)⁺.

### Example 118 (Compound 119)

In a manner similar to that in Example 37, Compound 119 (0.749 g, 93%) was obtained from Compound 118 (0.975 g, 1.70 mmol) prepared in Example 117, trifluoroacetic acid (20 mL) and dichloromethane (30 ml).
APCI-MS m/z: 474 (M+H)⁺.

### Example 119 (Compound 120)

Compound 119 (0.051 g, 0.108 mmol) prepared in Example 118 was dissolved in dichloromethane (2.0 mL). Then, to the solution was added pyridine (0.0175 mL, 0.216 mmol) and isobutyryl chloride (0.0137 mL, 0.130 mmol) at 0°C, and the mixture was stirred at room temperature for 5 hours. To the reaction mixture was added 1 mol/L hydrochloric acid and water, and the mixture was extracted with chloroform. The organic layer was washed with water, and then dried over anhydrous sodium sulfate, and the solvent was evaporated under reduced pressure. The residue was purified by preparative thin layer chromatography (chloroform/methanol = 9/1) to give Compound 120 (0.0475 g, 81%).
APCI-MS m/z: 544 (M+H)⁺.

### Example 120 (Compound 121)

In a manner similar to that in Example 119, Compound 121 (0.0471 g, 77%) was obtained from Compound 119 (0.0508 g, 0.107 mmol) prepared in Example 118, pyridine (0.0173 mL, 0.214 mmol) and trifluoroacetic anhydride (0.0181 mL, 0.128 mmol).
APCI-MS m/z: 570 (M+H)⁺.

### Example 121 (Compound 122)

In a manner similar to that in Example 119, Compound 122 (0.0912 g, 82%) was obtained from Compound 119 (0.0527 g, 0.111 mmol) prepared in Example 118, triethylamine (0.0311 mL, 0.223 mmol) and methyl chloroformate (0.0103 mL, 0.133 mmol).
APCI-MS m/z: 532 (M+H)⁺.

### Example 122 (Compound 123)

In a manner similar to that in Example 119, Compound 123 (0.0531 g, 88%) was obtained from Compound 119 (0.0519 g, 0.111 mmol) prepared in Example 118, pyridine (0.0178 mL, 0.220 mmol) and methanesulfonyl chloride (0.0102 mL, 0.132 mmol).
APCI-MS m/z: 552 (M+H)⁺.

### Example 123 (Compound 124)

In a manner similar to that in Example 119, Compound 124 (0.0450 g, 78%) was obtained from Compound 119 (0.0502 g, 0.106 mmol) prepared in Example 118, triethylamine (0.0212 mL, 0.152 mmol) and dimethylcarbamoyl chloride (0.0117 mL, 0.127 mmol).
APCI-MS m/z: 545 (M+H)⁺.

### Example 124 (Compound 125)

In a manner similar to that in Example 119, Compound 127 (0.0355 g, 78%) was obtained from Compound 27 (0.0416 g, 0.0875 mmol) prepared in Example 27, triethylamine (0.0237 mL, 0.170 mmol) and acetyl chloride (0.00933 mL, 0.131 mmol).
APCI-MS m/z: 518 (M+H)⁺.

### Example 125 (Compound 126)

In a manner similar to that in Example 119, Compound 126 (0.0491 g, 84%) was obtained from Compound 119 (0.0511 g, 0.108 mmol) prepared in Example 118, triethylamine (0.0218 mL, 0.158 mmol) and n-butyryl chloride (0.0135 mL, 0.130 mmol).
APCI-MS m/z: 544 (M+H)⁺.

### Example 126 (Compound 127)

In a manner similar to that in Reference Example 4, Compound 127 (0.0541 g, 83%) was obtained from Compound g (0.0511 g, 0.127 mmol) prepared in Reference Example 8, acetic acid (0.0520 mL, 0.908 mmol), N-ethylaniline (0.0769 g, 0.635 mmol) and triacetoxy sodium borohydride (0.108 g, 0.508 mmol).
APCI-MS m/z: 509 (M+H)⁺.

### Example 127 (Compound 128)

In a manner similar to that in Example 111, Compound 128 (0.0634 g, 77%) was obtained from Compound 119 (0.0757 g, 0.160 mmol) prepared in Example 118, acetic acid (0.0650 mL, 1.14 mmol), propionaldehyde (0.0465 g, 0.800 mmol) and triacetoxy sodium borohydride (0.203 g, 0.960 mmol).
APCI-MS m/z: 516 (M+H)⁺.

### Example 128 (Compound 129)

In a manner similar to that in Example 119, Compound 129 (0.0504 g, 88%) was obtained from Compound 119 (0.0502 g, 0.106 mmol) prepared in Example 118, triethylamine (0.0212 mL, 0.154 mmol) and cyclopropanecarbonyl chloride (0.0115 mL, 0.127 mmol).
APCI-MS m/z: 542 (M+H)⁺.

### Example 129 (Compound 130)

In a manner similar to that in Example 111, Compound 130 (0.0942 g, 73%) was obtained from Compound 29 (0.122 g, 0.264 mmol) prepared in Example 29, acetic acid (0.099 mL, 1.74 mmol), acetaldehyde (0.0581 g, 1.32 mmol) and triacetoxy sodium borohydride (0.335 g, 1.58 mmol).
APCI-MS m/z: 491 (M+H)⁺.

### Example 130 (Compound 131)

Compound x (50.2 mg, 0.116 mmol) prepared in Reference Example 24 was dissolved in toluene (2.0 mL). To the solution was added diethylamine (0.024 mL, 0.232 mmol) and diphenylphosphoryl azide (0.025 mL, 0.116 mmol), and the mixture was stirred at 80°C for 4 hours. To the reaction mixture was added water and 1 mol/L hydrochloric acid, and the mixture was extracted with ethyl acetate. The organic layer was washed with brine, and dried over anhydrous sodium sulfate, and the solvent was evaporated under reduced pressure. The resulting residue was purified by preparative silica gel column chromatography (chloroform/acetonitrile = 9/1) to give Compound 131 (0.0235 g, 40%).
APCI-MS m/z: 503 (M-H)⁻ .

### Example 131 (Compound 132)

In a manner similar to that in Reference Example 4, Compound 132 (13 mg, 60%) was obtained from Compound aa (20 mg, 0.037 mmol) prepared in Reference Example 27, acetic acid (0.013 mL, 0.23 mmol), 2-aminoethanol (0.011 mL, 0.18 mmol) and triacetoxy sodium borohydride (34 mg, 0.16 mmol).
APCI-MS m/z: 579 (M+H)⁺.

### Example 132 (Compound 133)

Compound 133 (11 mg, 0.019 mmol) prepared in Example 131 was dissolved in THF (0.5 mL). To the solution was added tetrabutylammonium fluoride (1.0 mol/L solution in THF, 0.029 mL, 0.10 mmol), and the mixture was stirred at room temperature for 40 minutes. The reaction mixture was concentrated under reduced pressure, and the residue was purified by preparative thin layer chromatography (chloroform containing ammonia/methanol = 9/1) to give Compound 133 (10 mg, quantitative).
APCI-MS m/z: 465 (M+H)⁺.

### Example 133 (Compound 134)

In a manner similar to that in Reference Example 4, Compound 134 (5.4 mg, 20%) was obtained from Compound aa (25 mg, 0.047 mmol) prepared in Reference Example 27, acetic acid (0.027 mL, 0.47 mmol), ethylenediamine (0.016 mL, 0.24 mmol) and triacetoxy sodium borohydride (45 mg, 0.21 mmol).
APCI-MS m/z: 578 (M+H)⁺.

### Example 134 (Compound 135)

In a manner similar to that in Example 132, Compound 134 (4.4 mg, 0.0076 mmol) prepared in Example 133 was treated with tetrabutylammonium fluoride (1.0 mol/L solution in THF, 0.011 mL, 0.038 mmol) to give Compound 135 (3.5 mg, 99%).
APCI-MS m/z: 464 (M+H)⁺.

### Example 135 (Compound 136)

1-Ethyl-3-(3'-dimethylaminopropyl)carbodiimide (200 mg, 1.29 mmol) was dissolved in DMF (3 mL). To the solution was added 3-dimethylaminopropionic acid hydrochloride (100 mg, 0.646 mmol) and 1-hydroxybenzotriazole monohydrate (198 mg, 1.29 mmol) under ice cooling, and the mixture was stirred at the same temperature for 5 minutes. Then, to the reaction mixture was added Compound o (100 mg, 0.256 mmol) prepared in Reference Example 15, and the mixture was stirred at room temperature for 6.7 hours. To the reaction mixture was added saturated aqueous sodium hydrogencarbonate, and the mixture was extracted with ethyl acetate. The organic layer was washed with brine, and then dried over anhydrous sodium sulfate, and the solvent was evaporated under reduced pressure. The residue was purified by preparative thin layer chromatography (chloroform/methanol/ammonia = 20/0.5/0.5) to give Compound 136 (109 mg, 87%).
APCI-MS m/z: 490 (M+H)⁺.

### Example 136 (Compound 137)

Compound 64 (195 mg, 0.387 mmol) prepared in Example 63 was dissolved in tert-butyl alcohol (7.8 mL) and 1 mol/L hydrochloric acid-1 mol/L sodium acetate buffer (pH = 3, 2.4 mL). To the solution was added sodium borohydride (293 mg, 7.74 mmol), and the mixture was stirred at 50°C for 1.2 hours. Then, the reaction mixture was added sodium borohydride (146 mg, 3.87 mmol) every 1 hour twice, and then the mixture was stirred for 3 hours. To the reaction mixture was added water, and the mixture was extracted with ethyl acetate. The organic layer was washed with brine, and then dried over anhydrous sodium sulfate, and the solvent was evaporated under reduced pressure. The residue was purified by preparative thin layer chromatography (chloroform/methanol/ammonia = 40/4/1) to give Compound 137 (78 mg, 48%).
APCI-MS m/z: 420 (M+H)⁺.

### Example 137 (Compound 138)

In a manner similar to that in Example 88, Compound 138 (36 mg, 30%) was obtained from Compound 137 (103 mg, 0.245 mmol) prepared in Example 136, dichloromethane (3.1 mL), pyridine (0.050 mL, 0.61 mmol), 4-bromobutyryl chloride (0.071 mL, 0.061 mmol) and sodium acetate (50 mg, 0.61 mmol).
APCI-MS m/z: 488 (M+H)⁺.

### Example 138 (Compound 139)

Compound o (90.1 mg, 0.231 mmol) prepared in Reference Example 15 was dissolved in dichloromethane (3.6 mL). To the solution was added pyridine (0.064 mL, 0.81 mmol) and 4-bromobutyryl chloride (0.067 mL, 0.058 mmol), and the mixture was stirred at room temperature for 0.7 hour. To the reaction mixture was added water, and the mixture was extracted with chloroform. The organic layer was washed with brine, and then dried over anhydrous sodium sulfate, and the solvent was evaporated under reduced pressure. Then, the residue was dissolved in DMF (6.8 mL). To the solution was added morpholine (0.403 mL, 4.62 mmol) and potassium carbonate (160 mg, 1.16 mmol), and the mixture was stirred at 100°C for 1.3 hours. To the reaction mixture was added water, and the mixture was extracted with ethyl acetate. The organic layer was washed with brine, and then dried over anhydrous sodium sulfate, and the solvent was evaporated under reduced pressure. The residue was purified by preparative thin layer chromatography (chloroform/methanol = 12/1) to give Compound 139 (31 mg, 25%).
APCI-MS m/z: 546 (M+H)⁺.

### Example 130 (Compound 140)

In a manner similar to that in Example 138, Compound 140 (51 mg, 33%) was obtained from Compound o (108 mg, 0.277 mmol) prepared in Reference Example 15, pyridine (0.077 mL, 0.97 mmol), 4-bromobutyryl chloride (0.080 mL, 0.069 mmol), N-methylpiperazine (0.615 mL, 5.54 mmol) and potassium carbonate (191 mg, 1.39 mmol).
APCI-MS m/z: 559 (M+H)⁺.

### Example 140 (Compound 141)

### Step 1

4-Methylaminobutyric acid (1.00 g, 6.51 mmol) was dissolved in 1,4-dioxane (30 mL). To the solution was added di-tert-butyl dicarbonate (1.42 g, 6.51 mmol) and 0.5 mol/L aqueous potassium hydroxide (130 mL), and the mixture was stirred at room temperature for 72 hours. The reaction mixture was extracted with ethyl acetate, and the organic layer was washed with 15% aqueous citric acid and brine, and then dried over anhydrous sodium sulfate. The solvent was evaporated under reduced pressure, and the residue was purified by silica gel column chromatography (chloroform/methanol = 30/1→7/1) to give 4-(N-tert-butoxycarbonyl-N-methylamino)butyric acid (689 mg, 49%).
APCI-MS m/z: 216 (M-H)⁻.

### Step 2

In a manner similar to that in Example 135, Compound 141 (137 mg, 58%) was obtained from trifluoroacetate of Compound o (200 mg, 0.396 mmol) prepared in Reference Example 15, 1-ethyl-3-(3'-dimethylaminopropyl)carbodiimide (185 mg, 1.19 mmol), N-hydroxybenzotriazole monohydrate (243 mg, 1.58 mmol) and 4-(N-tert-butoxycarbonyl-N-methylamino)butyric acid (258 mg, 1.19 mmol) obtained above.
APCI-MS m/z: 590 (M+H)⁺.

### Example 141 (Compound 142)

Compound 141 (84.0 mg, 0.142 mmol) prepared in Example 140 was dissolved in dichloromethane (3.5 mL). To the solution was added trifluoroacetic acid (0.109 mL, 1.42 mmol), and the mixture was stirred at room temperature for 72 hours. To the reaction mixture was added saturated aqueous sodium hydrogencarbonate, and the mixture was extracted with ethyl acetate. The organic layer was washed with brine, and then dried over anhydrous sodium sulfate, and the solvent was evaporated under reduced pressure. The residue was purified by preparative thin layer chromatography (chloroform/methanol/ammonia = 20/0.5/0.5→2/1/1) to give Compound 142 (34 mg, 48%).
APCI-MS m/z: 490 (M+H)⁺.

### Example 142 (Compound 143)

In a manner similar to that in Example 37, Compound cc (100 mg, 0.231 mmol) prepared in Reference Example 29 was treated with trifluoroacetic acid (0.356 mL, 4.62 mmol), then followed by reacting with 1-ethyl-3-(3'-dimethylaminopropyl)carbodiimide (108 mg, 0.693 mmol), N-hydroxybenzotriazole monohydrate (142 mg, 0.924 mmol) and 4-dimethylaminobutyric acid hydrochloride (116 mg, 0.693 mmol) in a manner similar to that in Example 135 to give Compound 143 (58 mg, 57%).
APCI-MS m/z: 446 (M+H)⁺.

### Example 143 (Compound 144)

In a manner similar to that in Example 37, Compound dd (120 mg, 0.269 mmol) prepared in Reference Example 30 was treated with trifluoroacetic acid (0.414 mL, 5.38 mmol), then followed by reacting with 1-ethyl-3-(3'-dimethylaminopropyl)carbodiimide (126 mg, 0.807 mmol), N-hydroxybenzotriazole monohydrate (165 mg, 1.08 mmol) and 4-dimethylaminobutyric acid hydrochloride (135 mg, 0.807 mmol) in a manner similar to that in Example 135 to give Compound 144 (80 mg, 65%).
APCI-MS m/z: 460 (M+H)⁺.

### Example 144 (Compound 145)

### Step 1

4 A Molecular sieves (642 mg) were suspended in DMF (8 mL). To the suspension was added cesium hydroxide monohydrate (646 mg, 3.84 mmol), cyclopropylamine (0.890 mL, 12.8 mmol) and ethyl 4-bromobutyrate (0.367 mL, 2.56 mmol), and the mixture was stirred at room temperature for 18.7 hours. The reaction mixture was filtered, then to the filtrate was added saturated aqueous sodium hydrogencarbonate, and the mixture was extracted with ethyl acetate. The organic layer was washed with brine, and then dried over anhydrous sodium sulfate. The solvent was evaporated under reduced pressure to give ethyl N-cyclopropyl-4-aminobutyrate (147 mg, 34%).
APCI-MS m/z: 172 (M+H)⁺.

### Step 2

Ethyl N-cyclopropyl-4-aminobutyrate (133 mg, 0.777 mmol) obtained above was dissolved in 1,2-dichloroethane (8 mL). To the solution was added acetic acid (0.289 mL), acetaldehyde (0.218 mL, 3.89 mmol) and triacetoxy sodium borohydride (988 mg, 4.66 mmol), and the mixture was stirred at room temperature for 3 hours.

To the reaction mixture was added saturated aqueous sodium hydrogencarbonate, and the mixture was extracted with ethyl acetate. The organic layer was washed with brine, and then dried over anhydrous sodium sulfate, and the solvent was evaporated under reduced pressure. The residue was purified by preparative thin layer chromatography (chloroform/methanol = 25/1) to give ethyl N-cyclopropyl-N-ethyl-4-aminobutyrate (105 mg, 68%).
APCI-MS m/z: 200 (M+H)⁺.

### Step 3

Ethyl N-cyclopropyl-N-ethyl-4-aminobutyrate (105 mg, 0.527 mmol) obtained above was dissolved in ethanol (5.3 mL). To the solution was added 4 mol/L aqueous potassium hydroxide (0.395 mL, 1.58 mmol), and the mixture was stirred at 50°C for 40 minutes. To the reaction mixture was added 4 mol/L hydrogen chloride - ethyl acetate (0.791 mL, 3.16 mmol), then the deposited solid was filtrated off, and the filtrate was concentrated under reduced pressure to give N-cyclopropyl-N-ethyl-4-aminobutyric acid hydrochloride (102 mg, 93%).
APCI-MS m/z: 172 (M+H)⁺.

### Step 4

In a manner similar to that in Example 135, trifluoroacetate of Compound o (121 mg, 0.234 mmol) prepared in Reference Example 15 was allowed to react with 1-ethyl-3-(3'-dimethylaminopropyl)carbodiimide (73 mg, 0.47 mmol), N-hydroxybenzotriazole monohydrate (108 mg, 0.702 mmol) and N-cyclopropyl-N-ethyl-4-aminobutyric acid hydrochloride (97.1 mg, 0.234 mmol) obtained above, and followed by being purified by preparative thin layer chromatography (chloroform/methanol/ammonia = 15/0.5/0.5) to give Compound 145 (47 mg, 37%).

APCI-MS m/z: 544 (M+H)⁺.

### Example 145 (Compound 146)

Hydrochloride of Compound m (500 mg, 1.21 mmol) prepared in Reference Example 31 was dissolved in dichloromethane (5 mL). To the solution was added 4-nitrophenyl chloroformate (293 mg, 1.45 mmol) dissolved in pyridine (0.431 mL, 5.33 mmol) and dichloromethane (5 mL) under ice cooling, and the mixture was stirred at room temperature for 1.5 hours. To the reaction mixture was added water, and the mixture was extracted with chloroform. The organic layer was washed with 1 mol/L hydrochloric acid, saturated aqueous sodium hydrogencarbonate and brine, then dried over anhydrous sodium sulfate, and concentrated under reduced pressure. The residue was purified by silica gel column chromatography (hexane/ethyl acetate = 9/1 →4/1→7/3) to give Compound 146 (460 mg, 70%).
APCI-MS m/z: 542 (M+H)⁺.

### Example 146 (Compound 147)

Compound 146 (74 mg, 0.14 mmol) prepared in Example 145 was dissolved in dichloromethane (1.5 mL). To the solution was added a 70% aqueous ethylamine (0.022 mL), and the mixture was stirred at room temperature for 1.5 hours. The reaction mixture was concentrated under reduced pressure, and the residue was purified by preparative thin layer chromatography (chloroform/methanol = 20/1), and then recrystallized from ethanol and water to give Compound 147 (22 mg, 36%).
APCI-MS m/z: 448 (M+H)⁺.

### Example 147 (Compound 148)

In a manner similar to that in Example 146, Compound 148 (48 mg, 78%) was obtained from Compound 146 (72 mg, 0.13 mmol) prepared in Example 145 and ethylenediamine (0.053 mL, 0.80 mmol).
APCI-MS m/z: 463 (M+H)⁺.

### Example 148 (Compound 149)

In a manner similar to that in Example 146, Compound 149 (54 mg, 84%) was obtained from Compound 146 (71 mg, 0.13 mmol) prepared in Example 145 and N,N-dimethylethylenediamine (0.029 mL, 0.26 mmol).
APCI-MS m/z: 491 (M+H)⁺.

### Example 149 (Compound 150)

In a manner similar to that in Example 146, Compound 150 (57 mg, 94%) was obtained from Compound 146 (71 mg, 0.13 mmol) prepared in Example 145 and 2-aminoethanol (0.016 mL, 0.27 mmol).
APCI-MS m/z: 464 (M+H)⁺.

### Example 150 (Compound 151)

In a manner similar to that in Example 146, Compound 151 (59 mg, 88%) was obtained from Compound 146 (72 mg, 0.13 mmol) prepared in Example 145 and 1-methylpiperazine (0.030 mL, 0.27 mmol).
APCI-MS m/z: 503 (M+H)⁺.

### Example 151 (Compound 152)

In a manner similar to that in Example 146, Compound 152 (43 mg, 67%) was obtained from Compound 146 (73 mg, 0.14 mmol) prepared in Example 145 and 1,3-propanediamine (0.067 mL, 0.80 mmol).
APCI-MS m/z: 477 (M+H)⁺.

### Example 152 (Compound 153)

In a manner similar to that in Example 146, Compound 153 (58 mg, 89%) was obtained from Compound 146 (70 mg, 0.13 mmol) prepared in Example 145 and N,N-dimethyl-1,3-propanediamine (0.032 mL, 0.25 mmol).
APCI-MS m/z: 505 (M+H)⁺.

### Example 153 (Compound 154)

Compound ff (520 mg, 1.01 mmol) prepared in Reference Example 32 was dissolved in DMF (20 mL). To the solution was added sodium iodide (3.03 g, 20.2 mmol), and the mixture was stirred at 100°C for 7 hours. To the reaction mixture was added water, and the mixture was extracted with ethyl acetate. The organic layer was washed with brine, and then dried over anhydrous sodium sulfate, and concentrated under reduced pressure. To the residue was added acetonitrile (15 mL) and 70% aqueous ethylamine (3.0 mL), and the mixture was stirred at room temperature for 2.5 hours. To the reaction mixture was further added sodium iodide (3.04 g, 20.3 mmol), and the mixture was stirred for 16.5 hours. Then, to the mixture was added water, and the mixture was extracted with ethyl acetate. The organic layer was washed with 1 mol/L hydrochloric acid and brine, then dried over anhydrous sodium sulfate, and concentrated under reduced pressure. The residue was purified by silica gel column chromatography (chloroform/methanol = 40/1), and then triturated with ethyl acetate to give Compound 154 (74 mg, 15%).
¹H NMR (300 MHz, CDCl₃) δ (ppm): 1.28 (s, 9H), 1.32 (s, 9H), 2.28-2.41 (m, 2H), 2.96 (dt,J = 8.5, 12.3 Hz, 1H), 3.16 (m, 1H), 3.38 (dt, J = 7.3, 14.8 Hz, 1H), 3.53 (m, 1H), 4.21 (d, J = 15.0 Hz, 1H), 4.56 (d, J = 15.0 Hz, 1H), 7.21-7.38 (m, 5H), 7.86 (s, 1H).

### Example 154 (Compound 155)

In a manner similar to that in Example 135, Compound 155 (76 mg, 65%) was obtained from hydrochloride of Compound m (100 mg, 0.240 mmol) prepared in Reference Example 31, 1-ethyl-3-(3'-dimethylaminopropyl)carbodiimide (112 mg, 0.720 mmol), 1-hydroxybenzotriazole monohydrate (147 mg, 0.960 mmol) and 4-dimethylaminobutyric acid hydrochloride (121 mg, 0.720 mmol).
APCI-MS m/z: 490 (M+H)⁺.

### Example 155 (Compound 156)

Compound jj (479 mg, 1.35 mmol) prepared in Reference Example 36 was dissolved in DMF (14 mL). To the solution was added N-tert-butoxybutoxycarbonylglycine (686 mg, 3.92 mmol), N-hydroxybenzotriazole (930 mg, 6.07 mmol) and 1-ethyl-3-(3'-dimethylaminopropyl)carbodiimide (566 mg, 3.64 mmol), and the mixture was stirred at room temperature for 12 hours. To the reaction mixture was added water, and the mixture was extracted with ethyl acetate. The organic layer was washed with brine, and then dried over anhydrous sodium sulfate, and the solvent was evaporated under reduced pressure. The residue was purified by silica gel column chromatography (chloroform/ethyl acetate = 3/7→2/8) to give Compound 156 (160 mg, 25%).
APCI-MS m/z: 476 (M+H)⁺.

### Example 156 (Compound 157)

In a manner similar to that in Step 5 of Example 77, Compound 157 (40.7 mg, 26%) was obtained from Compound 156 (160 mg, 0.336 mmol) prepared in Example 155 and 4 mol/L hydrogen chloride-ethyl acetate (3 mL).
APCI-MS m/z: 376 (M+H)⁺.

### Example 157 (Compound 158)

In a manner similar to that in Example 135, Compound 158 (222 mg, 33%) was obtained from Compound mm (518 mg, 1.40 mmol) prepared in Reference Example 37, N-tert-butoxybutoxycarbonylglycine (711 mg, 4.06 mmol), N-hydroxybenzotriazole (968 mg, 6.32 mmol) and 1-ethyl-3-(3'-dimethylaminopropyl)carbodiimide (589 mg, 3.79 mmol).
APCI-MS m/z: 490 (M+H)⁺.

### Example 158 (Compound 159)

In a manner similar to that in Step 5 of Example 77, Compound 161 (37.8 mg, 20%) was obtained from Compound 158 (222 mg, 0.453 mmol) prepared in Example 157 and 4 mol/L hydrogen chloride-ethyl acetate (3 mL).
APCI-MS m/z: 390 (M+H)⁺.

### Example 159 (Compound 160)

In a manner similar to that in Reference Example 4, Compound 160 (0.0222 g, 32%) was obtained from Compound qq (0.0625 g, 0.145mmol) prepared in Reference Example 42, acetic acid (0.060 mL, 1.05 mmol), cyclopropylamine (0.0414 g, 0.725 mmol) and triacetoxy sodium borohydride (0.123 g, 0.580 mmol).
APCI-MS m/z: 473 (M+H)⁺.

### Example 160 (Compound 161)

In a manner similar to that in Reference Example 4, Compound 161 (0.0222 g, 75%) was obtained from Compound qq (0.0448 g, 0.104 mmol) prepared in Reference Example 42, acetic acid (0.040 mL, 0.70 mmol), morpholine (0.0536 g, 0.535 mmol) and triacetoxy sodium borohydride (0.0880 g, 0.416 mmol).
APCI-MS m/z: 503 (M+H)⁺.

### Example 161 (Compound 162)

In a manner similar to that in Reference Example 4, Compound 162 (0.0502 g, 91%) was obtained from Compound qq (0.0463 g, 0.107 mmol) prepared in Reference Example 42, acetic acid (0.040 mL, 0.70 mmol), 4-methylpiperazine (0.0536 g, 0.535 mmol) and triacetoxy sodium borohydride (0.0852 g, 0.403 mmol).
APCI-MS m/z: 516 (M+H)⁺.

### Example 162 (Compound 163)

In a manner similar to that in Example 111, Compound 163 (0.00510 g, 32%) was obtained from Compound 160 (0.0151 g, 0.0319 mmol) prepared in Example 159, acetic acid (0.013 mL, 0.228 mmol), acetaldehyde (0.00705 g, 0.160 mmol) and triacetoxy sodium borohydride (0.0405 g, 0.191 mmol).
APCI-MS m/z: 501 (M+H)⁺.

### Example 163 (Compound 164)

### Step 1

In a manner similar to that in Step 1 of Reference Example 14, 4-benzoyl-1-(tert-butoxycarbonyl)piperidine (0.923 mg, 99%) was obtained from 4-benzoylpiperidine hydrochloride (0.721 g, 3.19 mmol), di-tert-butyl dicarbonate (1.66 g, 7.61 mmol) and dimethylaminopyridine (0.280 g, 2.29 mmol).

### Step 2

In a manner similar to that in Step 1 of Reference Example 1, phenyl (N-tert-butoxycarbonyl-4-piperidyl)methanone=thiosemicarbazone (0.326 g, 24%) was obtained from 4-benzoyl-1-(tert-butoxycarbonyl)piperidine (1.09 g, 3.77 mmol) prepared above and thiosemicarbazide hydrochloride (1.44 g, 11.3 mmol).

### Step 3

In a manner similar to that in Step 2 of Reference Example 1, Compound 164 (0.148 g, 84%) was obtained from phenyl (N-tert-butoxycarbonyl-4-piperidyl)methanone=thiosemicarbazone (0.120 g, 0.331 mmol) prepared above, pyridine (0.128 mL, 1.58 mmol) and trimethylacetyl chloride (0.147 mL, 1.32 mmol). APCI-MS m/z: 531 (M+H)⁺.

### Example 164 (Compound 165)

In a manner similar to that in Example 37, Compound 165 (0.0815 g, 99%) was obtained from Compound 164 (0.100 g, 0.188 mmol) prepared in Example 163 and trifluoroacetic acid (0.300 mL, 0.317 mmol).
APCI-MS m/z: 431 (M+H)⁺.

### Example 165 (Compound 166)

In a manner similar to that in Example 111, Compound 166 (0.0220 g, 67%) was obtained from Compound 165 (0.0307 g, 0.0713 mmol) prepared in Example 164, triacetoxy sodium borohydride (0.0907 g, 0.428 mmol), acetic acid (0.025 mL, 0.437 mmol) and acetaldehyde (0.020 mL, 0.357 mmol).
APCI-MS m/z: 459 (M+H)⁺.

### Example 166 (Compound 167)

Phenyl (N-tert-butoxycarbonyl-4-piperidyl)methanone=thiosemicarbazone (0.204 g, 0.563 mmol) prepared in Step 2 of Example 163 was dissolved in acetic anhydride (2.0 mL, 21.2 mmol), and the mixture was stirred at 80°C for 2 hours. The reaction mixture was concentrated under reduced pressure. To the residue was added diisopropyl ether, and the mixture was stirred. The deposited white crystals were collected by filtration, and dissolved in chloroform. Then, to the solution was added water and saturated aqueous sodium hydrogencarbonate, and the mixture was vigorously stirred. The mixture was extracted with chloroform, and then the organic layer was dried over anhydrous sodium sulfate. The solvent was evaporated under reduced pressure to give Compound 167 (0.214 g, 85%).
APCI-MS m/z: 447 (M+H)⁺.

### Example 167 (Compound 168)

Compound 167 (0.210 g, 0.470 mmol) prepared in Example 166 was dissolved in methanol (5 mL). To the solution was added ceric chloride heptahydrate (0.175 g, 0.470 mmol) and sodium borohydride (0.178 g, 0.470 mmol), and the mixture was stirred at room temperature for 30 minutes. To the reaction mixture was added water, and the mixture was extracted with chloroform. The organic layer was dried over anhydrous sodium sulfate, and the solvent was evaporated under reduced pressure. The residue was purified by silica gel column chromatography (chloroform/methanol = 9/1) to give Compound 168 (0.136 g, 71%).
APCI-MS m/z: 405 (M+H)⁺.

### Example 168 (Compound 169)

In a manner similar to that in Example 86, Compound 169 (0.139 g, 86%) was obtained from Compound 168 (0.136 g, 0.332 mmol) prepared in Example 167, pyridine (0.0652 mL, 0.806 mmol) and trimethylacetyl chloride (0.749 mL, 0.672 mmol).
APCI-MS m/z: 489 (M+H)⁺.

### Example 169 (Compound 170)

In a manner similar to that in Example 37, Compound 170 (0.0997 g, 90%) was obtained Compound 169 (0.139 g, 0.284 mmol) prepared in Example 168 and trifluoroacetic acid (0.900 mL, 0.951 mmol).
APCI-MS m/z: 389 (M+H)⁺.

### Example 170 (Compound 171)

In a manner similar to that in Example 111, Compound 171 (0.0211 g, 63%) was obtained from Compound 170 (0.0313 g, 0.0806 mmol) prepared in Example 169, triacetoxy sodium borohydride (0.103 g, 0.484 mmol), acetic acid (0.025 mL, 0.437 mmol) and acetaldehyde (0.0224 mL, 0.403 mmol).
APCI-MS m/z: 417 (M+H)⁺.

### Example 171 (Compound 172)

Compound rr (0.119 g, 0.213 mmol) prepared in Reference Example 43 was dissolved in tert-butyl alcohol (2.0 mL). To the solution was added N-(tert-butoxycarbonyl)ethanolamine (0.329 mL, 2.13 mmol) and potassium tert-butoxide (0.263 g, 2.34 mmol), and the mixture was stirred at room temperature for 18 hours. To the reaction mixture was added water and 1.0 mol/L hydrochloric acid, and the mixture was extracted with ethyl acetate. The organic layer was washed with water, and then dried over anhydrous sodium sulfate, and the solvent was evaporated under reduced pressure. The residue was purified by preparative thin layer chromatography (chloroform/methanol = 20/1) to give Compound 172 (0.068 g, 58%).
APCI-MS m/z: 549 (M+H)⁺.

### Example 172 (Compound 173)

In a manner similar to that in Example 37, Compound 175 (0.0186 g, 20%) was obtained from Compound 172 (0.116 g, 0.211 mmol) prepared in Example 171 and trifluoroacetic acid (0.300 mL, 0.317 mmol).
APCI-MS m/z: 449 (M+H)⁺.

### Example 173 (Compound 174)

Compound rr (0.103 g, 0.184 mmol) prepared in Reference Example 43 was dissolved in tert-butyl alcohol (5.0 mL). To the solution was added 2-mercaptoethylamine hydrochloride (0.103 mg, 0.184 mmol) and potassium tert-butoxide (0.206 g, 1.84 mmol), and the mixture was stirred at room temperature for 15 hours. To the reaction mixture was added water and 1.0 mol/L hydrochloric acid, and the mixture was extracted with ethyl acetate. The organic layer was washed with water, and then dried over anhydrous sodium sulfate, and the solvent was evaporated under reduced pressure. The residue was purified by preparative thin layer chromatography (chloroform/methanol/ammonia = 9/1/1) to give Compound 174 (0.0574 g, 67%).
APCI-MS m/z: 465 (M+H)⁺.

### Example 174 (Compound 175)

In a manner similar to that in Example 171, Compound ss (0.218 g, 0.380 mmol) prepared in Reference Example 44 was allowed to react with N-(tert-butoxycarbonyl)ethanolamine (0.306 mL, 1.90 mmol) and potassium tert-butoxide (0.426 g, 3.80 mmol), and followed by treating with trifluoroacetic acid (0.500 mL, 0.528 mmol) in a manner similar to that in Example 37 to give Compound 175 (0.0346 g, 46%).
APCI-MS m/z: 463 (M+H)⁺.

### Example 175 (Compound 176)

In a manner similar to that in Example 173, Compound 176 (0.0626 g, 62%) was obtained from Compound ss (0.122 g, 0.213 mmol) prepared in Reference Example 44, 2-mercaptoethylamine hydrochloride (0.122 mg, 0.107 mmol) and potassium tert-butoxide (0.239 g, 2.13 mmol).
APCI-MS m/z: 479 (M+H)⁺.

### Example 176 (Compound 177)

### Step 1

2-Hydroxyacetophenone (1.03 g, 7.60 mmol) was dissolved in DMF (50 mL). To the solution was added acetic anhydride (1.20 mL, 12.7 mmol) and N,N-dimethylaminopyridine (1.03 g, 8.41 mmol), and the mixture was stirred at room temperature for 4 hours. To the reaction mixture was added water, and the mixture was extracted with ethyl acetate. The organic layer was washed with saturated aqueous sodium hydrogencarbonate and brine, and then dried over anhydrous sodium sulfate, and the solvent was evaporated under reduced pressure. The residue was purified by silica gel column chromatography (hexane/ethyl acetate = 20/1→10/1) to give 2-acetoxyacetophenone (0.941 g, 69%).
¹H-NMR (300 MHz, CDCl₃): 2.24 (s, 3H), 5.35 (s, 2H), 7.45-7.54 (m, 2H), 7.57-7.65 (m, 1H), 7.88-7.95 (m, 2H).

### Step 2

2-Acetoxyacetophenone (0.637 g, 3.57 mmol) prepared above was dissolved in methanol (15 mL). To the solution was added thiosemicarbazide hydrochloride (508 mg, 3.98 mmol), and the mixture was stirred at room temperature for 2 hours. The reaction mixture was concentrated under reduced pressure, and then the residue was suspended in dichloromethane (15 mL). To the suspension was added pyridine (1.00 mL, 12.4 mmol) and trimethylacetyl chloride (1.40 mL, 11.4 mmol), and the mixture was stirred at room temperature for 12 hours. To the reaction mixture was added saturated aqueous sodium hydrogencarbonate, and the mixture was stirred at room temperature for 1 hour. Then, the mixture was extracted with ethyl acetate. The organic layer was washed with saturated aqueous sodium hydrogencarbonate and brine, and then dried over anhydrous sodium sulfate, and the solvent was evaporated under reduced pressure. The residue was purified by silica gel column chromatography (hexane/ethyl acetate = 9/1→4/1→2/1) to give Compound 177 (0.592 g, 39%).
APCI-MS m/z: 420 (M+H)⁺.

### Example 177 (Compound 178)

Compound w (0.304 g, 0.806 mmol) prepared in Reference Example 23 was dissolved in dichloromethane (15 mL). To the solution was added N,N'-carbonyldiimidazole (0.539 g, 3.32 mmol), and the mixture was stirred at room temperature for 2 hours. To the reaction mixture was added saturated aqueous sodium hydrogencarbonate, and the mixture was extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 4/1→2/1→1/1) to give Compound 178 (0.360 g, 95%).
¹H-NMR (300 MHz, CDCl₃): 1.27 (s, 9H), 1.31 (s, 9H), 5.28 (d, J = 11.0 Hz, 1H), 5.63 (d, J = 11.0 Hz, 1H), 7.06-7.09 (m, 1H), 7.25-7.45 (m, 6H), 8.03 (br s, 1H), 8.10 (br s, 1H).

### Example 178 (Compound 179)

Compound 178 (30.8 mg, 0.0653 mmol) prepared in Example 177 was dissolved in dichloromethane (2 mL). To the solution was added 2-aminoethanol (0.0784 mL, 1.31 mmol), and the mixture was stirred at room temperature for 3 hours. To the reaction mixture was added saturated aqueous ammonium chloride, and the mixture was extracted with ethyl acetate. The organic layer was washed with brine, and then dried over anhydrous sodium sulfate, and the solvent was evaporated under reduced pressure. The residue was purified by preparative thin layer chromatography (ethyl acetate) to give Compound 179 (27.1 mg, 89%).
ESI-MS m/z: 465 (M+H)⁺.

### Example 179 (Compound 180)

In a manner similar to that in Example 178, Compound 180 (23.6 mg, 74%) was obtained from Compound 178 (30.8 mg, 0.0653 mmol) prepared in Example 177 and N-ethylethylenediamine (0.138 mL, 1.31 mmol).
ESI-MS m/z: 492 (M+H)⁺.

### Example 180 (Compound 181)

In a manner similar to that in Example 178, Compound 181 (26.5 mg, 78%) was obtained from Compound 178 (30.8 mg, 0.0653 mmol) prepared in Example 177 and 1-(2-aminoethyl)pyrrolidine (0.166 mL, 1.31 mmol).
ESI-MS m/z: 518 (M+H)⁺.

### Example 181 (Compound 182)

Compound g (0.189 g, 0.469 mmol) prepared in Reference Example 8 was dissolved in methanol (6 mL). To the solution was added O-methylhydroxyamine hydrochloride (51.7 mg, 0.619 mmol), and the mixture was stirred at room temperature for 1.5 hours. To the reaction mixture was added saturated aqueous sodium hydrogencarbonate, and the mixture was extracted with chloroform. The organic layer was dried over anhydrous sodium sulfate, and the solvent was evaporated under reduced pressure. The residue was purified by preparative thin layer chromatography (chloroform/methanol = 60/1) to give Compound 182 (0.174 g, 86%).
APCI-MS m/z: 433 (M+H)⁺.

### Example 182 (Compound 183)

In a manner similar to that in Example 181, Compound 183 (0.146 g, 73%) was obtained from Compound g (0.191 g, 0.474 mmol) prepared in Reference Example 8 and hydroxyammonium chloride (42.5 mg, 0.612 mmol).
APCI-MS m/z: 419 (M+H)⁺.

The structures of Compounds a to ss prepared in Reference Examples 1 to 44 are shown below in Tables 9 to 12.

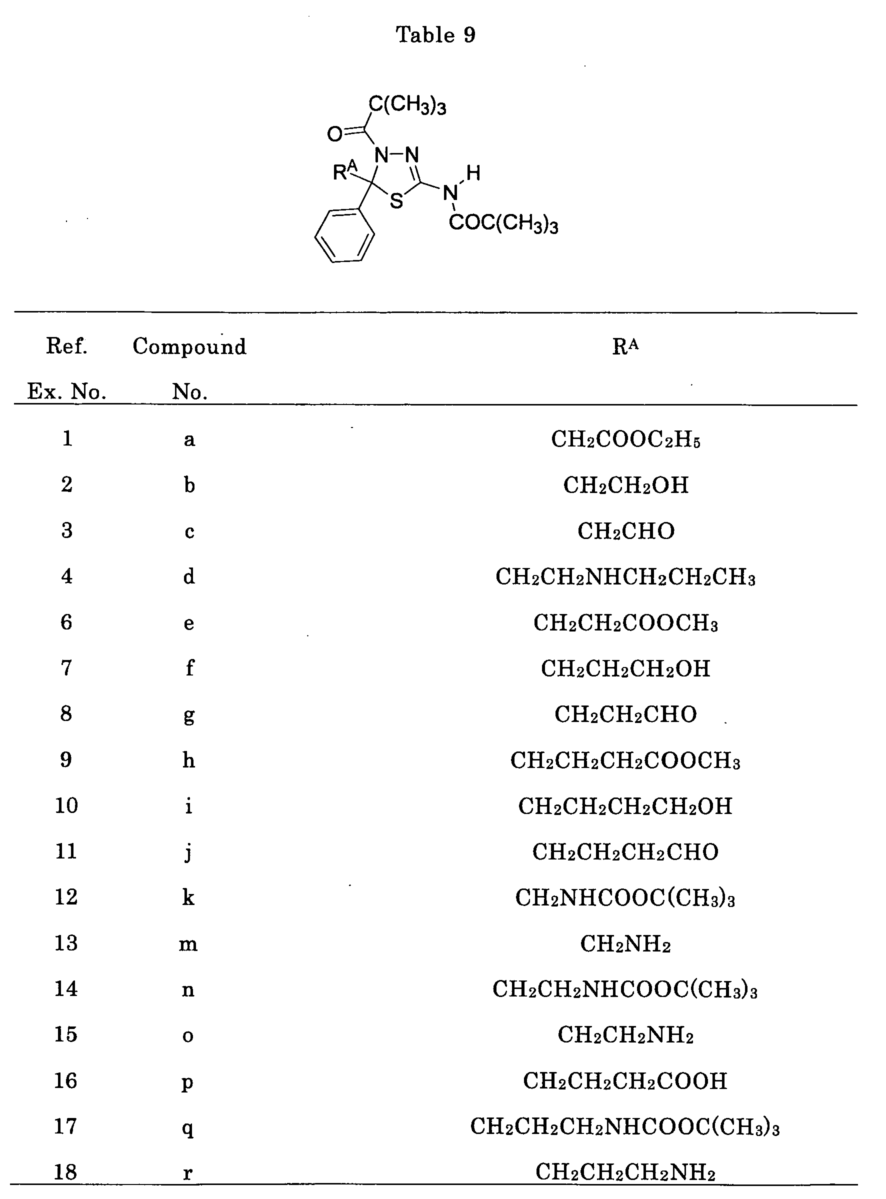

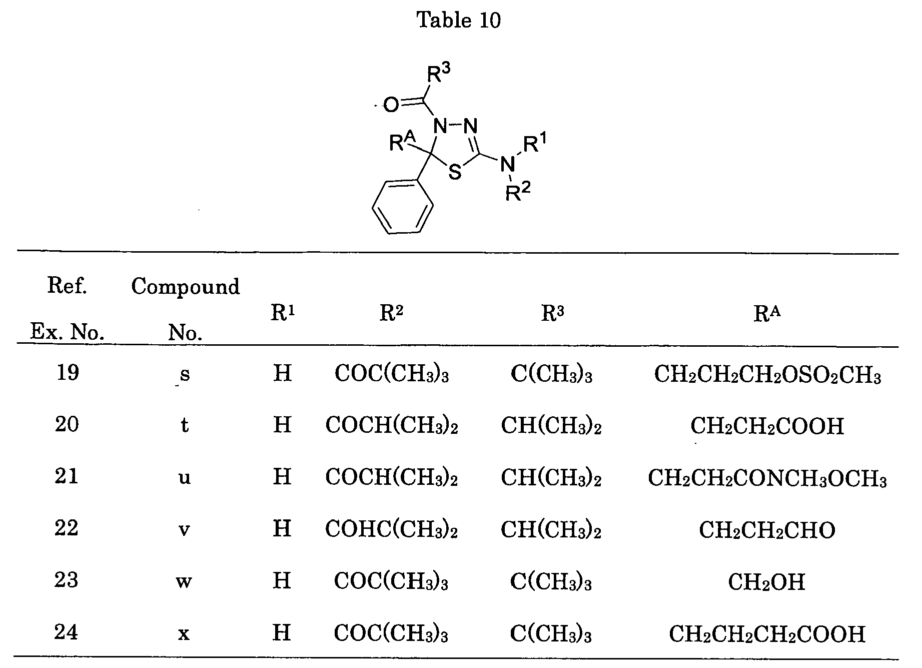

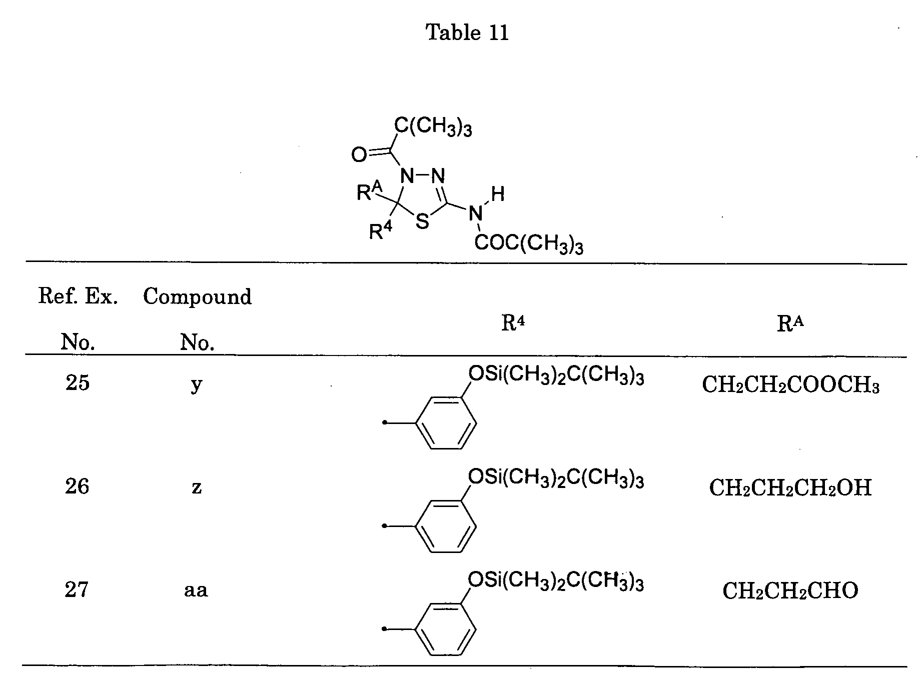

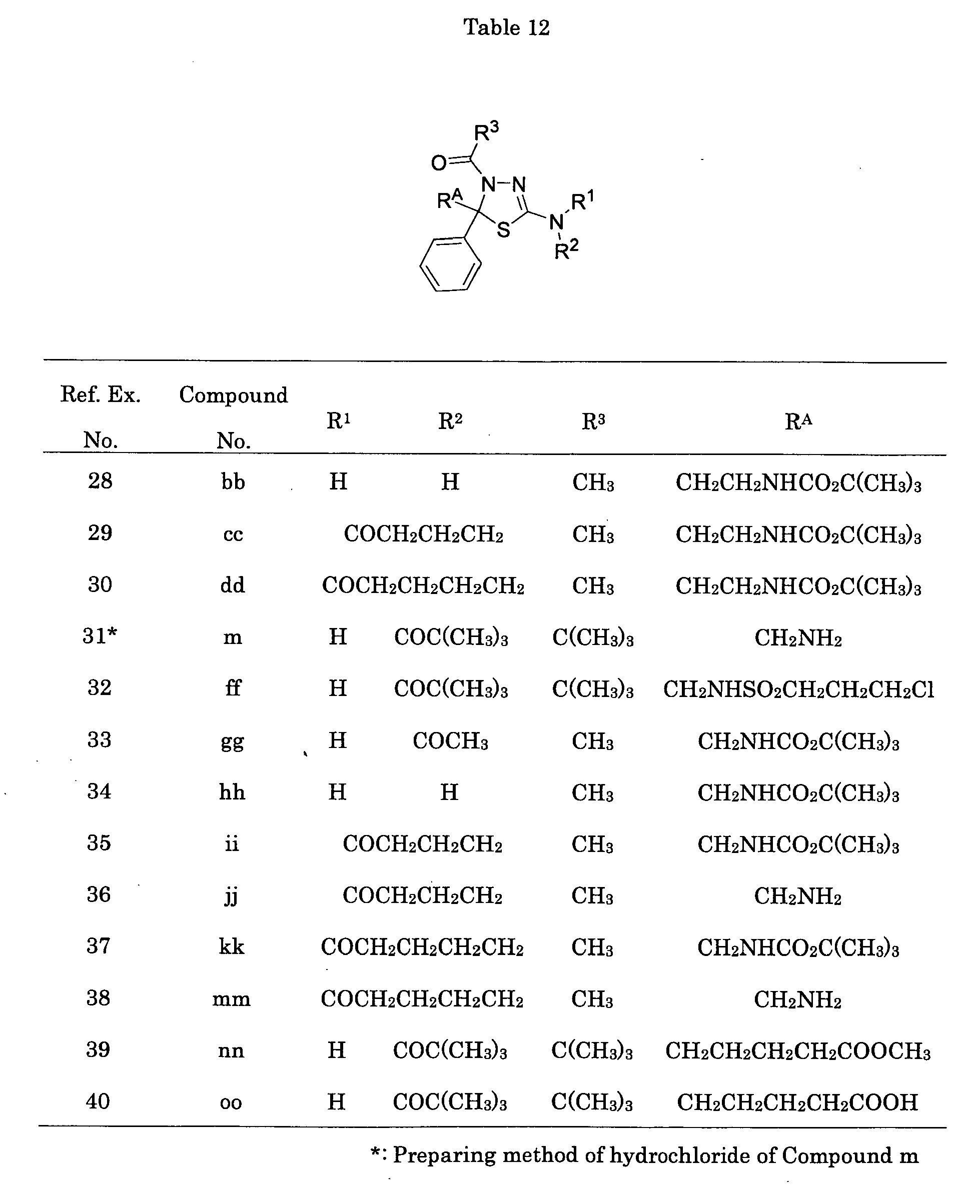

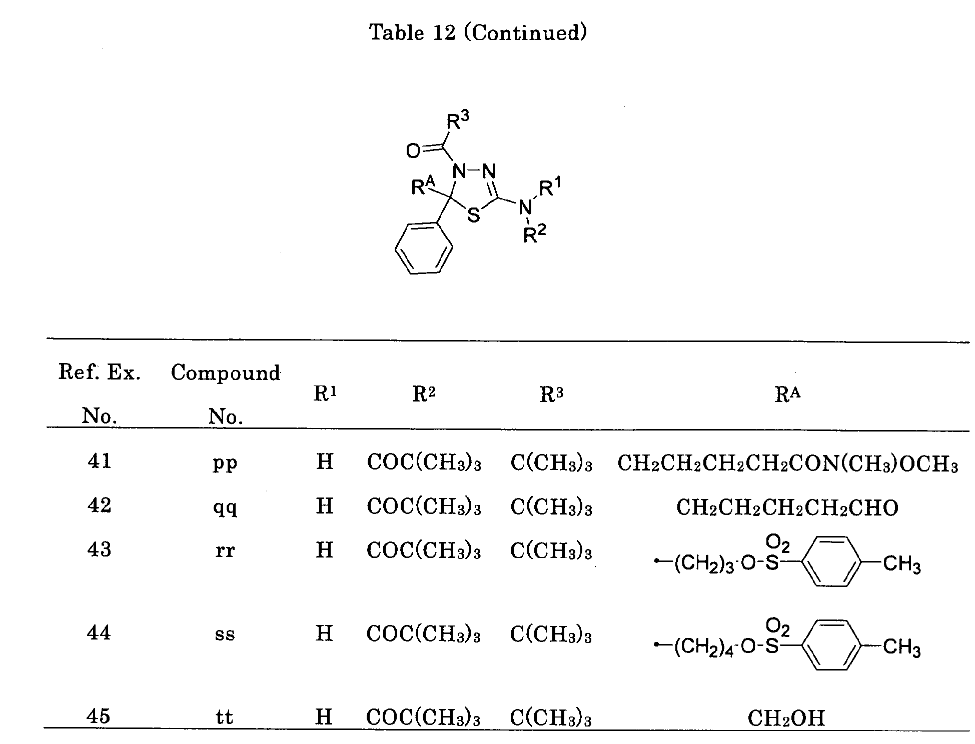

### Reference Example 1 (Compound a)

### Step 1

Thiosemicarbazide hydrochloride (8.30 g, 65.1 mmol) was dissolved in a mixed solvent of methanol (50 mL) and distilled water (50 mL). To the solution was added ethyl benzoylacetate (17.0 mL, 98.2 mmol) and concentrated hydrochloric acid (1.00 mL, 12.0 mmol), and the mixture was stirred at room temperature for 11 hours. The deposited solid was collected by filtration, washed with methanol and then dried to give 3-phenyl-3-thiosemicarbazonopropionic acid ethyl ester (11.1 g, 64%).

### Step 2

3-Phenyl-3-thiosemicarbazonopropionic acid ethyl ester (2.03 g, 7.65 mmol) obtained above was dissolved in dichloromethane (40 mL). To the solution was added pyridine (4.00 mL, 49.7 mmol) and trimethylacetyl chloride (5.60 mL, 45.5 mmol), and the mixture was stirred at room temperature for 12 hours. To the reaction mixture was added saturated aqueous sodium hydrogencarbonate, and the mixture was further stirred at room temperature for 1 hour and then extracted with ethyl acetate. The organic layer was washed with brine and dried over anhydrous sodium sulfate, and the solvent was evaporated under reduced pressure. The residue was purified by silica gel column chromatography (hexane/ethyl acetate = 20/1→9/1) to give Compound a (3.25 g, 98%).

### Reference Example 2 (Compound b)

Compound a (519 mg, 1.20 mmol) prepared in Reference Example 1 was dissolved in THF (10 mL). This solution was cooled to 0°C, and then to the solution was added a 0.93 mol/L solution of diisobutylaluminum hydride (5.30 mL, 4.93 mmol) in hexane, and the mixture was stirred for 2.5 hours. To the reaction mixture was added anhydrous sodium sulfate and saturated aqueous sodium sulfate, and the mixture was further stirred for 1 hour, then filtered. To the filtrate was added water, and the mixture was extracted with ethyl acetate. The organic layer was washed with brine and dried over anhydrous sodium sulfate, and the solvent was evaporated under reduced pressure. The residue was purified by silica gel column chromatography (hexane/ethyl acetate = 4/1→2/1) to give Compound b (348 mg, 74%). ESI-MS m/z 392 (M+H)⁺.

### Reference Example 3 (Compound c)

Compound b (234 mg, 0.597 mmol) prepared in Reference Example 2 was dissolved in dichloromethane (10 mL). To the solution was added pyridinium dichromate (783 mg, 2.08 mmol), and the mixture was stirred at room temperature for 60 hours. The reaction mixture was filtered, and then the resulting filtrate was concentrated under reduced pressure. The residue was purified by silica gel column chromatography (hexane/ethyl acetate = 4/1→2/1) to give Compound c (155 mg, 67%). Reference Example 4 (Compound d)

Compound c (55.8 mg, 0.143 mmol) prepared in Reference Example 3 was dissolved in 1,2-dichloroethane (5 mL). To the solution was added acetic acid (0.0450 mL, 0.786 mmol), n-propylamine (0.0538 mL, 0.654 mmol) and triacetoxy sodium borohydride (130 mg, 0.612 mmol), and the mixture was stirred at room temperature for 12 hours. To the reaction mixture was added saturated aqueous sodium hydrogencarbonate (30 mL), and the mixture was extracted with ethyl acetate. The organic layer was washed with brine and dried over anhydrous sodium sulfate, and the solvent was evaporated under reduced pressure. The residue was purified by preparative thin layer chromatography (chloroform/methanol/concentrated aqueous ammonia = 100/10/1) to give Compound d (51.9 mg, 84%).
ESI-MS m/z 865 (2M+H)⁺.

### Reference Example 6 (Compound e)

### Step 1

In a manner similar to that in Step 1 of Reference Example 1, 3-carbomethoxy-1-phenyl-1-propanone=thiosemicarbazone (10.6 g, 94%) was obtained from 3-carbomethoxy-1-phenyl-1-propanone (8.13 g, 42.3 mmol) and thiosemicarbazide (3.86 g, 42.3 mmol).

### Step 2

In a manner similar to that in Step 2 of Reference Example 1, Compound e (9.70 g, 77%) was obtained from 3-carbomethoxy-1-phenyl-1-propanone=thiosemicarbazone (7.76 g, 29.2 mmol) prepared above, pyridine (11.3 mL, 140 mmol) and trimethylacetyl chloride (14.4 mL, 117 mmol).

### Reference Example 7 (Compound f)

In a manner similar to that in Reference Example 2, Compound f (1.49 g, 100%) was obtained from Compound e (1.50 g, 3.46 mmol) prepared in Reference Example 6 and a 0.93 mol/L solution of diisobutylaluminum hydride in hexane (12.5 mL, 11.6 mmol).

### Reference Example 8 (Compound g)

In a manner similar to that in Reference Example 3, Compound g (517 mg, 52%) was obtained from Compound f (1.00 g, 2.47 mmol) prepared in Reference Example 7 and pyridinium dichromate (2.94 g, 7.81 mmol).

### Reference Example 9 (Compound h)

### Step 1

In a manner similar to that in Step 1 of Reference Example 1, 4-carbomethoxy-1-phenyl-1-butanone=thiosemicarbazone (0.700 g, 88%) was obtained from 4-carbomethoxy-1-phenyl-1-butanone (0.588 g, 2.85 mmol) and thiosemicarbazide (0.260 g, 2.85 mmol).

### Step 2

In a manner similar to that in Step 2 of Reference Example 1, Compound h (318 mg, 64%) was obtained from 4-carbomethoxy-1-phenyl-1-butanone=thiosemicarbazone (0.700 g, 2.51 mmol) obtained above, pyridine (0.431 mL, 5.34 mmol) and trimethylacetyl chloride (0.549 mL, 4.45 mmol).

### Reference Example 10 (Compound i)

In a manner similar to that in Reference Example 2, Compound i (0.393 g, 63%) was obtained from Compound h (667 mg, 1.49 mmol) prepared in Reference Example 9 and a 1.00 mol/L solution of lithium aluminum hydride in hexane (3.00 mL, 3.00 mmol).
ESI-MS m/z: 418 (M-H).

### Reference Example 11 (Compound j)

In a manner similar to that in Reference Example 3, Compound j (189 mg, 56%) was obtained from Compound i (338 mg, 0.805 mmol) prepared in Reference Example 10 and pyridinium dichromate (878 mg, 2.33 mmol).

### Reference Example 12 (Compound k)

### Step 1

2-Aminoacetophenone hydrochloride (2.93 g, 17.1 mmol) was dissolved in acetonitrile (100 mL). To the solution was added di-tert-butyl dicarbonate (5.09 g, 22.9 mmol) and 4-dimethylaminopyridine (2.21 g, 18.1 mmol), and the mixture was stirred at room temperature for 10 hours. To the reaction mixture was added saturated aqueous ammonium chloride, and the mixture was extracted with ethyl acetate. The organic layer was washed with brine and dried over anhydrous sodium sulfate, and the solvent was evaporated under reduced pressure. The residue was purified by silica gel column chromatography (hexane/ethyl acetate = 9/1→4/1) to give 2-(N-tert-butoxycarbonylamino)acetophenone (865 mg, 21%).

### Step 2

2-(N-tert-Butoxycarbonylamino)acetophenone (851 mg, 3.62 mmol) obtained above was dissolved in methanol (20 mL). To the solution was added thiosemicarbazide hydrochloride (1.03 g, 8.04 mmol), and the mixture was stirred at room temperature for 15 hours. To the reaction mixture was added water, and the mixture was extracted with ethyl acetate. The organic layer was washed with brine and dried over anhydrous sodium sulfate, and the solvent was evaporated under reduced pressure to give 2-(N-tert-butoxycarbonylamino)acetophenone=thiosemicarbazone. The resulting 2-(N-tert-butoxycarbonylamino)acetophenone=thiosemicarbazone was dissolved in dichloromethane (50 mL), to the solution was added pyridine (1.75 mL, 21.7 mmol) and trimethylacetyl chloride (2.23 mL, 18.1 mmol), and the mixture was stirred at room temperature for 16 hours. To the reaction mixture was added saturated aqueous sodium hydrogencarbonate, and the mixture was further stirred at room temperature for 1 hour and then extracted with ethyl acetate. The organic layer was washed with brine and dried over anhydrous sodium sulfate, and the solvent was evaporated under reduced pressure. The residue was purified by silica gel column chromatography (hexane/ethyl acetate = 9/1→4/1) to give Compound k (910 mg, 53%). APCI-MS m/z 477 (M+H)⁺.

### Reference Example 13 (Compound m)

Compound k (369 mg, 0.770 mmol) prepared in Reference Example 12 was dissolved in dichloromethane (10 mL). To this solution was added trifluoroacetic acid (1.0 mL), and the mixture was stirred at room temperature for 2 hours. Then, the reaction mixture was evaporated under reduced pressure to give Compound m (436 mg, 100%) as trifluoroacetate.

### Reference Example 14 (Compound n)

### Step 1

Palladium(II) acetate (125 mg, 0.559 mmol) and triphenylphosphine (317 mg, 1.21 mmol) were dissolved in THF (50 mL). To the solution was successively added N-tert-butoxycarbonyl-β-alanine (2.07 g, 10.9 mmol), phenylboronic acid (1.61 g, 13.2 mmol), distilled water (0.477 mL, 26.5 mmol) and trimethylacetic anhydride (3.23 mL, 15.9 mmol), and then the mixture was heated to 60°C and stirred for 24 hours. The reaction mixture was filtered, then to the filtrate was added saturated aqueous sodium hydrogencarbonate, and the mixture was extracted with ethyl acetate. The organic layer was washed with brine and dried over anhydrous sodium sulfate, and the solvent was evaporated under reduced pressure. The residue was purified by silica gel column chromatography (hexane/ethyl acetate = 9/1→4/1) to give 3-(tert-butoxycarbonylamino)propiophenone (1.85 g, 68%).

### Step 2

3-(tert-Butoxycarbonylamino)propiophenone (513 mg, 2.06 mmol) obtained above was dissolved in methanol (40 mL). To the solution was added thiosemicarbazide hydrochloride (562 mg, 4.40 mmol), and the mixture was stirred at room temperature for 8 hours. To the reaction mixture was added water, and the mixture was extracted with ethyl acetate. The organic layer was washed with brine and dried over anhydrous sodium sulfate, and the solvent was evaporated under reduced pressure to obtain pale yellow solid (3-(tert-butoxycarbonylamino)propiophenone=thiosemicarbazone, 513 mg). A part of the resulting solid (198 mg) was dissolved in dichloromethane (10 mL), to the solution was added pyridine (0.300 mL, 3.73 mmol) and trimethylacetyl chloride (0.415 mL, 3.37 mmol), and the mixture was stirred at room temperature for 22 hours. To the reaction mixture was added saturated aqueous sodium hydrogencarbonate, and the mixture was further stirred at room temperature for 1 hour, and then extracted with ethyl acetate. The organic layer was washed with brine and dried over anhydrous sodium sulfate, and the solvent was evaporated under reduced pressure. The residue was purified by preparative thin layer chromatography (hexane/ethyl acetate = 2/1) to give Compound n (319 mg, 82%).
APCI-MS m/z 491 (M+H)⁺.

### Reference Example 15 (Compound o)

In a manner similar to that in Example 37, Compound o (252 mg, 90%) was obtained as trifluoroacetate from Compound n (274 mg, 0.557 mmol) prepared in Reference Example 14 and trifluoroacetic acid (1.0 mL).
APCI-MS m/z: 391 (M+H)⁺.

### Reference Example 16 (Compound p)

Sodium hydroxide (2.68 g, 66.9 mmol) was dissolved in water (2 mL). To the solution was added 1,4-dioxane (4 mL), and then added Compound h (9.65 g, 22.3 mmol) prepared in Reference Example 9. The mixture was stirred at room temperature for 5 hours, and then to the mixture was added 1 mol/L hydrochloric acid (20 mL) and water (30 mL). The deposited white crystals were collected by filtration. The resulting white crystals were washed with water and further with diisopropyl ether, and then dried under reduced pressure to give Compound p (9.17 g, 95%). Reference Example 17 (Compound q)

Compound p (4.44 g, 10.2 mmol) prepared in Reference Example 16 was dissolved in tert-butanol (100 mL), and the solution was heated to 80°C. To this solution was added triethylamine (1.4 mL, 10.2 mmol) and diphenylphosphoryl azide (2.2 mL, 10.2 mmol), and the mixture was stirred at the same temperature for 9 hours. The reaction mixture was concentrated under reduced pressure, to the residue was added water (100 mL), and the mixture was extracted with ethyl acetate (300 mL).

The organic layer was washed with brine (50 mL), and then dried over anhydrous sodium sulfate, and the solvent was evaporated under reduced pressure. The residue was purified by silica gel column chromatography (hexane/ethyl acetate = 3/1) to give Compound q (1.91 g, 3.78 mmol).

### Reference Example 18 (Compound r)

Compound q (1.91 g, 3.78 mmol) prepared in Reference Example 17 was dissolved in 4 mol/L hydrogen chloride - ethyl acetate (50 mL), and the solution was left standing for 30 minutes. The solvent was evaporated under reduced pressure to give hydrochloride of Compound r (1.43 g, 3.24 mmol).
APCI-MS m/z: 405 (M+H)⁺.

### Reference Example 19 (Compound s)

Compound f (508 mg, 1.25 mmol) prepared in Reference Example 7 was dissolved in dichloromethane (20 mL). To the solution was added triethylamine (0.251 mL, 1.80 mmol), and the mixture was cooled to 0°C. Then, to the mixture was added methanesulfonyl chloride (0.116 mL, 1.50 mmol), and the mixture was stirred at room temperature for 30 minutes. To the reaction mixture was added water and 1 mol/L hydrochloric acid, and the mixture was extracted with chloroform. The organic layer was washed with brine, and then dried over anhydrous sodium sulfate, and the solvent was evaporated under reduced pressure to give Compound s (0.623 g, 99%).
APCI-MS m/z: 484 (M+H)⁺.

### Reference Example 20 (Compound t)

In a manner similar to that in Step 2 of Reference Example 1, 3-methoxycarbonyl-1-phenyl-1-propanone=thiosemicarbazone (1.00 g, 3.58 mmol) prepared in Step 1 of Reference Example 6 was allowed to react with isobutyryl chloride (1.49 mL, 14.3 mmol) and pyridine (1.48 mL, 17.2 mmol).

Then, the product of the above reaction was dissolved in a mixed solution of 5 mol/L aqueous sodium hydroxide (10 mL) and methanol (20 mL), and the solution was vigorously stirred for 2 hours. The reaction mixture was added dropwise to 1 mol/L hydrochloric acid (200 mL), and the deposited white precipitates were collected by filtration, and dried under reduced pressure to give Compound t (1.39 g, 99%). Reference Example 21 (Compound u)

Compound t (1.39 g, 3.55 mmol) prepared in Reference Example 20 was dissolved in THF (10 mL). To this solution was added N,O-dimethylhydroxyamine hydrochloride (0.416 g, 4.26 mmol) and N,N-carbonyldiimidazole (0.634 g, 3.91 mmol), and the mixture was stirred at room temperature for 15 hours. To the reaction mixture was added water, and the mixture was extracted with chloroform. The organic layer was washed with 1 mol/L hydrochloric acid and water, and then dried over anhydrous sodium sulfate, and the solvent was evaporated under reduced pressure. The residue was purified by silica gel column chromatography (chloroform/methanol = 99/1→95/1) to give Compound u (1.02 g, 66%).

### Reference Example 22 (Compound v)

Compound u (0.372 g, 0.856 mmol) prepared in Reference Example 21 was dissolved in THF (15 mL). This solution was cooled to 0°C, then to the solution was added a 1.01 mol/L solution of diisobutylaluminum hydride in hexane (1.68 mL, 1.70 mmol), and the mixture was stirred for 2.5 hours. To the reaction mixture was added anhydrous sodium sulfate and saturated aqueous sodium sulfate, and the mixture was further stirred for 1 hour, and then filtered. To the filtrate was added water, and the mixture was extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 3/1) to give Compound v (0.249 g, 77%).

### Reference Example 23 (Compound w)

Compound 177 (0.585 g, 1.40 mmol) prepared in Example 176 was dissolved in methanol (15 mL). To the solution was added sodium methoxide (0.170 g, 3.14 mmol), and the mixture was stirred at room temperature for 6 hours. To the reaction mixture was added saturated aqueous ammonium chloride, and the mixture was extracted with ethyl acetate. The organic layer was washed with saturated aqueous sodium hydrogencarbonate and brine, and then dried over anhydrous sodium sulfate, and the solvent was evaporated under reduced pressure. The residue was purified by silica gel column chromatography (hexane/ethyl acetate = 9/1→4/1→2/1→1/1) to give Compound w (0.206 g, 39%).
APCI-MS m/z: 378 (M+H)⁺.

### Reference Example 24 (Compound x)

Sodium hydroxide (2.7 g, 67 mmol) was dissolved in water (23 mL). To the solution was added methanol (30 mL). To this solution was added Compound h (254 mg, 0.567 mmol) prepared in Reference Example 9, and the mixture was stirred at room temperature for 5 hours. To the reaction mixture was added 1 mol/L hydrochloric acid (20 mL) and water (30 mL), and the deposited white solid was collected by filtration. The resulting solid was washed with water and diisopropyl ether, and then dried under reduced pressure to give Compound x (234 mg, 95%). ¹H NMR (270 MHz, CDCl₃) δ (ppm) : 1.29 (s, 9H), 1.32 (s, 9H), 1.65-1.75 (m, 1H), 2.10-2.35 (m, 2H), 2.50 (m, 2H), 3.10-3.20 (m, 1H), 7.23-7.35 (m, 6H), 7.92 (br s, 1H). Reference Example 25 (Compound y)

### Step 1

Monomethyl succinate (1.00 g, 7.57 mmol), 3-(tert-butyldimethylsilyloxy)phenylboronic acid (2.23 g, 8.84 mmol), triphenylphosphine (0.280 g, 1.07 mmol) and palladium(II) acetate (0.10 g, 0.46 mmol) were suspended in THF (20 mL). To the suspension was added water (0.340 mL, 18.9 mmol) and pivalic anhydride (2.30 mL, 11.3 mmol), and the mixture was stirred at 60°C for 33 hours under an argon atmosphere. The reaction mixture was concentrated under reduced pressure. Then, to the residue was added water and saturated aqueous sodium hydrogencarbonate, and the mixture was extracted with ethyl acetate. The organic layer was washed with brine, dried over anhydrous sodium sulfate, and then concentrated under reduced pressure. The residue was purified by using a 12-system parallel preparative chromatography (Hi-Flash™ column, Yamazen, hexane→ hexane/ethyl acetate = 3/2) to give 4-(3-tert-butyldimethylsilyloxophenyl)-4-oxobutyric acid methyl ester (557 mg, 23%).
APCI-MS m/z: 323 (M+H)⁺.

### Step 2

In a manner similar to that in Step 1 of Reference Example 1, 4-(3-tert-butyldimethylsilyloxophenyl)-4-oxobutyric acid methyl ester (557 mg, 1.73 mmol) prepared above was reacted with concentrated hydrochloric acid (several drops) and thiosemicarbazide (481 mg, 5.28 mmol) to give 4-(3-tert-butyldimethylsilyloxophenyl)-4-thiosemicarbazonobutyric acid methyl ester (540 mg, 79%).

### Step 3

In a manner similar to that in Step 2 of Reference Example 1, 4-(3-tert-butyldimethylsilyloxophenyl)-4-thiosemicarbazonobutyric acid methyl ester (540 mg, 1.37 mmol) prepared above was allowed to react with pyridine (0.662 mL, 8.19 mmol) and trimethylacetyl chloride (1.00 mL, 8.12 mmol) to give Compound y (309 mg, 40%).
APCI-MS m/z: 564 (M+H)⁺.

### Reference Example 26 (Compound z)

Compound y (246 mg, 0.436 mmol) prepared in Reference Example 25 was dissolved in THF (10 mL). To the solution was added diisobutylaluminum hydride (1.01 mol/L solution in toluene, 1.38 mL, 1.39 mmol) under ice cooling, and the mixture was stirred at the same temperature for 2 hours. To the reaction mixture was further added diisobutylaluminum hydride (1.01 mol/L solution in toluene, 0.86 mL, 0.87 mmol), and the mixture was stirred for 1 hour. Then, to the mixture was added saturated aqueous sodium sulfate and anhydrous sodium sulfate, and the mixture was stirred at room temperature for 45 minutes. The precipitates were filtrated off, and the filtrate was concentrated under reduced pressure. The residue was purified by using a 12-system parallel preparative chromatography (hexane→ hexane/ethyl acetate = 1/1) to give Compound z (145 mg, 62%).
¹H NMR (300 MHz, CDCl₃) δ (ppm): 0.17 (s, 6H), 0.96 (s, 9H), 1.29 (s, 9H), 1.33 (s, 9H), 1.57 (m, 1H), 2.05 (m, 1H), 2.29 (m, 1H), 3.13 (m, 1H), 3.70-3.80 (m, 2H), 6.70 (m, 1H), 6.80 (dd, J = 2.0, 2.2 Hz, 1H), 6.93 (m, 1H), 7.17 (dd, J = 8.1, 8.1 Hz, 1H), 7.89 (s, 1H).

### Reference Example 27 (Compound aa)

Compound z (72 mg, 0.13 mmol) prepared in Reference Example 26 was dissolved in dichloromethane (1 mL). To the solution was added pyridinium dichromate (156 mg, 0.415 mmol), and the mixture was stirred at room temperature for 24 hours. The precipitates were filtrated off, and washed with chloroform. Then, the filtrate and washing solution were collected, and concentrated under reduced pressure. The residue was purified by preparative thin layer chromatography (chloroform/acetone = 9/1) to give Compound aa (43 mg, 60%).
APCI-MS m/z: 534 (M+H)⁺.

### Reference Example 28 (Compound bb)

3-(tert-Butoxycarbonylamino)propiophenone=thiosemicarbazone (4.07g, 12.6 mmol) prepared as an intermediate in Step 2 of Reference Example 14 was dissolved in acetone (20 mL), to the solution was added pyridine (5.4 mL, 63.1 mmol) and acetic anhydride (6.0 mL, 63.1 mmol), and the mixture was stirred at room temperature for 24 hours. To the reaction mixture was added saturated aqueous sodium hydrogencarbonate, and the mixture was extracted with ethyl acetate. The organic layer was washed with brine and dried over anhydrous sodium sulfate, and the solvent was evaporated under reduced pressure. To the residue was added methanol (30 mL) and hydrazine monohydrate (20 mL), and the mixture was stirred at room temperature for 1 hour. To the reaction mixture was added 1 mol/L hydrochloric acid, and the mixture was extracted with ethyl acetate. The organic layer was washed with brine and dried over anhydrous sodium sulfate, and the solvent was evaporated under reduced pressure. The residue was reslurried in diisopropyl ether (30 mL) to give Compound bb (4.38 g, 95%).
APCI-MS *m*/*z:* 365 (M+H)⁺.

### Reference Example 29 (Compound cc)

In a manner similar to that in Example 88, Compound cc (103 mg, 84%) was obtained from Compound bb (103 mg, 0.283 mmol) prepared in Reference Example 28, 4-bromobutyryl chloride (0.082 mL, 0.707 mmol), pyridine (0.072 mL, 0.848 mmol) and sodium acetate (232 mg, 2.83 mmol).
APCI-MS m/z 433 (M+H)⁺.

### Reference Example 30 (Compound dd)

In a manner similar to that in Example 81, Compound dd (490 mg, 100%) was obtained from Compound bb (400 mg, 1.10 mmol) prepared in Reference Example 28, pyridine (0.222 mL, 2.75 mmol), 5-bromovaleryl chloride (0.367 mL, 2.75 mmol) and sodium acetate (225 mg, 2.75 mmol).
¹H NMR (270 MHz, CDCl₃) δ (ppm): 1.44 (s, 9H), 1.85-1.98 (m, 4H), 2.30 (s, 3H), 2.44-2.55 (m, 3H), 3.17-3.29 (m, 2H), 3.68 (m, 1H), 3.86 (m, 2H), 4.64 (br s, 1H), 7.21-7.33 (m, 5H).

### Reference Example 31 (hydrochloride of Compound m)

In a manner similar to that in Step 5 of Example 77, hydrochloride of Compound m (2.80 g, quantitative) was obtained from Compound k (3.13 g, 6.57 mmol) prepared in Reference Example 12 and 4 mol/L hydrogen chloride-ethyl acetate (30 mL).
¹H NMR (270 MHz, DMSO-*d*_{*6*}) δ (ppm): 1.17 (s, 9H), 1.32 (s, 9H), 4.06 (d, *J=* 13.7 Hz, 1H), 4.21 (d, J= 13.7 Hz, 1H), 7.20-7.44 (m, 5H), 8.30 (brs, 3H), 11.17 (s, 1H).

### Reference Example 32 (Compound ff)

Hydrochloride of Compound m (2.80 g, 6.78 mmol) prepared in Reference Example 31 was suspended in dichloromethane (50 mL). To the suspension was added triethylamine (3.80 mL, 27.3 mmol) and 3-chloropropanesulfonyl chloride (1.24 mL, 10.2 mmol) under ice cooling, and the mixture was stirred at the same temperature for 20 minutes. To the reaction mixture was added water and 1 mol/L hydrochloric acid, and the mixture was extracted with chloroform. The organic layer was washed with saturated aqueous sodium hydrogencarbonate and brine, then dried over anhydrous sodium sulfate, and concentrated under reduced pressure. The residue was triturated with a mixed solvent of diisopropyl ether and ethyl acetate to give Compound ff(3.01 g, 86%).
ESI-MS m/z: 515, 517 (M-H)⁻.

### Reference Example 33 (Compound gg)

Acetic anhydride (30 mL) was added to 2-(tert-butoxycarbonylamino)acetophenone=thiosemicarbazone (2.91 g, 9.44 mmol) prepared in Step 2 of Reference Example 12 as an intermediate, and the mixture was stirred at 130°C for 5 minutes and subsequently at 70°C for 1 hour. The reaction mixture was left to cool and then triturated with a mixed solvent of diisopropyl ether and n-hexane to give Compound gg (2.06 g, 56%).
APCI-MS *m*/*z:* 393 (M+H)⁺.

### Reference Example 34 (Compound hh)

Compound gg (2.01 g, 5.12 mmol) prepared in Reference Example 33 was dissolved in acetonitrile (20 mL), to the solution was added hydrazine monohydrate (8.0 mL, 0.16 mol), and the mixture was stirred at room temperature for 6 hours. To the reaction mixture was added water, and the mixture was extracted with ethyl acetate. The organic layer was washed with brine, dried over anhydrous sodium sulfate and concentrated under reduced pressure. The residue was purified by using a 12-system parallel preparative chromatography (Hi-Flash™ column, Yamazen, hexane/ethyl acetate = 2/3) to give Compound hh (1.42 g, 79%).
APCI-MS *m*/*z:* 351 (M+H)⁺.

### Reference Example 35 (Compound ii)

In a manner similar to that in Example 88, Compound hh (1.01 g, 2.88 mmol) prepared in Reference Example 34 was allowed to react with 4-bromobutyryl chloride (0.840 mL, 7.24 mmol) in the presence of pyridine (0.585 mL, 7.23 mmol) followed by treating with sodium acetate (608 mg, 7.41 mmol) in DMSO (20 mL) to give Compound ii (0.99 g, 82%).
APCI-MS *m*/*z*: 419 (M+H)⁺.

### Reference Example 36 (Compound jj)

In a manner similar to that in Step 5 of Example 77, Compound ii (3.81 g, 9.10 mmol) prepared in Reference Example 35 was dissolved in 4 mol/L hydrogen chloride-ethyl acetate (30 mL), and the mixture was stirred at room temperature for 30 minutes. The reaction mixture was evaporated under reduced pressure, and then the residue was reslurried in diethyl ether to give Compound jj (2.64 g, 91%).
APCI-MS m/z: 319 (M+H)⁺.

### Reference Example 36 (Compound kk)

In a manner similar to that in Example 81, Compound kk (1.85 g, 95%) was obtained from Compound hh (1.57 g, 4.48 mmol) prepared in Reference Example 34, pyridine (1.20 mL, 13.4 mmol), 5-bromovaleryl chloride (1.50 mL, 11.2 mmol) and sodium acetate (3.7 g, 44.8 mmol).
APCI-MS *m*/*z:* 433 (M+H)⁺.

### Reference Example 37 (Compound mm)

In a manner similar to that in Step 5 of Example 77, Compound mm (1.42 g, 90%) was obtained from Compound kk (1.85 g, 4.28 mmol) prepared in Reference Example 36 and 4 mol/L hydrogen chloride-ethyl acetate (20 mL).
APCI-MS m/z: 333 (M+H)⁺

### Reference Example 39 (Compound nn)

### Step 1

In a manner similar to that in Step 3 of Example 77, 5-(methoxycarbonyl)-valerophenone=thiosemicarbazone (quantitative) was obtained from 5-(ethoxycarbonyl)valerophenone (0.299 g, 1.28 mmol) and thiosemicarbazide hydrochloride (0.490 g, 3.84 mmol).

### Step 2

In a manner similar to that in Step 2 of Reference Example 1, Compound nn (0.200 g, 42%) was obtained from 5-(methoxycarbonyl)valerophenone=thiosemicarbazone (0.233 g, 0.994 mmol) obtained above, pyridine (0.387 mL, 4.78 mmol) and trimethylacetyl chloride (0.444 mL, 3.98 mmol).

### Reference Example 40 (Compound oo)

In a manner similar to that in Reference Example 20, Compound oo (0.185 g, 98%) was obtained from Compound nn (0.200 g, 0.420 mmol) prepared in Reference Example 39, 5 mol/L aqueous sodium hydroxide (10 mL) and methanol (20 mL). Reference Example 41 (Compound pp)

In a manner similar to that in Reference Example 21, Compound pp (0.198 g, 90%) was obtained from Compound oo (0.200 g, 0.447 mmol) prepared in Reference Example 40, N,N'-carbonyldiimidazole (79.8 g, 492 mmol) and N,O-dimethylhydroxyamine hydrochloride (6.2 g, 64.0 mmol).

### Reference Example 42 (Compound qq)

In a manner similar to that in Reference Example 22, Compound qq (0.154 g, 88%) was obtained from Compound pp (0.198 g, 0.404 mmol) prepared in Reference Example 41 and diisobutylaluminum hydride (0.95 mol/L solution in hexane, 0.51 mL, 0.485 mmol).

### Reference Example 43 (Compound rr)

Compound f (0.541 g, 1.33 mmol) prepared in Reference Example 7 was dissolved in dichloromethane (7.0 mL). To the solution was added triethylamine (0.464 mL, 3.33 mmol) and p-toluenesulfonyl chloride (259 mg, 1.36 mmol), and the mixture was stirred at room temperature for 15 hours. To the reaction mixture was added 1.0 mol/L hydrochloric acid and water, and the mixture was extracted with chloroform. The organic layer was washed with water, and then dried over anhydrous sodium sulfate, and the solvent was evaporated under reduced pressure. The residue was purified by silica gel column chromatography (chloroform) to give Compound rr (0.515 g, 69%).
APCI-MS m/z: 560 (M+H)⁺.

### Reference Example 44 (Compound ss)

In a manner similar to that in Reference Example 43, Compound ss (0.277 g, 50%) was obtained from Compound i (0.405 g, 0.965 mmol) prepared in Reference Example 10, triethylamine (0.336 mL, 2.41 mmol) and p-toluenesulfonyl chloride (202 mg, 1.06 mmol).

### Pharmaceutical Preparation Example 1: Tablets (Compound 7)

Tablets having the following composition are prepared in a conventional manner. Compound 7 (40 g), lactose (286.8 g) and potato starch (60 g) are mixed, and 10% aqueous solution of hydroxypropylcellulose (120 g) is added to the mixture. This mixture is kneaded, granulated and dried in a conventional manner, and then the granules are sized to obtain granules for tablet pressing. Magnesium stearate (1.2 g) is added to the granules for tablet pressing and mixed. Tableting is performed with a tableting machine having a pestle of 8 mm a diameter (Kikusui, RT-15) to obtain tablets (containing 20 mg/tablet of active ingredient).

| Formulation | | |
|---|---|---|
| Compound 7 | 20 mg | |
| Lactose | 143.4 mg | |
| Potato starch | 30 mg | |
| Hydroxypropylcellulose | 6 mg | |
| Magnesium stearate | 0.6 mg | |
| | 200 mg | |

### Industrial Applicability

The present invention provides a thiadiazoline derivative or a pharmacologically acceptable salt thereof which is useful for therapeutic treatment of a disease involving cell proliferation, for example, a malignant tumor (breast cancer, gastric cancer, ovarian cancer, colon cancer, lung cancer, brain cancer, laryngeal cancer, hematological cancer, urinary or genital tumor including bladder cancer and prostate cancer, renal cancer, skin cancer, liver cancer, pancreatic cancer, uterine cancer, etc.), restenosis, cardiac hypertrophy, an immunologic disease, and the like.

## Claims

1. A thiadiazoline derivative represented by the general formula (I), or a pharmacologically acceptable salt thereof: 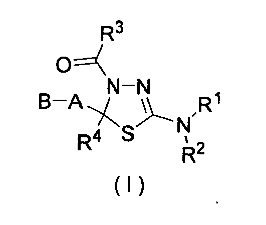 <wherein,
R¹ represents a hydrogen atom, substituted or unsubstituted lower alkyl, substituted or unsubstituted lower alkenyl, substituted or unsubstituted lower alkynyl, or substituted or unsubstituted cycloalkyl,
R² represents a hydrogen atom, or -COR⁵ (wherein R⁵ represents substituted or unsubstituted lower alkyl, substituted or unsubstituted lower alkenyl, substituted or unsubstituted lower alkynyl, or substituted or unsubstituted cycloalkyl), or
R¹ and R² are combined together with the adjacent nitrogen atom to form a substituted or unsubstituted heterocyclic group,
R³ represents a hydrogen atom, substituted or unsubstituted lower alkyl, substituted or unsubstituted lower alkenyl, substituted or unsubstituted lower alkynyl, or substituted or unsubstituted cycloalkyl,
R⁴ represents substituted or unsubstituted aryl, or a substituted or unsubstituted heterocyclic group,
A represents -(CH₂)ₙ- (wherein n represents an integer of 1 to 6), or a group of the formula (II) 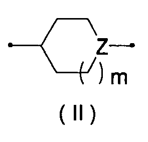 (wherein m represents an integer of 0 to 2, and Z represents CH or a nitrogen atom capable of binding to B), and
(i) when A is -(CH₂)ₙ-, and n is 1 or 2,
B represents -NR⁶R⁷ {wherein R⁶ represents a hydrogen atom, or lower alkyl, R⁷ represents substituted lower alkyl, -COR⁸ [wherein R⁸ represents substituted lower alkyl (provided that R⁸ is not trifluoromethyl), substituted lower alkoxy, substituted or unsubstituted aryloxy, a substituted or unsubstituted heterocyclic group, or -NR⁹R¹⁰ (wherein R⁹ and R¹⁰ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R⁹ and R¹⁰ are combined together with the adjacent nitrogen atom to form a substituted or unsubstituted heterocyclic group)], or R⁶ and R⁷ are combined together with the adjacent nitrogen atom to form a substituted or unsubstituted heterocyclic group},
-OR¹¹ (wherein R¹¹ represents substituted lower alkyl, substituted or unsubstituted lower alkanoyl, substituted or unsubstituted lower alkylcarbamoyl, substituted or unsubstituted di-(lower alkyl)carbamoyl, or substituted or unsubstituted heterocyclylcarbonyl),
-SR¹² (wherein R¹² has the same meaning as that of the aforementioned R¹¹), or CH=NR13 (wherein R¹³ represents hydroxy, or substituted or unsubstituted lower alkoxy),
(ii) when A is -(CH₂)ₙ-, and n is an integer of 3 to 6 ,
B represents -NR¹⁴R¹⁵ {wherein R¹⁴ and R¹⁵ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, -COR¹⁶ [wherein R¹⁶ represents substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, substituted or unsubstituted lower alkoxy, substituted or unsubstituted aryloxy, or -NR¹⁷R¹⁸ (wherein R¹⁷ and R¹⁸ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R¹⁷ and R¹⁸ are combined together with the adjacent nitrogen atom to form a substituted or unsubstituted heterocyclic group)], or -SO₂R¹⁹ [wherein R¹⁹ represents substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, or -NR²⁰R²¹ (wherein R²⁰ and R²¹ are the same or different, and represent a hydrogen atom, substituted or unsubstituted lower alkyl, substituted or unsubstituted lower alkenyl, substituted or unsubstituted lower alkynyl, or substituted or unsubstituted cycloalkyl, or R²⁰ and R²¹ are combined together with the adjacent nitrogen atom to form a substituted or unsubstituted heterocyclic group)], or R¹⁴ and R¹⁵ are combined together with the adjacent nitrogen atom to form a substituted or unsubstituted heterocyclic group},
-OR²² (wherein R²² has the same meaning as that of the aforementioned R¹¹),
-SR²³ (wherein R²³ has the same meaning as that of the aforementioned R¹¹), or
-CH=NR²⁴ (wherein R²⁴ has the same meaning as that of the aforementioned R¹³),
(iii) when A is a group of the formula (II),
B represents a hydrogen atom, substituted or unsubstituted lower alkyl, substituted or unsubstituted lower alkanoyl, substituted or unsubstituted lower alkoxycarbonyl, or substituted or unsubstituted lower alkylsulfonyl>.

2. The thiadiazoline derivative or a pharmacologically acceptable salt thereof according to claim 1, wherein R¹ is a hydrogen atom, or lower alkyl.

3. The thiadiazoline derivative or a pharmacologically acceptable salt thereof according to claim 1 or 2, wherein R² is -COR⁵ (wherein R⁵ has the same meaning as that mentioned above).

4. The thiadiazoline derivative or a pharmacologically acceptable salt thereof according to claim 3, wherein R⁵ is lower alkyl.

5. The thiadiazoline derivative or a pharmacologically acceptable salt thereof according to claim 3, wherein R⁵ is tert-butyl.

6. The thiadiazoline derivative or a pharmacologically acceptable salt thereof according to any one of claims 1 to 5, wherein R³ is lower alkyl.

7. The thiadiazoline derivative or a pharmacologically acceptable salt thereof according to any one of claims 1 to 5, wherein R³ is tert-butyl.

8. The thiadiazoline derivative or a pharmacologically acceptable salt thereof according to any one of claims 1 to 7, wherein R⁴ is substituted or unsubstituted aryl.

9. The thiadiazoline derivative or a pharmacologically acceptable salt thereof according to any one of claims 1 to 7, wherein R⁴ is phenyl.

10. The thiadiazoline derivative or a pharmacologically acceptable salt thereof according to any one of claims 1 to 9, wherein A is -(CH₂)ₙ- (wherein n has the same meaning as that mentioned above).

11. The thiadiazoline derivative or a pharmacologically acceptable salt thereof according to claim 10, wherein n is 1 or 2.

12. The thiadiazoline derivative or a pharmacologically acceptable salt thereof according to claim 11, wherein B is -NR⁶R⁷ (wherein R⁶ and R⁷ have the same meanings as those mentioned above, respectively).

13. The thiadiazoline derivative or a pharmacologically acceptable salt thereof according to claim 12, wherein R⁶ is a hydrogen atom.

14. The thiadiazoline derivative or a pharmacologically acceptable salt thereof according to claim 12 or 13, wherein R⁷ is -COR⁸ (wherein R⁸ has the same meaning as that mentioned above).

15. The thiadiazoline derivative or a pharmacologically acceptable salt thereof according to claim 12, wherein R⁶ and R⁷ are combined together with the adjacent nitrogen atom to form a substituted or unsubstituted heterocyclic group.

16. The thiadiazoline derivative or a pharmacologically acceptable salt thereof according to claim 10, wherein n is an integer of 3 to 6.

17. The thiadiazoline derivative or a pharmacologically acceptable salt thereof according to claim 10, wherein n is 3.

18. The thiadiazoline derivative or a pharmacologically acceptable salt thereof according to claim 16 or 17, wherein B is -NR¹⁴R¹⁵ (wherein R¹⁴ and R¹⁵ have the same meanings as those mentioned above, respectively).

19. The thiadiazoline derivative or a pharmacologically acceptable salt thereof according to claim 18, wherein R¹⁴ is a hydrogen atom.

20. The thiadiazoline derivative or a pharmacologically acceptable salt thereof according to claim 18 or 19, wherein R¹⁵ is substituted or unsubstituted lower alkyl.

21. The thiadiazoline derivative or a pharmacologically acceptable salt thereof according to claim 18 or 19, wherein R¹⁵ is -COR¹⁶ (wherein R¹⁶ has the same meaning as that mentioned above).

22. The thiadiazoline derivative or a pharmacologically acceptable salt thereof according to claim 21, wherein R¹⁶ is a substituted or unsubstituted heterocyclic group.

23. The thiadiazoline derivative or a pharmacologically acceptable salt thereof according to claim 21, wherein R¹⁶ is -NR¹⁷R¹⁸ (wherein R¹⁷ and R¹⁸ have the same meanings as those mentioned above, respectively).

24. The thiadiazoline derivative or a pharmacologically acceptable salt thereof according to claim 18 or 19, wherein R¹⁵ is -SO₂R¹⁹ (wherein R¹⁹ has the same meaning as that mentioned above).

25. The thiadiazoline derivative or a pharmacologically acceptable salt thereof according to any one of claims 1 to 9, wherein A is a group of the formula (II).

26. The thiadiazoline derivative or a pharmacologically acceptable salt thereof according to claim 25, wherein Z is a nitrogen atom.

27. The thiadiazoline derivative or a pharmacologically acceptable salt thereof according to claim 25 or 26, wherein B is a hydrogen atom, or substituted or unsubstituted lower alkyl.

28. A pharmaceutical composition which comprises the thiadiazoline derivative or a pharmacologically acceptable salt thereof according to any one of claims 1 to 27 as an active ingredient.

29. A mitotic kinesin Eg5 inhibitor which comprises the thiadiazoline derivative or a pharmacologically acceptable salt thereof according to any one of claims 1 to 27 as an active ingredient.

30. An antitumor agent which comprises the thiadiazoline derivative or a pharmacologically acceptable salt thereof according to any one of claims 1 to 27 as an active ingredient.

31. A method for inhibiting a mitotic kinesin Eg5 which comprises administering an effective amount of the thiadiazoline derivative or a pharmacologically acceptable salt thereof according to any one of claims 1 to 27.

32. A method for therapeutic treatment of a malignant tumor which comprises administering an effective amount of the thiadiazoline derivative or a pharmacologically acceptable salt thereof according to any one of claims 1 to 27.

33. Use of the thiadiazoline derivative or a pharmacologically acceptable salt thereof according to any one of claims 1 to 27 for the manufacture of a mitotic kinesin Eg5 inhibitor.

34. Use of the thiadiazoline derivative or a pharmacologically acceptable salt thereof according to any one of claims 1 to 27 for the manufacture of the antitumor agent.
